(19) **Europäisches Patentamt / European Patent Office / Office européen des brevets**

(11) **EP 4 692 344 A1**

(12) **EUROPEAN PATENT APPLICATION**
published in accordance with Art. 153(4) EPC

(43) Date of publication:
**11.02.2026 Bulletin 2026/07**

(21) Application number: **24764152.5**

(22) Date of filing: **26.02.2024**

(51) International Patent Classification (IPC):
*C12N 15/11* (2006.01)   *C12N 15/62* (2006.01)
*C12N 9/48* (2006.01)   *C07K 14/005* (2006.01)
*A61K 39/12* (2006.01)   *A61P 31/12* (2006.01)

(52) Cooperative Patent Classification (CPC):
A61K 39/12; A61P 31/12; C07K 14/005;
C12N 9/48; C12N 15/11; C12N 15/62

(86) International application number:
**PCT/KR2024/002461**

(87) International publication number:
**WO 2024/181753 (06.09.2024 Gazette 2024/36)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **27.02.2023 KR 20230026201**

(71) Applicant: **Microuni Co., Ltd.**
**Cheongju-si, Chungcheongbuk-do 28644 (KR)**

(72) Inventors:
• **SONG, Min-Suk**
**Cheongju-si, Chungcheongbuk-do 28382 (KR)**
• **BAEK, Yun Hee**
**Cheongju-si, Chungcheongbuk-do 28382 (KR)**
• **CHOI, Won-Suk**
**Suwon-si, Gyeonggi-do 16508 (KR)**
• **OH, Sol**
**Jincheon-gun, Chungcheongbuk-do 27869 (KR)**

(74) Representative: **ABG Intellectual Property Law, S.L.**
**Avenida de Burgos, 16D**
**Edificio Euromor**
**28036 Madrid (ES)**

Remarks:
The complete document including Reference Table(s) and the Sequence Listing(s) can be downloaded from the EPO website

(54) **SELF-AMPLIFYING RNA CONSTRUCT AND USE THEREOF**

(57) The present invention relates to a self-amplifying RNA construct by which the expression of a target protein is optimized. Compared to a conventional mRNA and enterovirus-based self-amplifying RNA, an enterovirus-based optimized self-amplifying RNA according to the present invention increased the expression level of a target protein. In addition, the multiple-antigen self-amplifying RNA expressed one or more target proteins simultaneously, and the expression level thereof was much higher than that of a conventional mRNA. Therefore, the self-amplifying RNA construct according to the present invention can be widely used for vaccines and the like.

Figure 15

Single-antigen self-amplifying RNA construct

Multiple-antigen self-amplifying RNA construct

LEALFQ/GPPVYR (SEQ ID NO: 50)

**Description**

**Technical Field**

The present invention relates to a self-amplifying RNA construct and a use thereof.

**Background Art**

Vaccines play an important role in health by preventing infection and transmission of various diseases worldwide. According to the World Health Organization, it is estimated that vaccines can prevent 2 to 3 million deaths annually. Traditional vaccines such as attenuated pathogens, inactivated pathogens, and subunit vaccines have prevented various fatal diseases. However, rapid development and large-scale distribution of vaccines against various infectious pathogens that can evade adaptive immune response still remain difficult.

On the other hand, nucleic acid therapeutics have been attracting attention as an alternative that can overcome the above problems (Vogel et al., Clin Microbiol Rev., 8(3):406-410 (1995)). In particular, mRNA-based RNA vaccines are safe because they are not integrated into the genome, unlike some viral vaccines, so there is no concern about mutations, and they can be manufactured in a cell-free manner, allowing for economical, rapid, and efficient vaccine production. In addition, a single mRNA vaccine has the advantage of being able to encode multiple antigens, thereby enhancing the immune response to the pathogen, and being able to evade the immune response to the vector *in vivo.*

Among these, self-amplifying RNA (saRNA) vaccines have a construct that has RNA polymerase activity in non-structural proteins and encodes structural proteins by replacing them with antigen proteins (gene of interest, GOI). Therefore, the self-amplifying RNA vaccine has the advantage of being able to sufficiently produce antibodies with just one vaccination, and many studies are being conducted on it as a vaccine construct. In fact, saRNA constructs can induce immune responses even when administered at a dose that is about 10 to about 100 times lower than that of mRNA, and it was confirmed that the expression of antigen proteins was maintained for up to 60 days when administered to mice.

Most self-amplifying RNA vaccines currently in use are based on the alphavirus genome (Bogers et al., J Infect Dis., 211(6):947-955(2015)). In addition, there are viruses with self-amplifying RNA comprising RNA polymerase within non-structural proteins as their genomes, such as picornavirus, flavivirus, and coronavirus, but none of them, other than alphavirus, have been commercialized yet, and more research is needed.

**Detailed Description of Invention**

**Technical Problem**

Accordingly, the present inventors studied to develop a self-amplifying RNA vaccine construct that can be used quickly and efficiently. As a result, a novel self-amplifying RNA construct optimized for the expression of antigen proteins was produced by modifying the 5' untranslated region (UTR), protease cleavage site, stop codon, and poly(A) tail sequence of an enterovirus-based self-amplifying RNA construct. In addition, it was confirmed that the modified self-amplifying RNA construct can significantly increase the expression of antigen protein in cells compared to conventional mRNA and conventional enterovirus-based self-amplifying RNA constructs. Based on the above, the present inventors completed the present invention.

**Solution to Problem**

In order to achieve the above object, in one aspect of the present invention, there is provided a polynucleotide comprising a nucleic acid sequence encoding a fusion protein, comprising: a non-structural protein of a self-amplifying virus; a first target protein; and a protease cleavage site.

In another aspect of the present invention, there is provided a recombinant vector loaded with the polynucleotide.

In another aspect of the present invention, there is provided a method for producing an RNA replicon, comprising: producing the recombinant vector; and synthesizing RNA from the recombinant vector.

In another aspect of the present invention, there is provided a vaccine composition comprising the polynucleotide or the recombinant vector as an active ingredient.

In another aspect of the present invention, there are provided a 2A protease cleavage site comprising the amino acid sequence of SEQ ID NO: 22; a 3C protease cleavage site comprising the amino acid sequence of SEQ ID NO: 50; an IRES comprising the nucleic acid sequence of SEQ ID NO: 81 or SEQ ID NO: 82; a 5' untranslated region comprising the nucleic acid sequence of SEQ ID NO: 86 or SEQ ID NO: 87; a Kozak sequence comprising the nucleic acid sequence of SEQ ID NO: 35; a 2A protease comprising the amino acid sequence of SEQ ID NO: 75; a severe fever with thrombocytopenia syndrome virus antigen comprising the amino acid sequence of SEQ ID NO: 45, SEQ ID NO: 85, SEQ ID NO:

116, or SEQ ID NO: 119; and a human papillomavirus (HPV) antigen comprising the amino acid sequence of SEQ ID NO: 139, SEQ ID NO: 142, SEQ ID NO: 145, or SEQ ID NO: 148.

In another aspect of the present invention, there is provided a method for preventing or treating a disease, comprising administering to a subject the polynucleotide, the recombinant vector, or the vaccine composition comprising the same.

**Effects of Invention**

Compared to a conventional mRNA and enterovirus-based self-amplifying RNA construct, an enterovirus-based optimized self-amplifying RNA construct according to the present invention increased the expression level of a target protein. In addition, the multiple-antigen self-amplifying RNA construct was able to express one or more target proteins simultaneously, and the expression level thereof was much higher than that of a conventional mRNA. Therefore, the self-amplifying RNA construct according to the present invention can be widely used for vaccines and the like.

**Brief Description of Drawings**

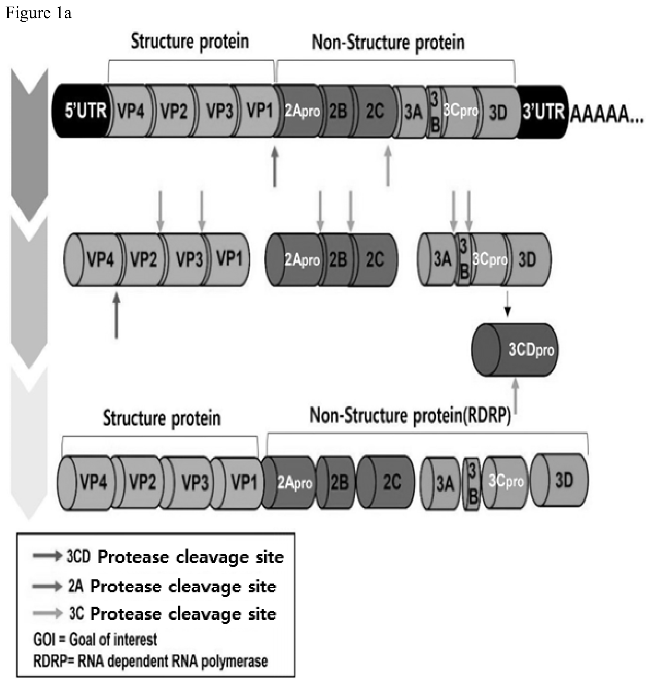

Figure 1a is a schematic diagram of the RNA structure of wild-type enterovirus.

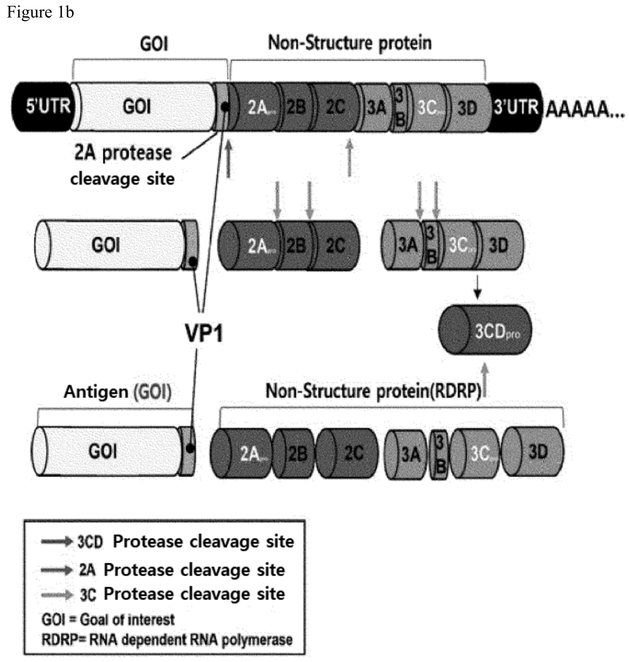

Figure 1b is a schematic diagram of the structure of an enterovirus-based self-amplifying RNA construct (saRNA).

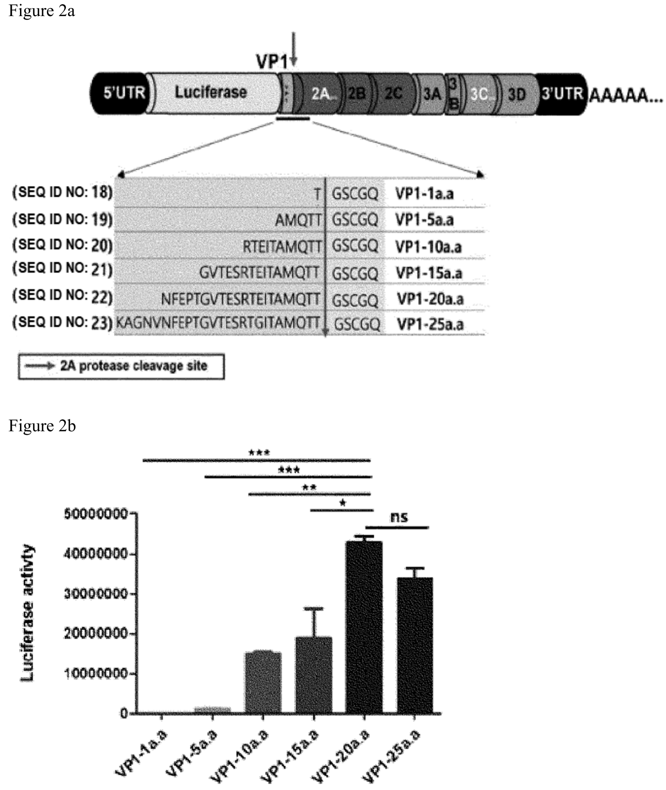

Figure 2a is a diagram showing the amino acid sequence of the 2A protease cleavage site included in an enterovirus-based self-amplifying RNA construct according to one embodiment of the present invention.

Figure 2b is a graph showing the results obtained by confirming the expression level of a target protein according to the amino acid sequence of the 2A protease cleavage site included in an enterovirus-based self-amplifying RNA construct according to one embodiment of the present invention, using a luciferase system. *p<0.05, **p<0.01, ***p<0.001, n.s: not significant

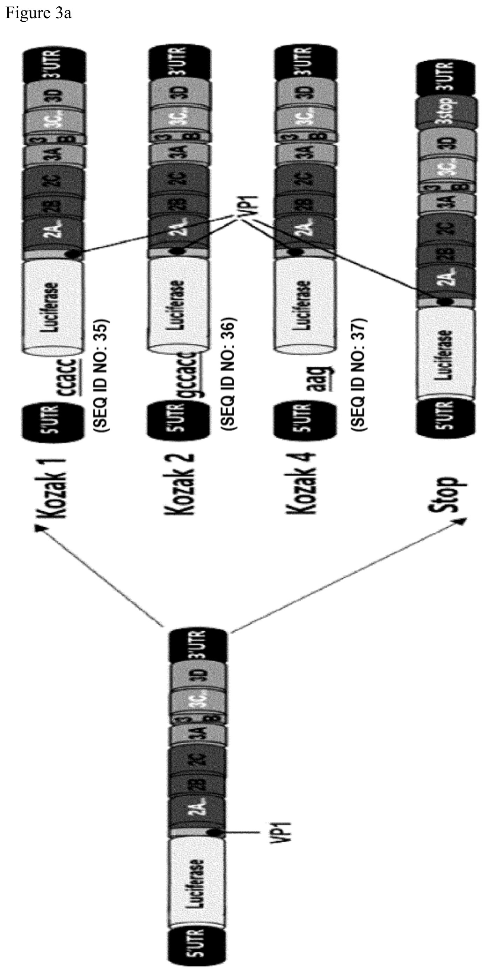

Figure 3a is a schematic diagram of the structure of an enterovirus-based self-amplifying RNA construct into which a Kozak sequence is inserted according to one embodiment of the present invention.

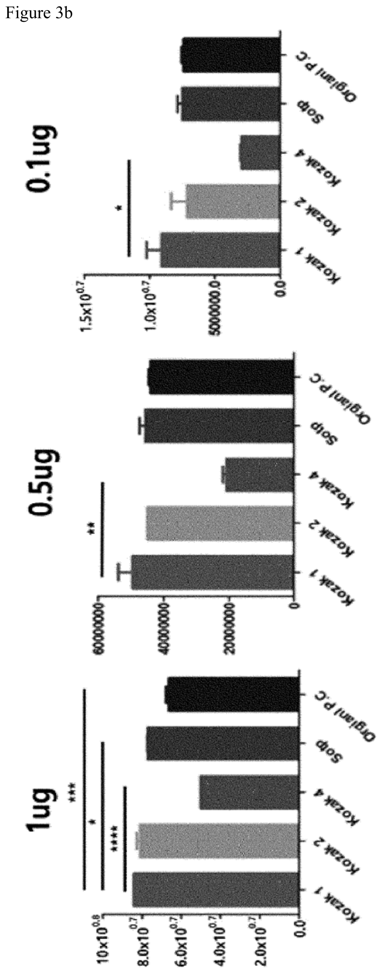

Figure 3b is a graph showing the results obtained by confirming the expression level of a target protein according to the inserted sequence in an enterovirus-based self-amplifying RNA construct into which a Kozak sequence is inserted according to one embodiment of the present invention, using a luciferase system. *p<0.05, **p<0.01, ***p<0.001, ****p<0.0001

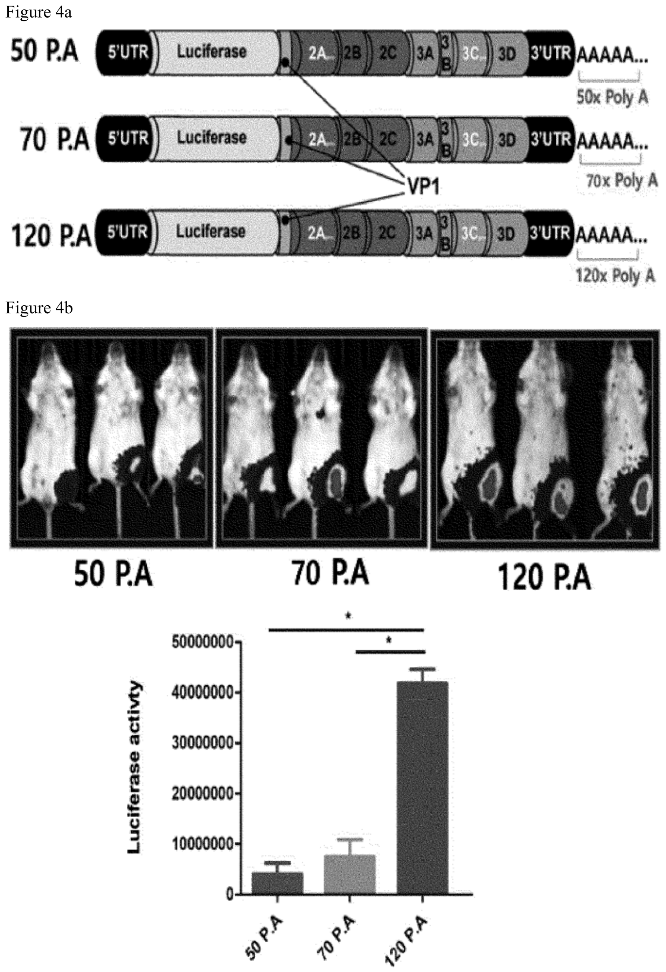

Figure 4a is a schematic diagram of the structure of an enterovirus-based self-amplifying RNA construct into which a poly(A) tail (poly(A) tail) is inserted according to one embodiment of the present invention.

Figure 4b is a graph showing the results obtained by confirming the expression level of a target protein according to each inserted sequence in an enterovirus-based self-amplifying RNA construct into which a poly(A) tail (poly(A) tail) is inserted according to one embodiment of the present invention at the cellular (bottom) and animal (top) levels, using a luciferase system. *p<0.05

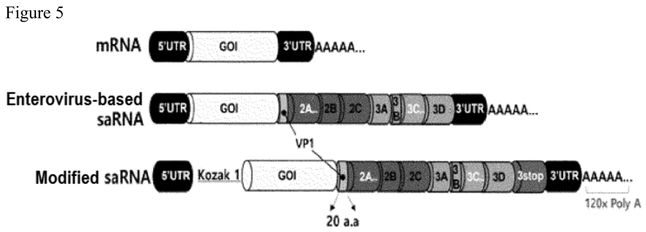

Figure 5 is a schematic diagram comparing the structures of mRNA, an enterovirus-based self-amplifying RNA construct, and a self-amplifying RNA (modified saRNA) construct optimized by modifying the enterovirus-based self-amplifying RNA construct according to one embodiment of the present invention.

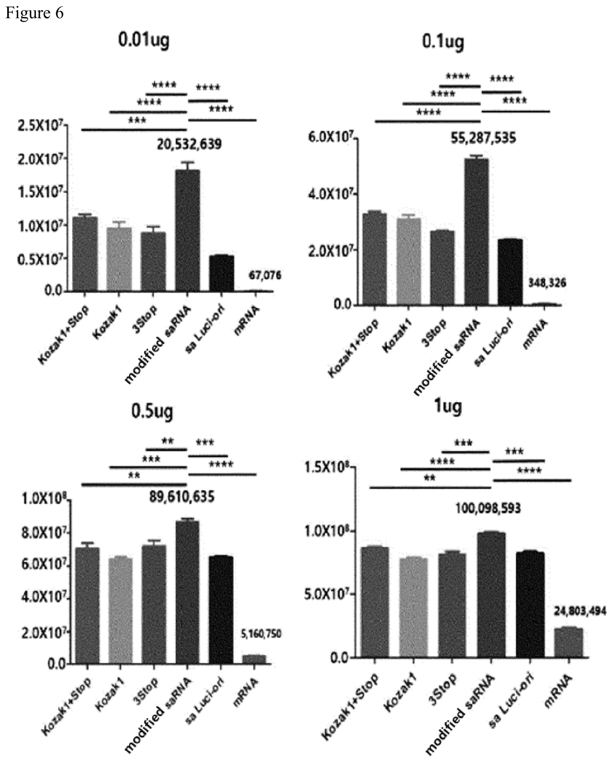

Figure 6 is a graph showing the results obtained by confirming the expression level of a target protein by mRNA, an enterovirus-based self-amplifying RNA (sa luci) construct, and a modified self-amplifying RNA construct according to one embodiment of the present invention at the cellular level, using a luciferase system. **p<0.01, ***p<0.001, ****p<0.0001

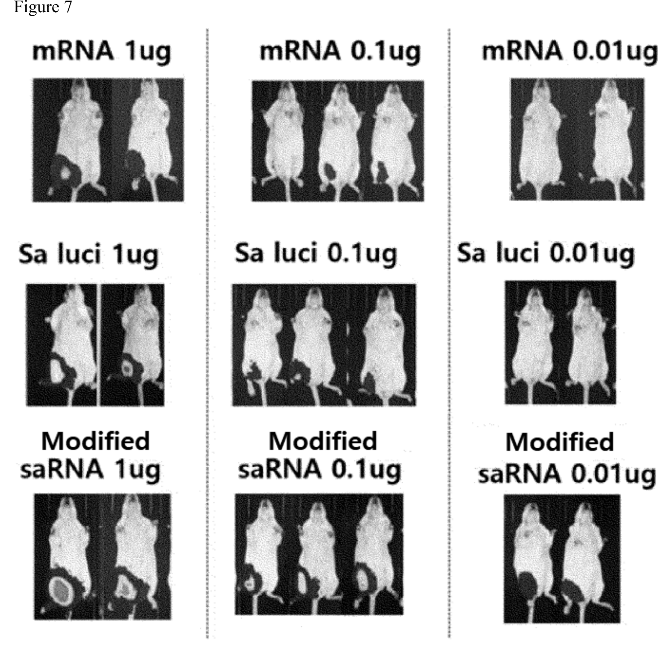

Figure 7 is a diagram showing the results obtained by confirming the expression level of a target protein by mRNA, an enterovirus-based self-amplifying RNA (sa luci) construct, and a modified self-amplifying RNA construct according to one embodiment of the present invention at the animal level, using a luciferase system.

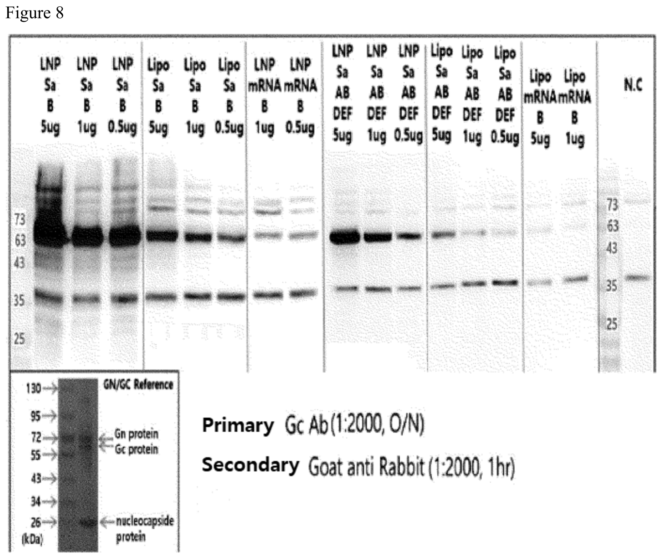

Figure 8 is a diagram showing the results obtained by confirming the expression level of an antigen protein at the cellular level of a modified self-amplifying RNA construct comprising a severe fever with thrombocytopenia syndrome (SFTS) virus antigen as a target protein used in one embodiment of the present invention by Western blot. Sa B: a self-amplifying RNA construct comprising a SFTS consensus B antigen, Sa ABDEF: a self-amplifying RNA construct comprising a SFTS consensus ABDEF antigen, mRNA B: mRNA comprising a SFTS consensus B antigen

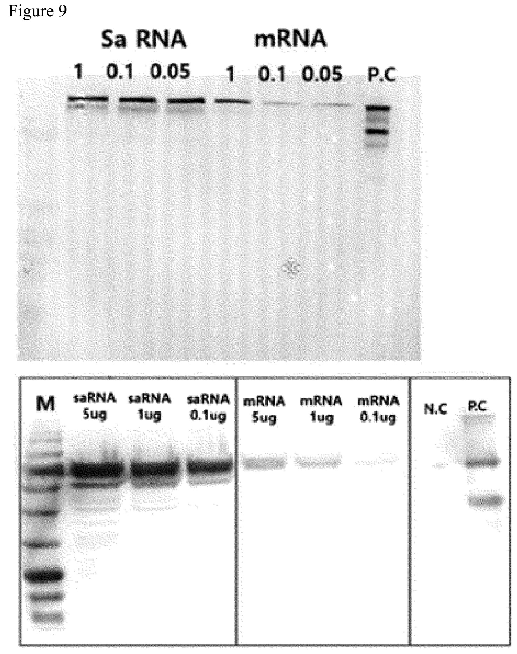

Figure 9 is a diagram showing the results obtained by confirming the expression level of an antigen protein at the cellular level of a modified self-amplifying RNA construct comprising the spike protein antigen of coronavirus (SARS-CoV2) (top) or the highly pathogenic avian influenza (HPAI) virus antigen (H5N8, bottom) as a target protein used in one embodiment of the present invention by Western blot.

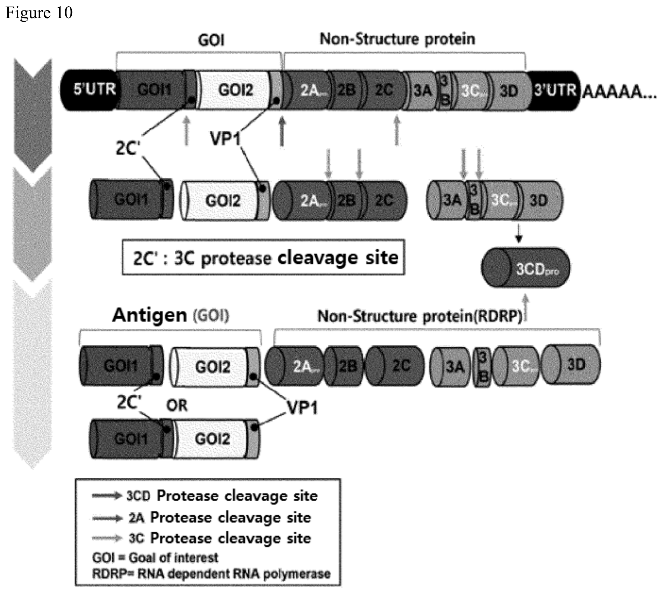

Figure 10 is a schematic diagram of the structure of a modified self-amplifying RNA construct comprising multiple antigens according to one embodiment of the present invention.

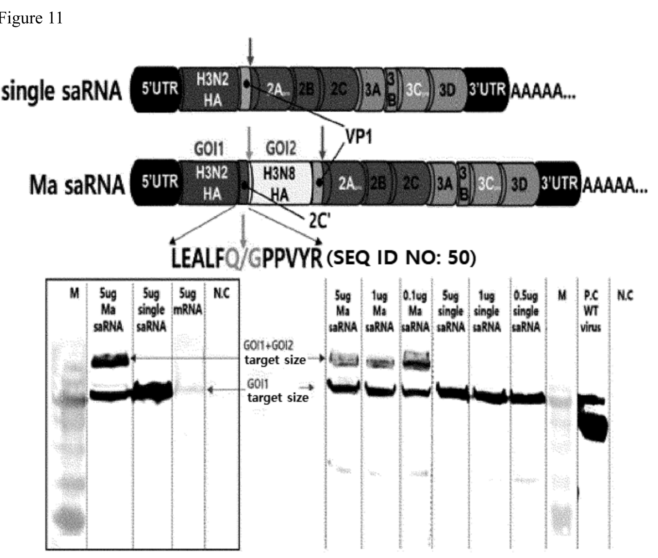

Figure 11 is a diagram showing a schematic diagram of a modified self-amplifying RNA construct comprising two types of canine influenza virus antigens (H3N2 and H3N8) as a target protein used in one embodiment of the present invention (top); and the results obtained by confirming the expression level of an antigen protein at the cellular level by Western blot (bottom). Ma saRNA: a modified self-amplifying RNA construct comprising H3N2 and H3N8, single saRNA: a modified self-amplifying RNA construct comprising H3N2, P.C WT virus: wild-type canine influenza virus

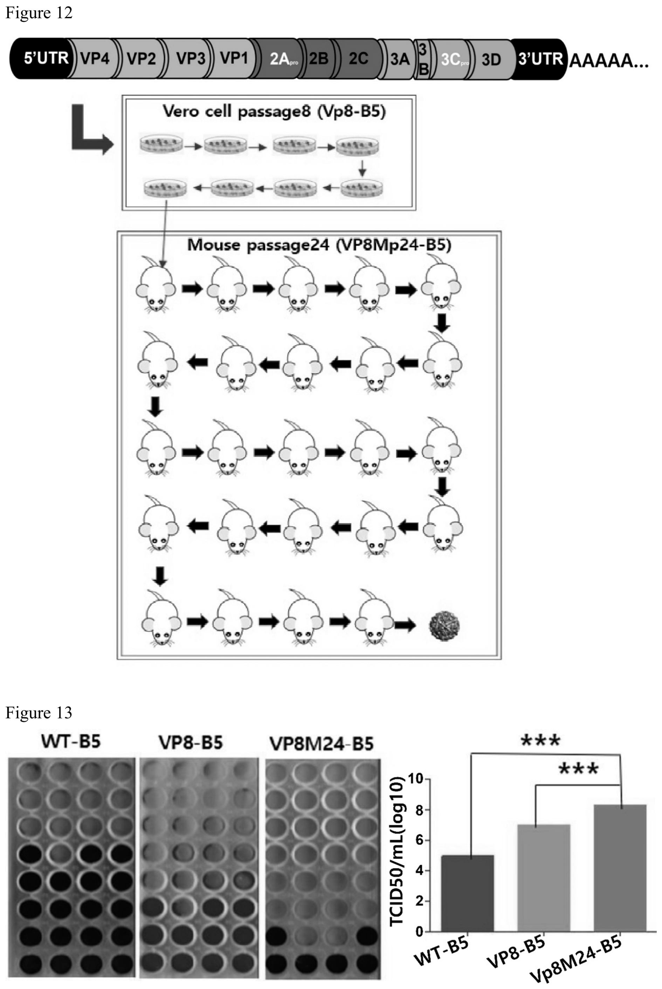

Figure 12 is a schematic diagram of a method for producing cell- and mouse-adapted coxsackievirus B5.

Figure 13 is a diagram (left) and a graph (right) showing the results obtained by measuring and comparing the titers of wild-type (WT-B5), cell-adapted (Vp8-B5), and cell- and mouse-adapted (Vp8Mp24-B5) coxsackievirus B5. ***$p<0.001$

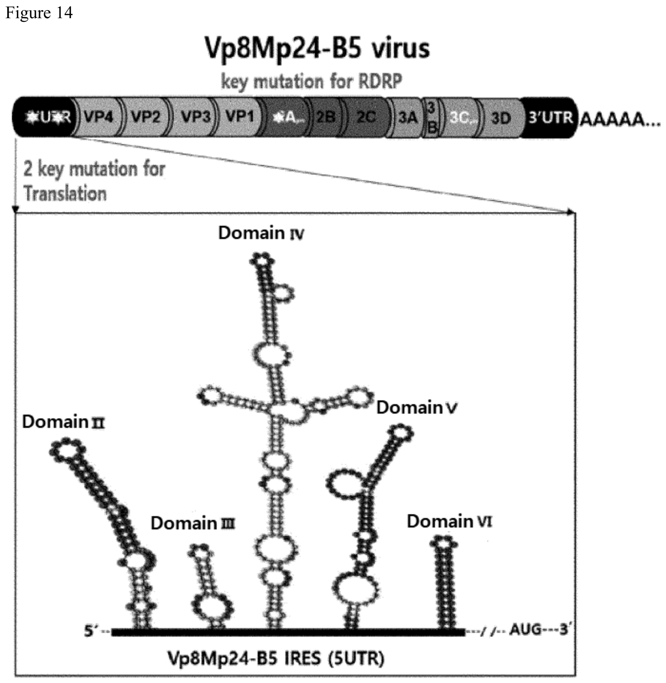

Figure 14 is a schematic diagram of the RNA structure of coxsackievirus B5 with an increased proliferation rate according to one embodiment of the present invention.

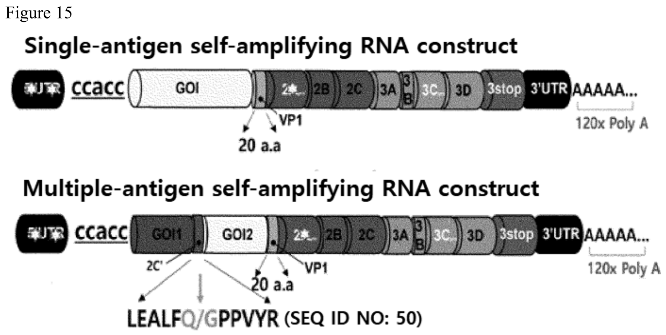

Figure 15 is a schematic diagram of the structure of an optimized enterovirus-based self-amplifying RNA construct according to one embodiment of the present invention.

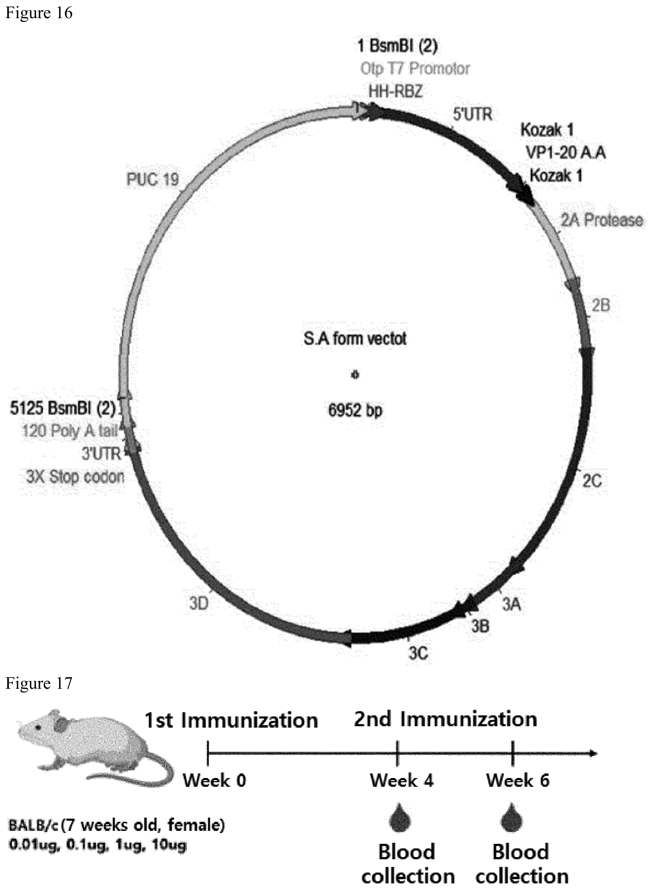

Figure 16 is a schematic diagram of the structure of a clone vector in the form of an enterovirus-based self-amplifying RNA construct used in the present invention.

Figure 17 is a diagram showing an animal experiment schedule for confirming the antiviral effect of a modified self-amplifying RNA or mRNA comprising a highly pathogenic influenza virus antigen as a target protein used in one embodiment of the present invention against a highly pathogenic influenza virus.

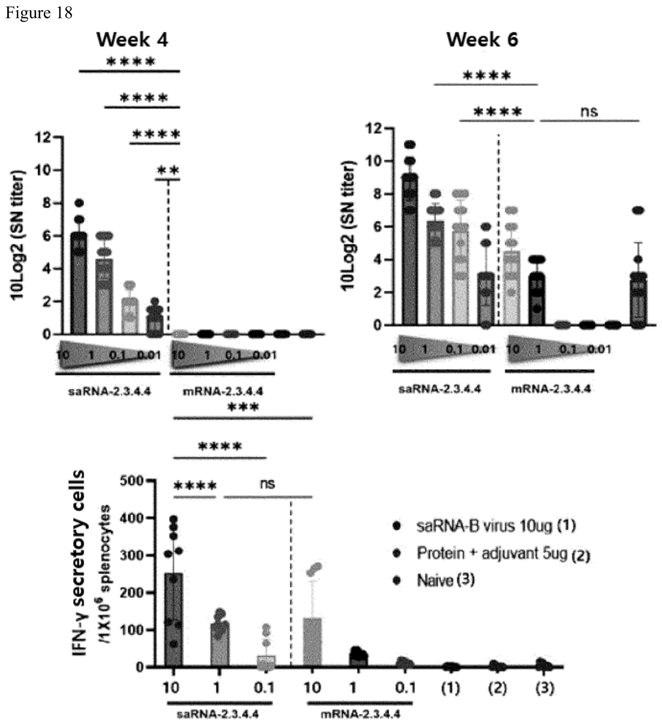

Figure 18 is a graph showing the results obtained by confirming the production of neutralizing antibodies (top) and T cell response (bottom) by extracting the spleen of mice after administration of a modified self-amplifying RNA or mRNA comprising a highly pathogenic influenza virus antigen as a target protein used in one embodiment of the present invention.

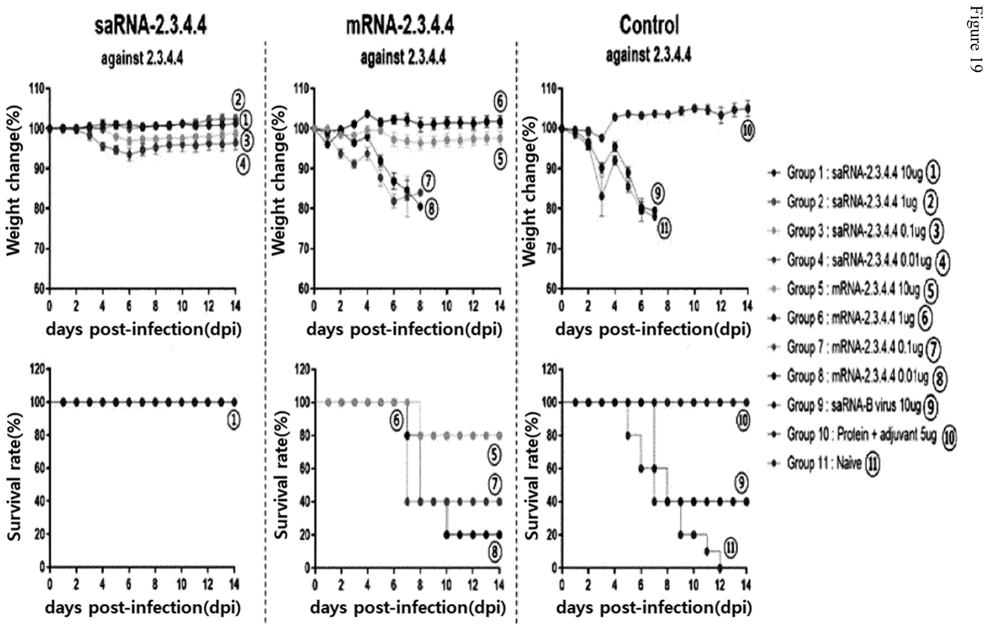

Figure 19 is a graph showing the results obtained by confirming the change in body weight and survival rate of mice after infecting the mice to which a modified self-amplifying RNA or mRNA comprising a highly pathogenic influenza virus antigen as a target protein used in one embodiment of the present invention was administered with a highly pathogenic influenza virus.

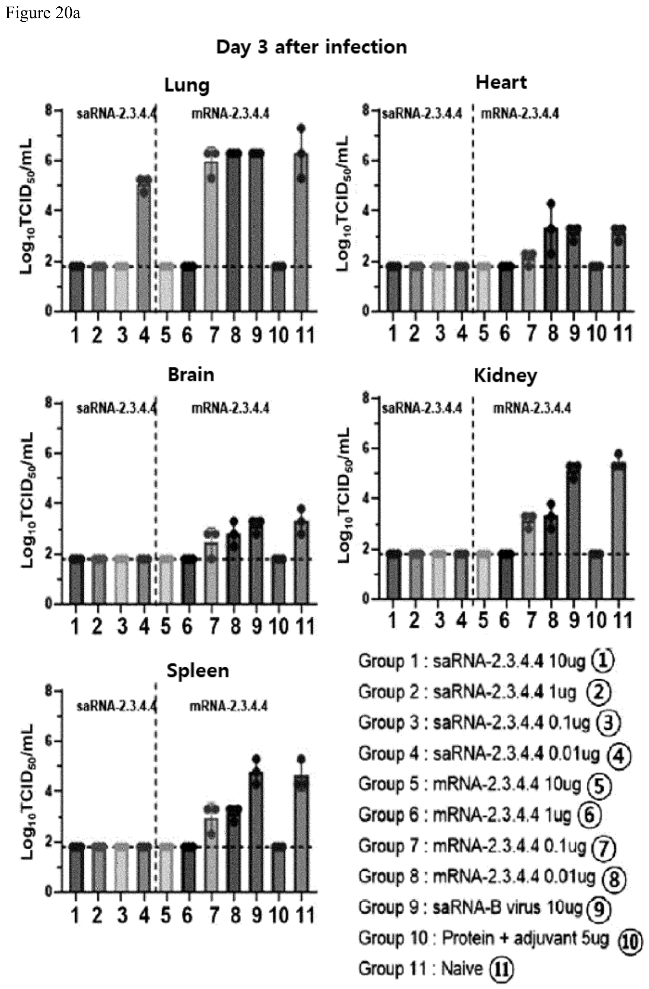

Figure 20a is a graph showing the results obtained by measuring the titer of the virus remaining in each organ by extracting the organs of mice (day 3) after infecting the mice to which a modified self-amplifying RNA or mRNA comprising a highly pathogenic influenza virus antigen as a target protein used in one embodiment of the present invention was administered with a highly pathogenic influenza virus.

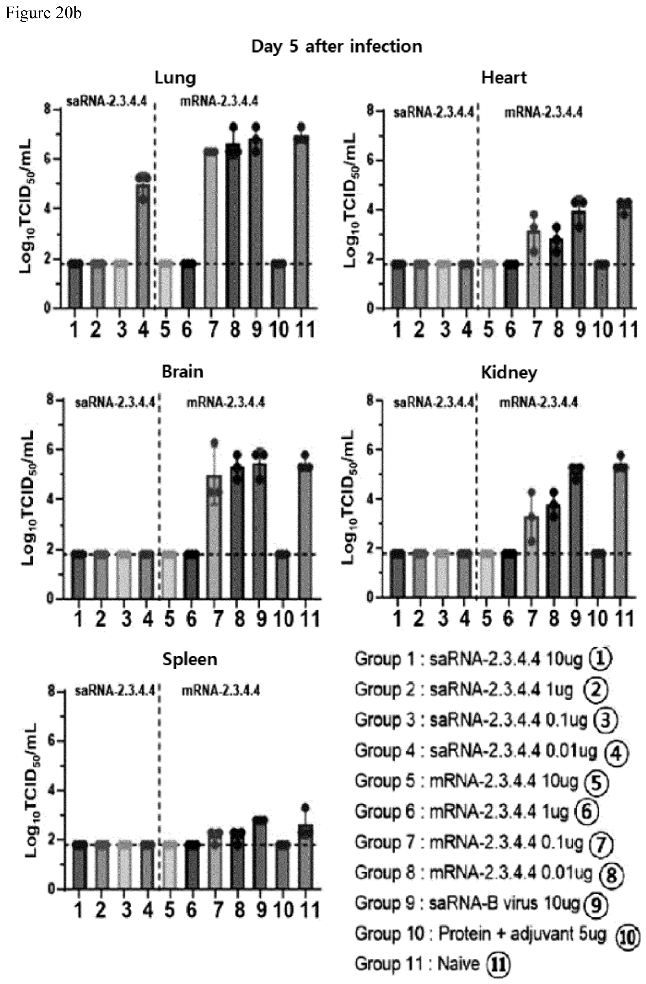

Figure 20b is a graph showing the results obtained by measuring the titer of the virus remaining in each organ by extracting the organs of mice (day 5) after infecting the mice to which a modified self-amplifying RNA or mRNA comprising a highly pathogenic influenza virus antigen as a target protein used in one embodiment of the present invention was administered with a highly pathogenic influenza virus.

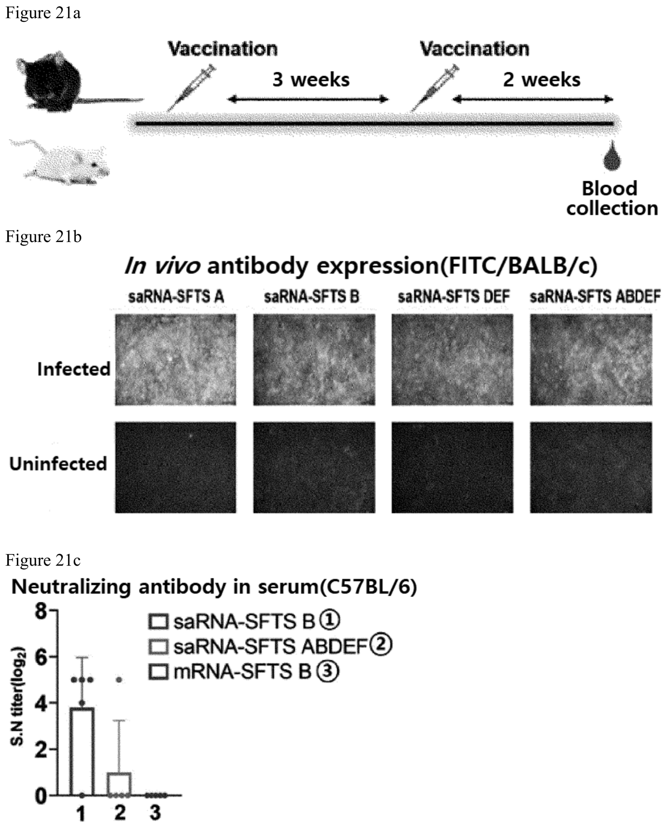

Figure 21a is a diagram showing an animal experiment schedule for confirming the neutralizing antibody production ability of a modified self-amplifying RNA or mRNA comprising a severe fever with thrombocytopenia syndrome virus antigen (SFTS) as a target protein used in one embodiment of the present invention.

Figure 21b is a diagram showing the results obtained by confirming whether neutralizing antibodies are produced in the blood of mice after administering a modified self-amplifying RNA comprising a severe fever with thrombocytopenia syndrome virus antigen (A antigen, B antigen, DEF antigen, ABDEF antigen) as a target protein used in one embodiment of the present invention to the mice (BALB/c mice) through immunofluorescence staining.

Figure 21c is a graph showing the results obtained by confirming whether neutralizing antibodies are produced in the blood of mice after administering a modified self-amplifying RNA (B antigen, ABDEF antigen) or mRNA (B antigen) comprising a severe fever with thrombocytopenia syndrome virus antigen (SFTS) as a target protein used in one embodiment of the present invention to the mice (C57BL6 mice).

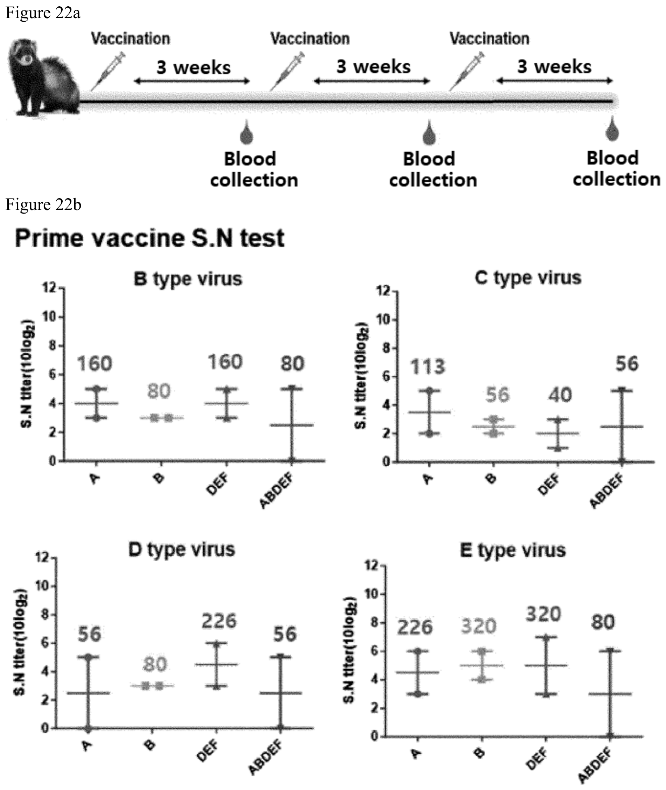

Figure 22a is a schematic diagram showing an animal experiment (ferret) schedule for confirming the neutralizing antibody production ability of a modified self-amplifying RNA comprising a severe fever with thrombocytopenia syndrome virus antigen (A antigen, B antigen, DEF antigen, ABDEF antigen) as a target protein used in one embodiment of the present invention.

Figure 22b is a graph showing the results obtained by confirming whether neutralizing antibodies are produced in the blood of a ferret at 3 weeks after the first administration of a modified self-amplifying RNA comprising a severe fever with thrombocytopenia syndrome virus antigen (A antigen, B antigen, DEF antigen, ABDEF antigen) as a target protein used in one embodiment of the present invention.

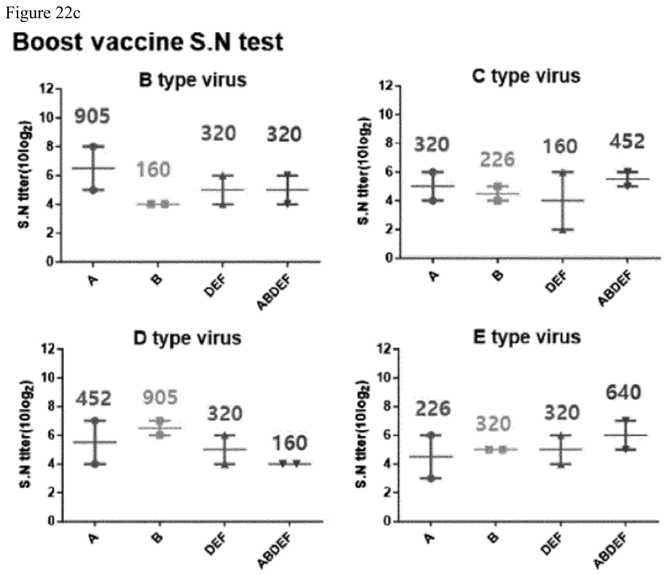

Figure 22c is a graph showing the results obtained by confirming whether neutralizing antibodies are produced in the blood of a ferret at 3 weeks after the second administration of a modified self-amplifying RNA comprising a severe fever with thrombocytopenia syndrome virus antigen (A antigen, B antigen, DEF antigen, ABDEF antigen) as a target protein used in one embodiment of the present invention.

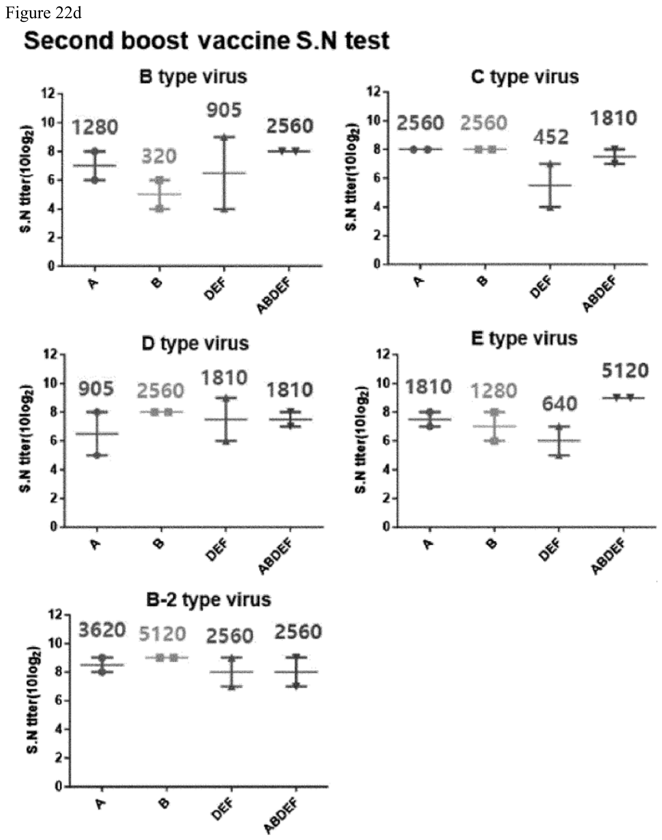

Figure 22d is a graph showing the results obtained by confirming whether neutralizing antibodies are produced in the blood of a ferret at 3 weeks after the third administration of a modified self-amplifying RNA comprising a severe fever with thrombocytopenia syndrome virus antigen (A antigen, B antigen, DEF antigen, ABDEF antigen) as a target protein used in one embodiment of the present invention.

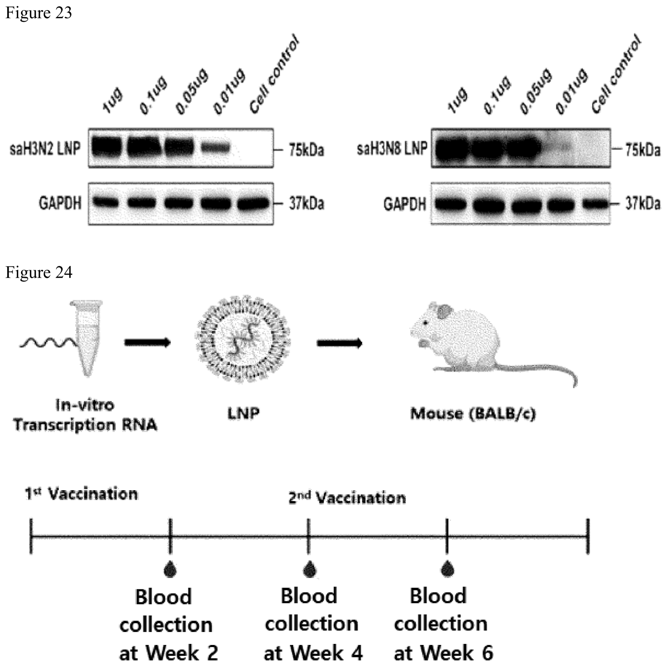

Figure 23 is a diagram showing the results obtained by confirming the expression level of a modified self-amplifying RNA comprising a canine influenza virus antigen (H3N2 or H3N8) as a target protein used in one embodiment of the present invention according to the concentration at the cellular level by Western blot.

Figure 24 is a diagram showing an animal experiment schedule for confirming the neutralizing antibody production ability of a modified single-antigen self-amplifying RNA (H3N2 or H3N8) or multiple-antigen self-amplifying RNA (H3N2 and H3N8) comprising a canine influenza virus antigen as a target protein used in one embodiment of the present invention.

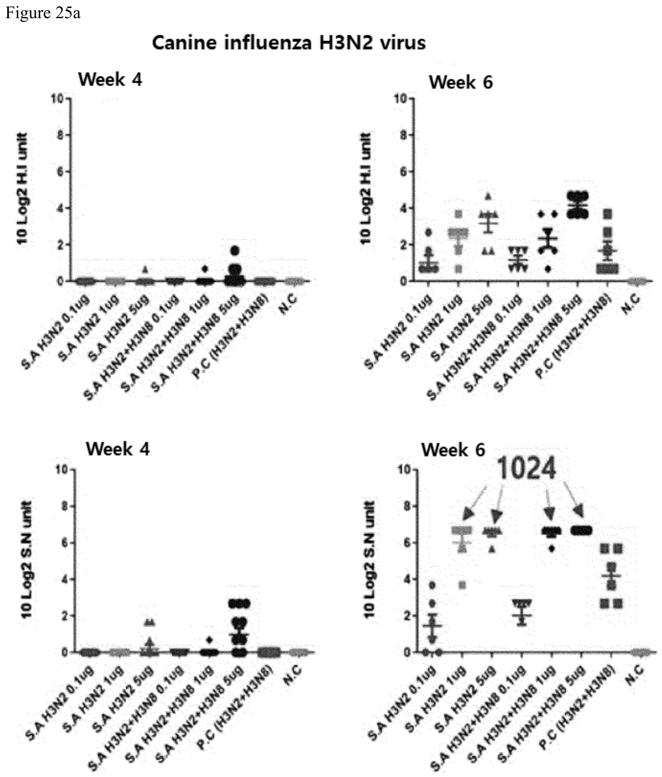

Figure 25a is a graph showing the results obtained by analyzing the production of neutralizing antibodies in the blood of mice by confirming binding to inactivated canine influenza H3N2 virus at 4 weeks and 6 weeks (2 weeks after the second administration) after administering a modified single-antigen self-amplifying RNA (H3N2 or H3N8) or multiple-antigen self-amplifying RNA (H3N2 and H3N8) comprising a canine influenza virus antigen as a target protein used in one embodiment of the present invention to the mice.

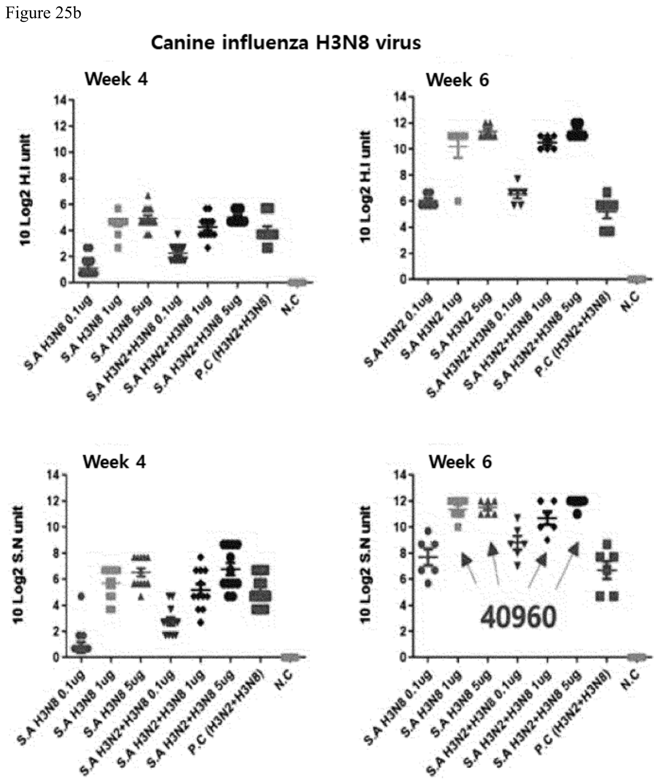

Figure 25b is a graph showing the results obtained by analyzing the production of neutralizing antibodies in the blood of mice by confirming binding to inactivated canine influenza H3N8 virus at 4 weeks and 6 weeks (2 weeks after the second administration) after administering a modified single-antigen self-amplifying RNA (H3N2 or H3N8) or multiple-antigen self-amplifying RNA (H3N2 and H3N8) comprising a canine influenza virus antigen as a target protein used in one embodiment of the present invention to the mice.

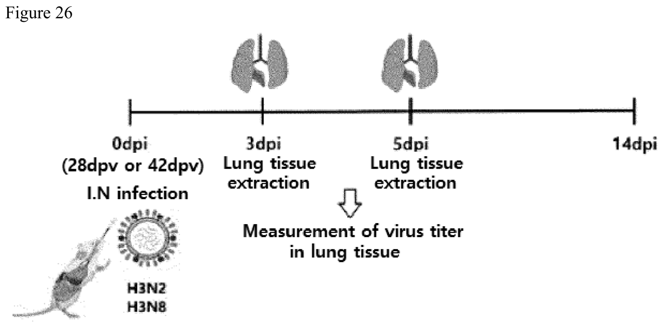

Figure 26 is a diagram showing an animal experiment schedule for evaluating the antiviral activity against canine influenza after administering a modified single-antigen self-amplifying RNA (H3N2 or H3N8) or multiple-antigen self-amplifying RNA (H3N2 and H3N8) comprising a canine influenza virus antigen as a target protein used in one embodiment of the present invention to mice, and then infecting the mice with H3N2 or H3N8 virus at 4 weeks and 6 weeks (2 weeks after the second administration).

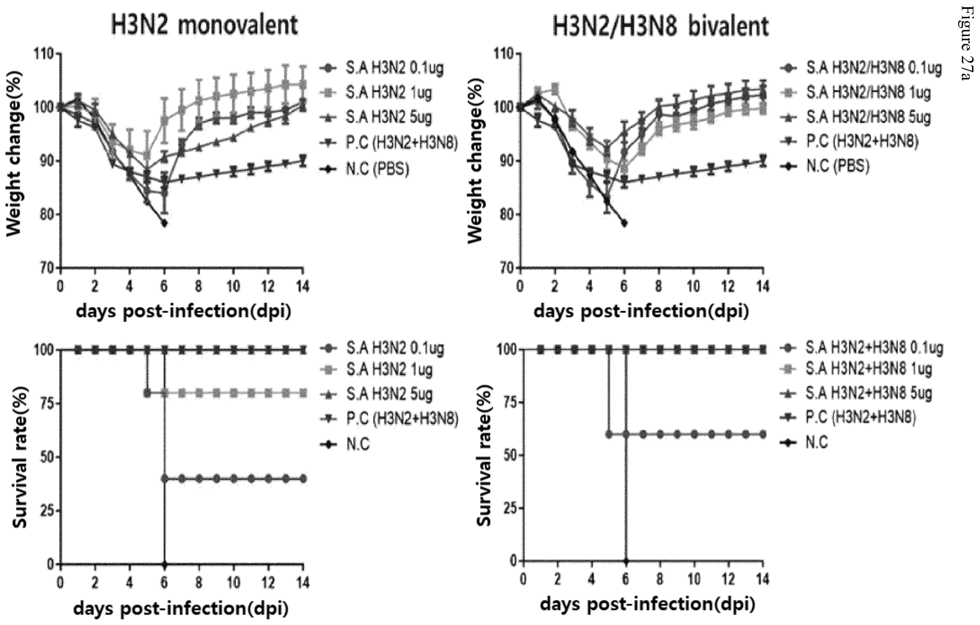

Figure 27a is a graph showing the results obtained by confirming the change in body weight and survival rate of mice after administering a modified single-antigen self-amplifying RNA (H3N2) or multiple-antigen self-amplifying RNA (H3N2 and H3N8) comprising a canine influenza virus antigen as a target protein used in one embodiment of the present invention to the mice, and then infecting the mice with a canine influenza H3N2 virus at 4 weeks.

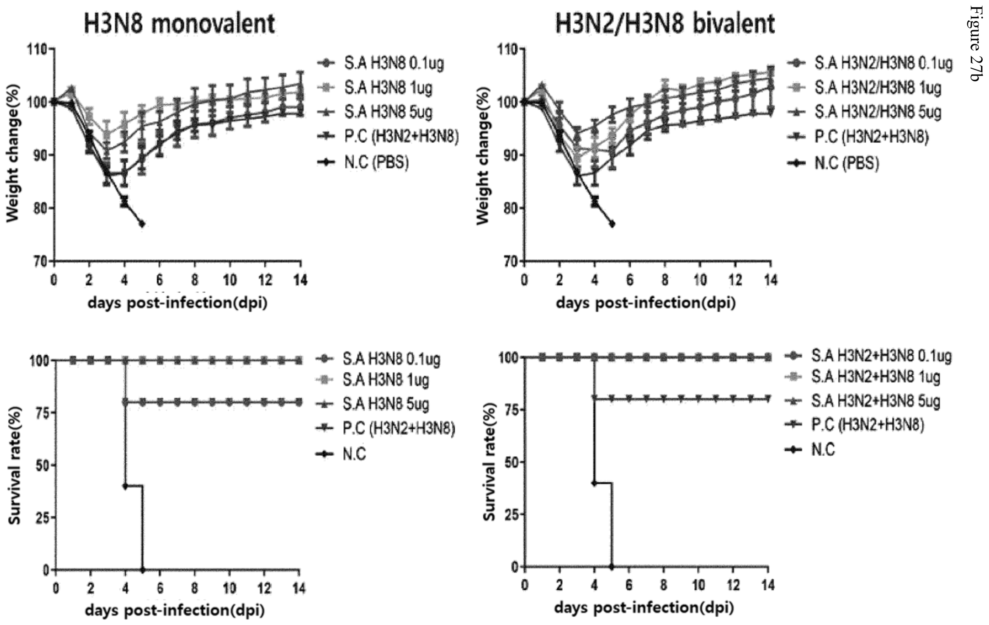

Figure 27b is a graph showing the results obtained by confirming the change in body weight and survival rate of mice after administering a modified single-antigen self-amplifying RNA (H3N8) or multiple-antigen self-amplifying RNA (H3N2 and H3N8) comprising a canine influenza virus antigen as a target protein used in one embodiment of the present invention to the mice, and then infecting the mice with a canine influenza H3N8 virus at 4 weeks.

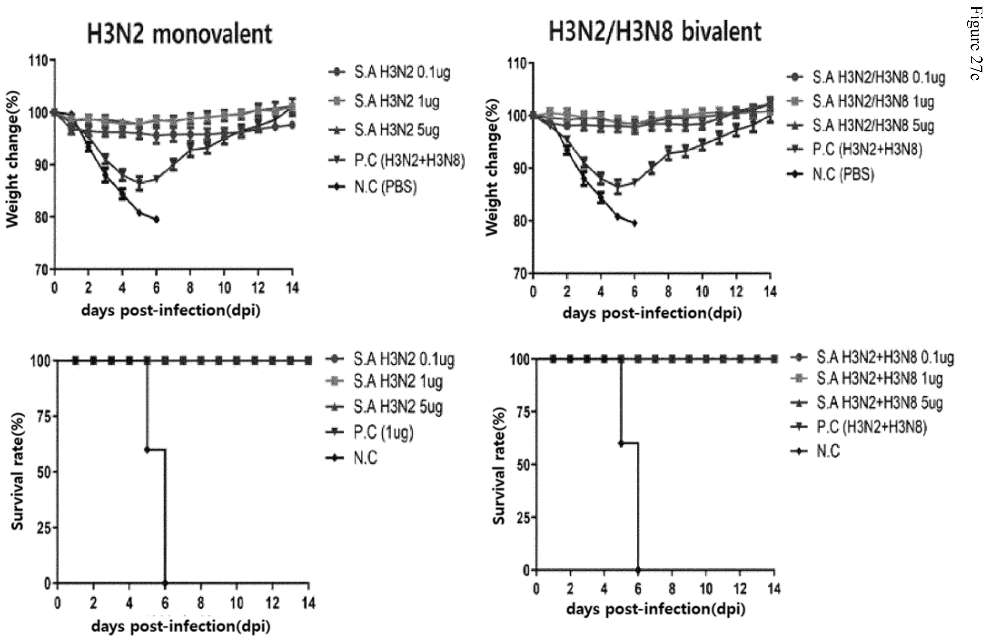

Figure 27c is a graph showing the results obtained by confirming the change in body weight and survival rate of mice after administering a modified single-antigen self-amplifying RNA (H3N2) or multiple-antigen self-amplifying RNA (H3N2 and H3N8) comprising a canine influenza virus antigen as a target protein used in one embodiment of the present invention to the mice, and then infecting the mice with a canine influenza H3N2 virus at 6 weeks (2 weeks after the second administration).

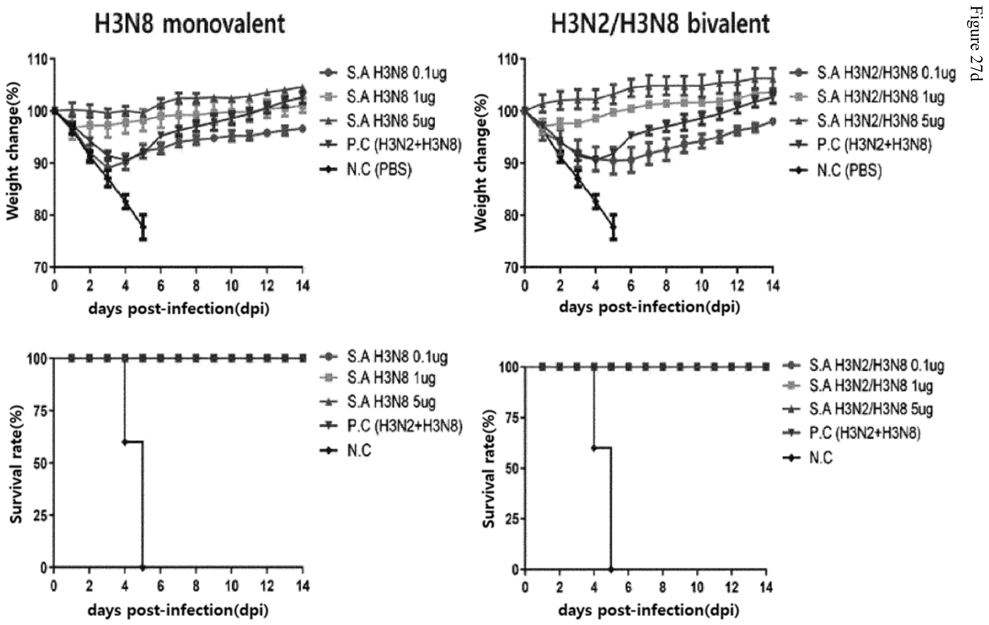

Figure 27d is a graph showing the results obtained by confirming the change in body weight and survival rate of mice after administering a modified single-antigen self-amplifying RNA (H3N8) or multiple-antigen self-amplifying RNA (H3N2 and H3N8) comprising a canine influenza virus antigen as a target protein used in one embodiment of the present invention to the mice, and then infecting the mice with a canine influenza H3N8 virus at 6 weeks (2 weeks after the second administration).

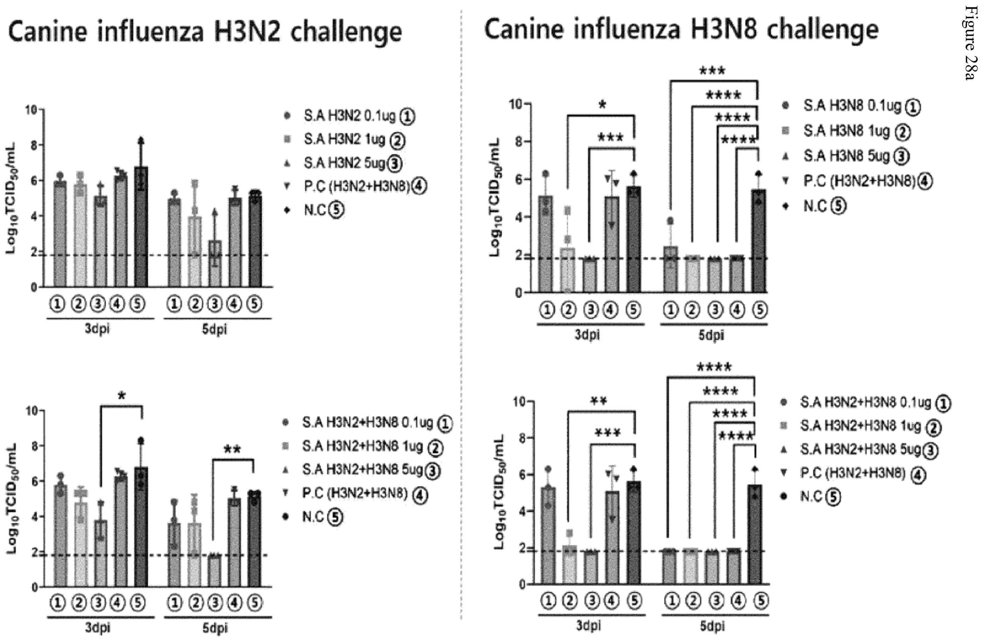

Figure 28a is a graph showing the results obtained by measuring the titer of the virus remaining in the lung tissue of mice on the 3rd day after administering a modified single-antigen self-amplifying RNA (H3N2) or multiple-antigen self-amplifying RNA (H3N2 and H3N8) comprising a canine influenza virus antigen as a target protein used in one embodiment of the present invention to the mice, and then infecting the mice with a H3N2 or H3N8 virus.

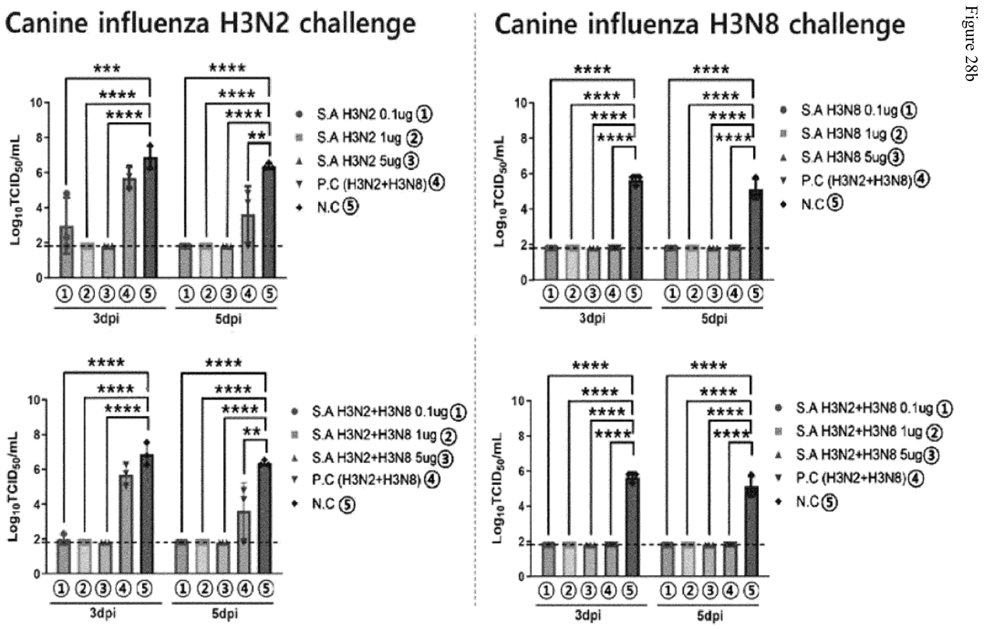

Figure 28b is a graph showing the results obtained by measuring the titer of the virus remaining in the lung tissue of mice on the 5th day after administering a modified single-antigen self-amplifying RNA (H3N2) or multiple-antigen self-amplifying RNA (H3N2 and H3N8) comprising a canine influenza virus antigen as a target protein used in one embodiment of the present invention to the mice, and then infecting the mice with a H3N2 or H3N8 virus.

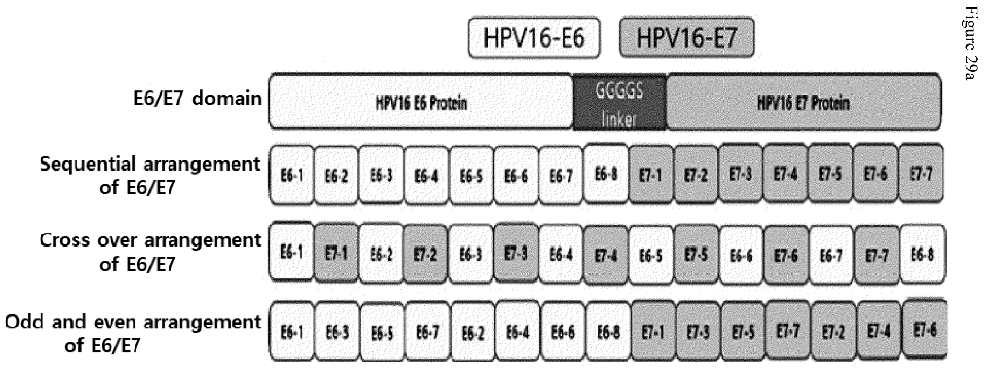

Figure 29a is a schematic diagram of a human papillomavirus (HPV) antigen (E6/E7 domain, sequential arrangement of E6/E7, cross over arrangement of E6/E7, odd and even arrangement of E6/E7).

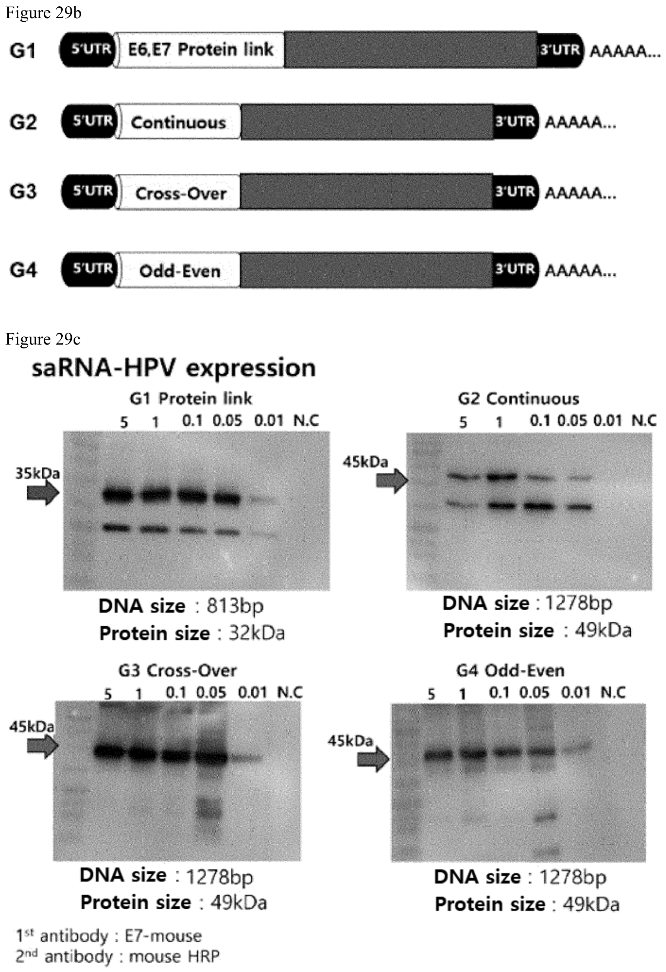

Figure 29b is a schematic diagram of a modified self-amplifying RNA comprising a human papillomavirus (HPV) antigen (E6/E7 domain, sequential arrangement of E6/E7, cross over arrangement of E6/E7, odd and even arrangement of E6/E7) as a target protein. G1 E6,E7 protein linker: E6/E7 domain, G2 Continuous: sequential arrangement of E6/E7, G3 Cross over: cross over arrangement of E6/E7, G4 Odd-Even: odd and even arrangement of E6/E7

Figure 29c is a diagram showing the results obtained by confirming the expression of a modified self-amplifying RNA comprising a human papillomavirus (HPV) antigen (E6/E7 domain, sequential arrangement of E6/E7, cross over arrangement of E6/E7, odd and even arrangement of E6/E7) as a target protein at the cellular level by Western blot.

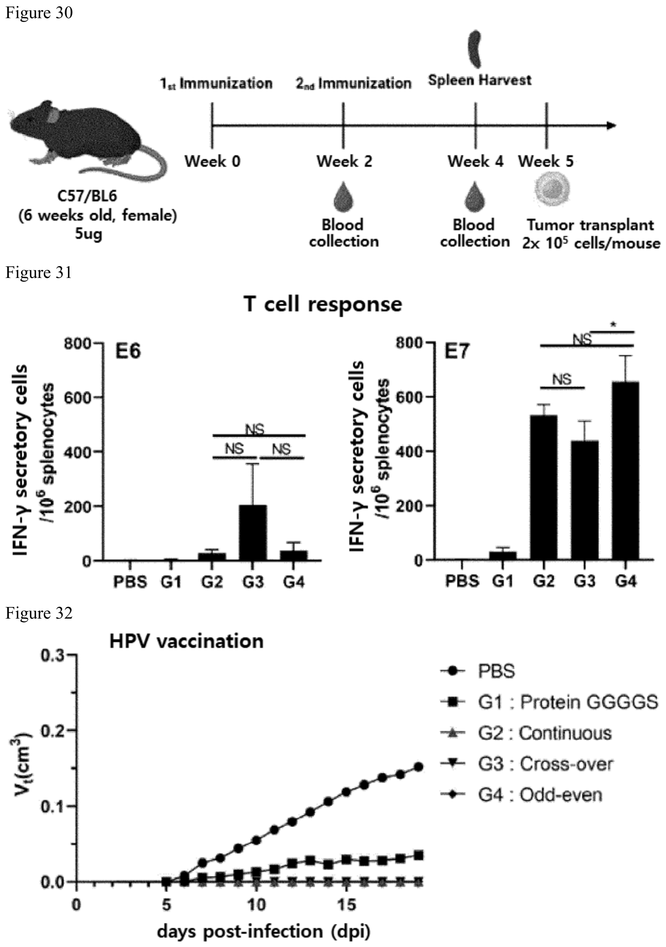

Figure 30 is a diagram showing an animal experiment schedule for confirming the cancer prevention activity of a modified self-amplifying RNA comprising a human papillomavirus (HPV) antigen (E6/E7 domain, sequential arrangement of E6/E7, cross over arrangement of E6/E7, odd and even arrangement of E6/E7) as a target protein.

Figure 31 is a graph showing the results obtained by confirming the T cell response in the spleen of mice at 4 weeks (2 weeks after the second administration) after administration of a modified self-amplifying RNA comprising a human papillomavirus (HPV) antigen (E6/E7 domain, sequential arrangement of E6/E7, cross over arrangement of E6/E7, odd and even arrangement of E6/E7) as a target protein.

Figure 32 is a graph showing the results obtained by measuring the tumor size after administering a modified self-amplifying RNA comprising a human papillomavirus (HPV) antigen (E6/E7 domain, sequential arrangement of E6/E7, cross over arrangement of E6/E7, odd and even arrangement of E6/E7) as a target protein to mice, and then transplanting the mice with TC-1 tumor cells, which are E6 and E7 gene-expressing cells of human papillomavirus (type 16), at 5 weeks (3 weeks after the second administration).

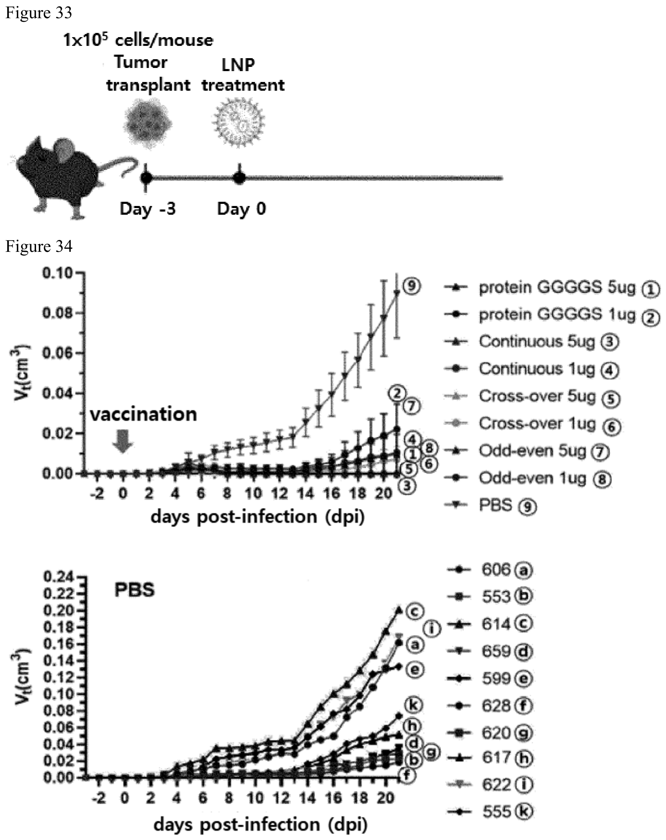

Figure 33 is a diagram showing an animal experiment schedule for confirming the tumor growth inhibitory activity of modified self-amplifying RNA (4 types) comprising a human papillomavirus (HPV) antigen (E6/E7 domain, sequential arrangement of E6/E7, cross over arrangement of E6/E7, odd and even arrangement of E6/E7) as a target protein.

Figure 34 is a graph showing the results obtained by measuring the tumor size after transplanting mice with TC-1 tumor cells, which are E6 and E7 gene-expressing cells of human papillomavirus (type 16), and then administering modified self-amplifying RNA (4 types) comprising a human papillomavirus (HPV) antigen (E6/E7 domain, sequential arrangement of E6/E7, cross over arrangement of E6/E7, odd and even arrangement of E6/E7) as a target protein at the 3rd day. At this time, PBS is the control.

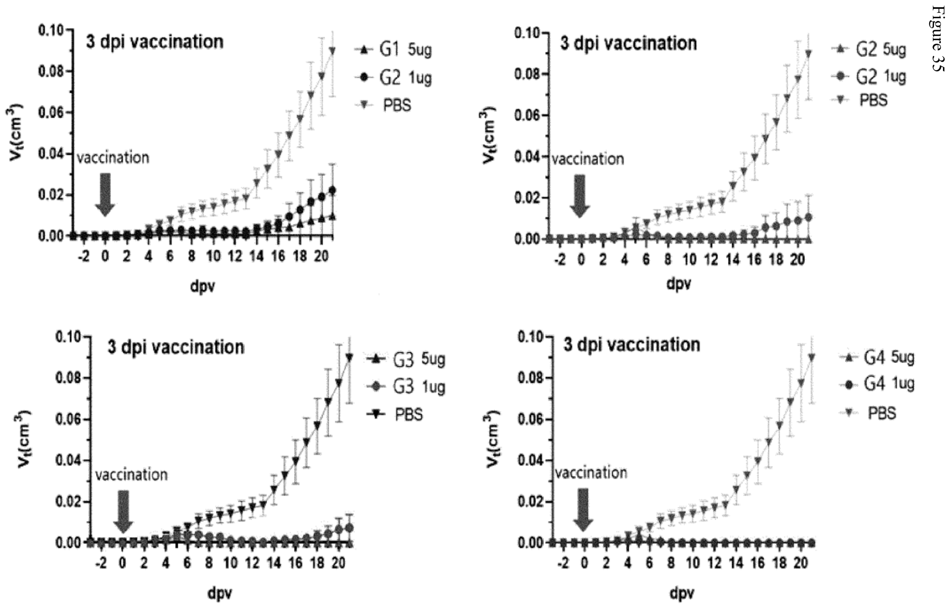

Figure 35 is a graph showing the results obtained by measuring the tumor size by administration group after transplanting mice with TC-1 tumor cells, which are E6 and E7 gene-expressing cells of human papillomavirus (type 16), and then administering modified self-amplifying RNA (4 types) comprising a human papillomavirus (HPV) antigen (E6/E7 domain, sequential arrangement of E6/E7, cross over arrangement of E6/E7, odd and even arrangement of E6/E7) as a target protein at the 3rd day. At this time, PBS is the control.

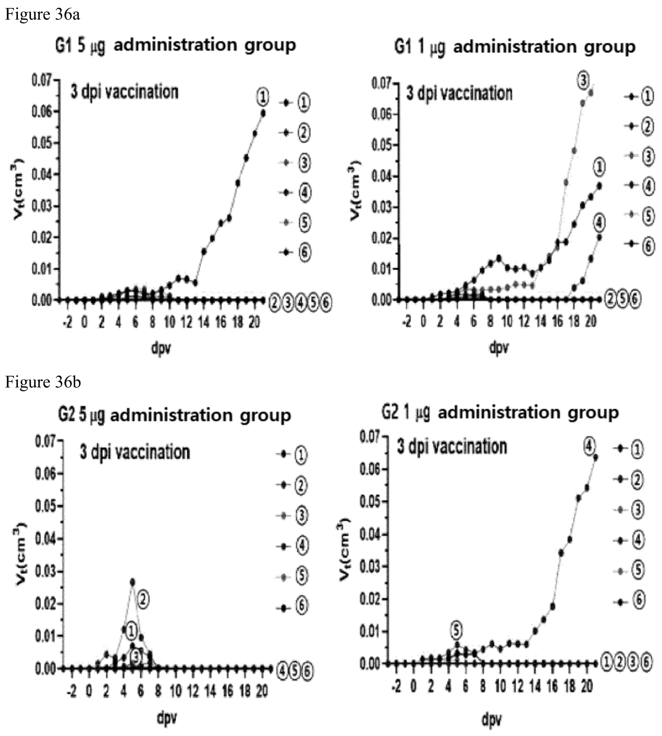

Figure 36a is a graph showing the results obtained by measuring the tumor size by mouse subject after transplanting mice with TC-1 tumor cells, which are E6 and E7 gene-expressing cells of human papillomavirus (type 16), and then administering a modified self-amplifying RNA (G1) comprising a human papillomavirus (HPV) antigen (E6/E7 domain) as a target protein, and PBS at the 3rd day. G1: protein GGGGS

Figure 36b is a graph showing the results obtained by measuring the tumor size by mouse subject after transplanting mice with TC-1 tumor cells, which are E6 and E7 gene-expressing cells of human papillomavirus (type 16), and then administering a modified self-amplifying RNA (G2) comprising a human papillomavirus (HPV) antigen (sequential arrangement of E6/E7) as a target protein, and PBS at the 3rd day. G2: Continuous

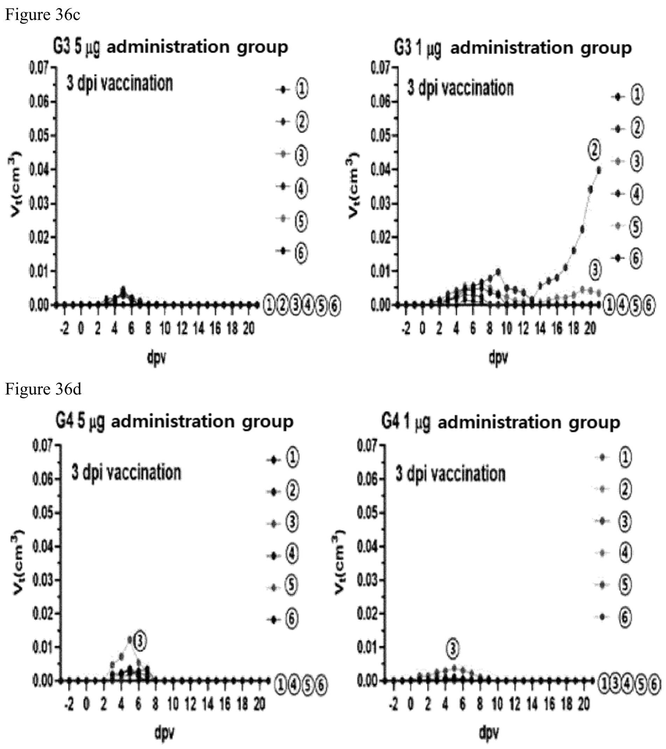

Figure 36c is a graph showing the results obtained by measuring the tumor size by mouse subject after transplanting mice with TC-1 tumor cells, and then administering a modified self-amplifying RNA (G3) comprising a human papillomavirus (HPV) antigen (cross over arrangement of E6/E7) as a target protein and PBS at the 3rd day. G3: Cross over

Figure 36d is a graph showing the results obtained by measuring the tumor size by mouse subject after transplanting mice with TC-1 tumor cells, and then administering a modified self-amplifying RNA (G4) comprising a human papillomavirus (HPV) antigen (odd and even arrangement of E6/E7) as a target protein, and PBS at the 3rd day. G4: Odd-Even

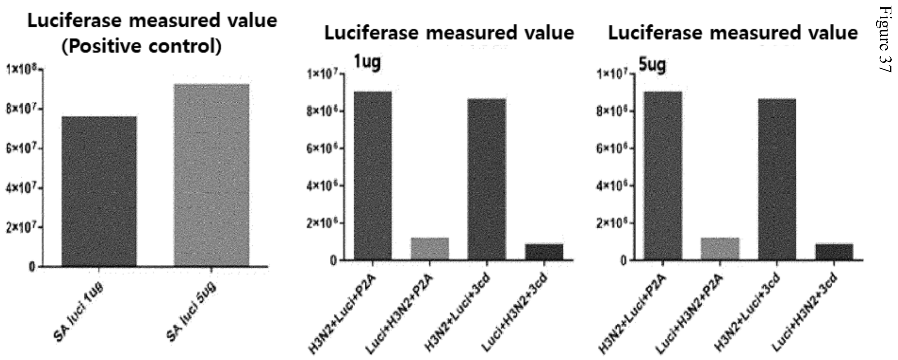

Figure 37 is a graph showing the results obtained by confirming the target protein expression level of a multiple-antigen self-amplifying RNA construct (first target protein: luciferase, second target protein: canine influenza H3N2 virus antigen) or a single-antigen self-amplifying RNA construct (target protein: luciferase) according to one embodiment of the present invention using a luciferase system.

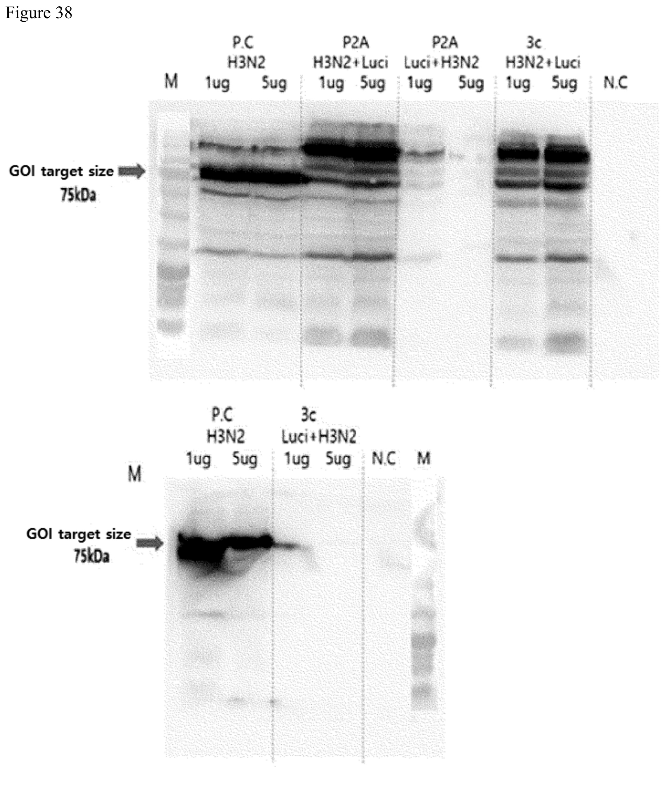

Figure 38 is a diagram showing the results obtained by confirming the target protein expression level of the multiple-antigen self-amplifying RNA construct of Figure 37 by Western blot. N.C: negative control, P.C.: positive control (wild-type H3N2 virus)

## Best Mode for Carrying out the Invention

## Polynucleotide construct

**Non-structural protein**

In one aspect of the present invention, there is provided a polynucleotide comprising a nucleic acid sequence encoding a fusion protein, comprising: a non-structural protein of a self-amplifying virus; a first target protein; and a protease cleavage site. At this time, the polynucleotide may be DNA or RNA. In addition, the self-amplifying virus may be a virus of the family Picornaviridae. Specifically, the self-amplifying virus may be a virus of the genus Enterovirus. More specifically, in the present invention, the non-structural protein may be a non-structural protein derived from enterovirus. Preferably, it may be a non-structural protein derived from a coxsackievirus.

As used herein, the term "polynucleotide" refers to a single- or double-stranded sequence of nucleotides, in which the 3' and 5' terminus of each nucleotide are linked by phosphodiester bonds. The polynucleotide may be composed of deoxyribonucleic acids (DNA) or ribonucleic acids (RNA).

In the present specification, the polynucleotide comprises DNA and RNA, and may be produced synthetically *in vitro* or isolated from natural sources. The size of the polynucleotide is typically expressed in the number of base pairs (bp) for a double-stranded polynucleotide, or in the number of nucleotides (nt) for a single-stranded polynucleotide.

In the present specification, the nucleic acid sequence is used in the same sense as the polynucleotide, and may be DNA or RNA. At this time, the RNA may comprise mRNA. In addition, the nucleic acid sequence may further comprise modified DNA or RNA, for example, methylated DNA or RNA, or RNA that has been subjected to post-translational modifications, 3' processing such as cleavage and polyadenylation, and splicing. The nucleic acid may also comprise synthetic nucleic acid (XNA), hexitol nucleic acid (HNA), cyclohexene nucleic acid (CeNA), threose nucleic acid (TNA), glycerol nucleic acid (GNA), locked nucleic acid (LNA), and peptide nucleic acid (PNA).

As used herein, the term "enterovirus" refers to a virus belonging to the genus Enterovirus of the family Picornaviridae and having a size of about 24 nm to about 30 nm without an envelope. It has an icosahedral shape and comprises a single-stranded positive sense RNA having a size of about 7.2 kb to about 7.5 kb as its genetic material. Enteroviruses consist of three serotypes (PV: 1-3) of poliovirus; 23 serotypes of coxsackievirus group A (CVA: 1-22, 24); six serotypes of coxsackievirus group B (CVB: 1-6); 28 serotypes of echovirus group (ECV: 1-7, 9, 11-21, 24-27, 29-33), and other human enteroviruses (EV: 68-116).

The enterovirus enters the host cell, releases RNA into the cytoplasm, and then functions as mRNA itself to initiate translation at the IRES (internal ribosomal entry site) of the 5' untranslated region (UTR), thereby inducing the production of a large polyprotein. The genetic material of the enterovirus consists of one open reading frame (ORF) and an untranslated region (UTR); or non-coding region (NCR) that is not expressed as a protein at the 5' and 3' terminus. The ORF accounts for about 90% of the entire gene and is expressed as one polyprotein. The polyprotein consists of about 2,185 amino acids and is divided into several different proteins by the viral proteolytic enzyme.

The above one polyprotein is divided into a P1 domain, a P2 domain, and a P3 domain, and the P1 domain is called a structural protein, and the P2 domain and the P3 domain are called a non-structural protein. The P1 domain of the above polyprotein is the capsid protein of the virus and encodes the components VP4, VP2, VP3 and VP1 in order from the N-terminus to the C-terminus, and the capsid is composed of a 32-mer capsomer. The P2 domain encodes 2A protease (2A$^{pro}$), 2B, and 2C in order from the N-terminus to the C-terminus, and the P3 domain encodes 3A, 3B (VPg protein), 3C protease (3C$^{pro}$), and 3D polymerase (3D$^{pol}$) in that order. The 2A protease and the 3C protease are proteolytic enzymes that serve to recognize and cleave cleavage sites within the viral polyprotein, thereby processing it into individual proteins. Meanwhile, the 3D polymerase is an RNA-dependent RNA polymerase (RdRp) that serves to synthesize complementary RNA using RNA as a template during viral RNA self-replication.

In the present invention, the enterovirus may be poliovirus (PV; 1-3), rhinovirus (RV), enterovirus (A71, A76, A89-A92, A120, B69, B73, B74, B75, B77, B78, B80-B88, B93, B97, B98, B100, B101, B106, C99, C105, C109, C116, D68, D94, D111), coxsackievirus (A1-A22, A24, B1-B6, C96), echovirus (E2-E7, E9, E11-E21, E24-E27, E29-E33), and other human enteroviruses (70, 79, 107, C104). Preferably, the enterovirus of the present invention may be coxsackievirus B5.

In the present invention, the non-structural protein derived from enterovirus may comprise a P2 domain and a P3 domain. At this time, the non-structural protein may comprise a P2 domain and a P3 domain in order from the N-terminus to the C-terminus.

In the present invention, the P2 domain may comprise 2A protease, 2B, and 2C. Specifically, the P2 domain of the present invention may comprise 2A protease, 2B, and 2C of coxsackievirus B5. At this time, the P2 domain may comprise 2A protease, 2B, and 2C in order from the N-terminus to the C-terminus. In one embodiment, the 2A protease, 2B, and 2C may comprise the amino acid sequences of SEQ ID NO: 11, SEQ ID NO: 12, and SEQ ID NO: 13, respectively. Therefore, in the present invention, the polynucleotide may comprise or consist of a nucleic acid sequence encoding the amino acid sequences of SEQ ID NO: 11, SEQ ID NO: 12, and SEQ ID NO: 13, respectively, in order from the 5' terminus to the 3' terminus. In one embodiment of the present invention, when the polynucleotide is DNA, it may comprise or consist of the nucleotide sequences of SEQ ID NO: 2, SEQ ID NO: 3, and SEQ ID NO: 4, respectively, in order from the 5' terminus to the 3' terminus. When the polynucleotide is RNA, may comprise or consist of the nucleotide sequences of SEQ ID NO: 61, SEQ ID NO: 62, and SEQ ID NO: 63 in order from the 5' terminus to the 3' terminus.

In addition, in the present invention, the 2A protease, 2B, and 2C may be composed of an amino acid sequence in which one or several amino acids are deleted, substituted and/or inserted, as long as they have the same biological function. Specifically, the 2A protease, 2B, and 2C may comprise or consist of an amino acid sequence having about 90%, about 91%, about 92%, about 93%, about 94%, about 95%, about 96%, about 97%, about 98%, about 99%, or about 100% identity to the amino acid sequences of SEQ ID NO: 11, SEQ ID NO: 12, and SEQ ID NO: 13, respectively. At this time, the deletion, substitution and/or insertion of the amino acid may not cause changes in the characteristics of the original protein, such as the secondary structure and activity.

In the present invention, the 2A protease may further comprise a variation.

As used herein, the term "variation" refers to a form in which a part of an amino acid or a polynucleotide encoding an amino acid is substituted compared to a wild-type. In the present invention, the variant may refer to a protein or nucleic acid (polynucleotide) comprising the variation.

That is, in the present invention, the 2A protease variant may have an amino acid sequence different from that of the wild-type 2A protease. However, the 2A protease variant may have an activity equivalent to or similar to that of the wild-type 2A protease or an activity that is further improved. The 2A protease variant may have an increased protein expression level compared to the wild-type. Here, "2A protease activity" may mean specifically recognizing and cleaving a cleavage site within an amino acid sequence.

Specifically, the 2A protease variant may be a form in which a part of an amino acid of the wild-type 2A protease is substituted. One specific example of a 2A protease variant by amino acid substitution may be one in which the 87th amino acid in the amino acid sequence of SEQ ID NO: 11 is substituted with another amino acid.

At this time, the "another amino acid" introduced by the substitution may be any one selected from the group consisting of alanine, arginine, asparagine, aspartic acid, cysteine, glutamic acid, glutamine, histidine, isoleucine, leucine, methionine, phenylalanine, proline, serine, threonine, tryptophan, tyrosine, and valine, excluding glycine.

In one embodiment of the present invention, the 2A protease variant may be one in which the 87th amino acid, glycine, in the amino acid sequence of SEQ ID NO: 11 is substituted with serine (G87S). Preferably, it may comprise the amino acid sequence of SEQ ID NO: 75. Therefore, in the present invention, the polynucleotide may comprise or consist of a nucleic acid sequence encoding the amino acid sequence of SEQ ID NO: 75 as the 2A protease variant. Specifically, when the polynucleotide is DNA, the nucleic acid sequence may comprise or consist of the nucleotide sequence of SEQ ID NO: 73. When the polynucleotide is RNA, the nucleic acid sequence may comprise or consist of the nucleotide sequence of SEQ ID NO: 74.

In addition, the 2A protease variant may comprise or consist of an amino acid sequence having about 90%, about 91%, about 92%, about 93%, about 94%, about 95%, about 96%, about 97%, about 98%, about 99%, or about 100% identity to the amino acid sequence of SEQ ID NO: 75.

In the present invention, the non-structural protein P3 domain may comprise 3A, 3B, 3C protease, and 3D polymerase. Specifically, the P3 domain of the present invention may comprise 3A, 3B, 3C protease, and 3D polymerase of coxsackievirus B5. At this time, the P3 domain may comprise 3A, 3B, 3C protease, and 3D polymerase in order from the N-terminus to the C-terminus.

In one embodiment, the 3A, 3B, 3C protease, and 3D polymerase may comprise the amino acid sequences of SEQ ID NO: 14, SEQ ID NO: 15, SEQ ID NO: 16, and SEQ ID NO: 17, respectively. Therefore, in the present invention, the polynucleotide may sequentially comprise or consist of a nucleic acid sequence encoding the amino acid sequences of SEQ ID NO: 14, SEQ ID NO: 15, SEQ ID NO: 16, and SEQ ID NO: 17, respectively, at the 3' terminus of a nucleic acid sequence encoding an amino acid sequence of 2C. In one embodiment of the present invention, when the polynucleotide is DNA, the nucleic acid sequence may sequentially comprise or consist of the nucleotide sequences of SEQ ID NO: 5, SEQ ID NO: 6, SEQ ID NO: 7, and SEQ ID NO: 8, respectively, at the 3' terminus of a nucleic acid sequence encoding an amino acid sequence of 2C, and when the polynucleotide is RNA, it may comprise or consist of the nucleotide sequences of SEQ ID NO: 64, SEQ ID NO: 65, SEQ ID NO: 66, and SEQ ID NO: 67, respectively.

In addition, the 3A, 3B, 3C protease, and 3D polymerase may be composed of an amino acid sequence in which one or several amino acids are deleted, substituted and/or inserted, as long as they have the same biological function or the gene positions encoding the 3A, 3B, 3C protease, and 3D polymerase on the chromosome are the same. Specifically, it may comprise or consist of an amino acid sequence having about 90%, about 91%, about 92%, about 93%, about 94%, about 95%, about 96%, about 97%, about 98%, about 99%, or about 100% identity to the amino acid sequences of SEQ ID NO: 14 to SEQ ID NO: 17, respectively.

**Target protein**

In the present invention, the polynucleotide may comprise a nucleic acid sequence encoding at least one or one or more target proteins. At this time, the target protein may be linked to a protease cleavage site.

As used herein, the term "target protein" may be various proteins for medical or industrial purposes, and may comprise protein fragments, peptides and variants. Industrially useful target proteins comprise hormones, hormone analogues, enzymes, enzyme inhibitors, cytokines, coagulation factors, transport proteins, receptors, receptor fragments, attachment proteins, regulatory proteins, structural proteins, toxic proteins, transcription factors, antigens, antibodies, antibody fragments, monoclonal antibodies, and the like. Preferably, it may be an antigen, but is not limited thereto. The antigen may be any antigenic protein associated with infection or disease. Examples of the antigen may comprise a tumor antigen, an animal antigen, a plant antigen, a virus antigen, a bacterial antigen, a fungal antigen, a protozoan antigen, an autoimmune antigen, or an allergy antigen, etc. At this time, the antigen may be a surface antigen of a tumor cell, or a protein and peptide derived from a secreted form of a viral pathogen, a bacterial pathogen, a fungal pathogen, or a protozoan pathogen, respectively.

In the present invention, the viral pathogen may be isolated from or derived from, for example, filovirus, adenovirus, enterovirus, severe fever with thrombocytopenia syndrome virus (SFTSV; bunyavirus), arbovirus, astrovirus, coronavirus, coxsackievirus, cytomegalovirus, Dengue virus, Epstein-Barr virus, hepatitis virus, herpesvirus, human immunodeficiency virus, human papillomavirus (HPV), human T-lymphotropic virus, influenza virus, canine influenza virus, highly pathogenic avian influenza (HPAI) virus, JC virus, lymphocytic choriomeningitis virus, measles virus, molluscum contagiosum virus, mumps virus, norovirus, parovirus, poliovirus, rabies virus, respiratory syncytial virus, rhinovirus, rotavirus, rubella virus, smallpox virus, varicella zoster virus, West Nile virus, zika virus, and the like.

In the present invention, the bacterial pathogen may be isolated from or derived from, for example, Campylobacter jejuni, Escherichia coli, Helicobacter pylori, Mycobacterium tuberculosis, Neisseria gonorrhoeae, Neisseria meningitides, Salmonella, Shigella, Staphylococcus aureus, Streptococcus, and the like. In the present invention, the fungal pathogen may be isolated from or derived from, for example, Coccidioides immitis, Blastomyces dermatitidis, Cryptococcus neoformans, Candida species, Aspergillus species, and the like.

In the present invention, the protozoan pathogen may be isolated from or derived from, for example, Plasmodium, Leishmania, Trypanosome, Cryptosporidiums, Isospora, Naegleria fowleri, Acanthamoeba, Balamuthia mandrillaris, Toxoplasma gondii, Pneumocystis carinii, and the like.

As used herein, the term "tumor antigen" refers to an antigen presented by a class I major histocompatibility complex on the surface of a tumor cell. Tumor-specific antigens presented only by tumor cells are generally generated by mutations in the tumor. The tumor antigen refers to a tumor-specific antigen. Common tumor antigens comprise tumor-associated antigens that are expressed more in tumor cells than in normal cells. When cytotoxic T cells (Tc) recognize tumor-associated antigens, they may destroy tumor cells before rapid proliferation or metastasis of the tumor cells occurs.

In the present invention, the tumor antigen may be isolated from or derived from cystitis, breast cancer, colon and rectal cancer, endometrial cancer, renal cancer, leukemia, lung cancer, melanoma, non-Hodgkin's lymphoma, pancreatic cancer, prostate cancer, thyroid cancer, and the like. For example, it may be 5T4, 707-AP, 9D7, AFP, AlbZIP HPG1, $\alpha$-5-$\beta$-1-integrin, $\alpha$-5-$\beta$-6-integrin, $\alpha$-actinin-4/m, $\alpha$-methylacyl-coA racemase, ART-4, ARTC1/m, B7H4, BAGE-1, BCL-2, ber/abl, B-catenin/m, BING-4, BRCA1/m, BRCA2/m, CA 15-3/CA 27-29, CA 19-9, CA72-4, CA125, calreticulin, CAMEL, CASP-8/m, cathepsin B, cathepsin L, CD19, CD20, CD22, CD25, CD30, CD33, CD4, CD52, CD55, CD56, CD80, CDC27/m, CDK4/m, CDKN2A/m, CEA, CLCA2, CML28, CML66, COA-1/m, coactosin-like protein (COTL1), collagen type XXIII, COX-2, CT_9/BRD6, Cten, cyclin B1, cyclin D1, CypB, CYPB1, DAM-10, DAM-6, DEK-CAN, EFTUD2/m, EGFR, ELF2/m, EMMPRIN, EpCam, EphA2, EphA3, ErbB3, ETV6-AML1, EZH2, FGF-5, FN, Frau-1, G250, GAGE-1, GAGE-2, GAGE-3, GAGE-4, GAGE-5, GAGE-6, GAGE7b, GAGE-8, GDEP, GnT-V, gp100, GPC3, GPNMB/m, HAGE, HAST-2, hepsin, Her2/neu, HERV-K-MEL, HLA-A*0201-R17I, HLA-A11/m, HLA-A2/m, HNE, NKX3.1, HOM-TES-14/SCP-1, HOM-TES-85, HPV-E6, HPV-E7, HSP70-2M, HST-2, hTERT, iCE, IGF-1R, IL-13Ra2, IL-2R, IL-5, immature laminin Receptor, kallikrein-2(KLK2), kallikrein-4(LKL4), Ki67, KIAA0205, KIAA0205/m, KK-LC-1, K-Ras/m, LAGE-A1, LDLR-FUT, MAGE-A1, MAGE-A2, MAGE-A3, MAGE-A4, MAGE-A6, MAGE-A9, MAGE-A10, MAGE-A12, MAGE-B1, MAGE-B2, MAGE-B3, MAGE-B4, MAGE-B5, MAGE-B6, MAGE-B10, MAGE-B16, MAGE-B17, MAGE-C1, MAGE-C2, MAGE-C3, MAGE-D1, MAGE-D2, MAGE-D4, MAGE-E1, MAGE-E2, MAGE-F1, MAGE-H1, MAGEL2, mammaglobin-A, MART- 1/melan-A, MART-2, MART_2/m, matrix protein 22, MC1R, M-CSF, ME1/m, mesothelin, MG50/PXDN, MMP11, MN/CA IX-antigen, MRP-3, MUC-1, MUC-2, MUM-1/m, MUM-2/m, MUM-3/m, myosin class I/m, NA88-A, N-acetylglucosaminyltransferase-V, Neo-PAP, Neo-PAP/m, NFYC/m, NGEP, NMP22, NPM/ALK, N-Ras/m, NSE, NY-ESO-1, NY-ESO-B, OA1, OFA-iLRP, OGT, OGT/m, OS-9, OS-9/m, osteocalcin, osteopontin, p15, p190 minor ber-abl, p53, p53/m, PAGE-4, PAI-1, PAI-2, PART-1, PATE, PDEF, Pim-1-kinase, Pin-1, Pml/PARalpha, POTE, PRAME, PRDX5/m, prostein, proteinase-3 (PR3), PSA, PSCA, PSGR, PSM, PSMA, PTPRK/m, RAGE-1, BAF600/m, RHAMM/CD168, RU1, RU2, S-100, SAGE, SART-1, SART-2, SART-3, SCC, SIRT2/m, Sp17, SSX-1, SSX_2/HOM-MEL-40, SSX-4, STAMP-1, STEAP, survivin, survivin-2B, SYT-SSX-1, SYT-SSX-2, TA-90, TAG-72, TARP, TEL-AML1, TGF$\beta$, TGFBRII, TGM-4, TPI/m, TRAG-3, TRG, TRP-1, TRP-2/6b, TRP/INT2, TRP-p8, tyrosinase, UPA, VEGF, VEGFR-2/FLK-1, and WT1, etc.

In the present specification, when the polynucleotide comprises one target protein, the target protein may be interchangeably described with the "first target protein".

In the present invention, the first target protein may be located at the N-terminus of the non-structural protein P2 domain. Specifically, it may be located at the N-terminus of the 2A protease of the non-structural protein P2 domain.

Therefore, in the present invention, the nucleic acid sequence encoding the first target protein may be located at the 5' terminus of a nucleic acid sequence encoding an amino acid sequence of the 2A protease in the polynucleotide.

In one embodiment of the present invention, the first target protein may be a virus antigen. In one embodiment, the virus antigen may be a severe fever with thrombocytopenia syndrome (SFTS) virus antigen, a SARS-CoV2 spike protein (COVID) antigen, a highly pathogenic avian influenza (HPAI) virus antigen, a canine influenza antigen, or a human papillomavirus (HPV) antigen.

As used herein, the term "severe fever with thrombocytopenia syndrome (SFTS) virus" is also known as Dabie bandavirus, a virus that causes SFTS. SFTS viruses are currently classified into six genotypes (A, B, C, D, E, and F). SFTS is a disease with high fever and thrombocytopenia as its main symptoms, and is transmitted by the Haemaphysalis longicornis tick, a member of the family Ixodidae of the order Metastigmata, which carries the SFTS virus. During the blood sucking process, the virus carried by the tick enters the body, proliferates, and causes the disease.

In the present invention, the severe fever with thrombocytopenia syndrome virus antigen may comprise the amino acid sequence of SEQ ID NO: 45, SEQ ID NO: 85, SEQ ID NO: 116, or SEQ ID NO: 119.

As used herein, the term "SARS-CoV2(COVID) virus" is a single-stranded positive RNA virus with an envelope. It has a specific structure in the shape of a flame or crown due to spike proteins, which are club-shaped protrusions, embedded in the envelope. The above spike protein (S protein) is a spike-shaped protein that the coronavirus uses when invading human cells, and is composed of S1 and S2 subunits. It infects the human body by binding to the angiotensin converting enzyme 2 (ACE-2) receptor on human cells, penetrating the cells and pushing genetic material (RNA) into them to replicate itself.

In the present invention, the SARS-CoV2 virus antigen may comprise the amino acid sequence of SEQ ID NO: 48.

As used herein, the term "highly pathogenic avian influenza (HPAI) virus" refers to a virus that may cause avian influenza in chickens, ducks, wild birds, and the like. The virus has various serotypes, such as 16 types of HA and 9 types of NA depending on the genes of HA and NA in the envelope, and it is known that there is no cross-protection between different serotypes. Among the various serotypes of AIV, all highly pathogenic avian influenza (HPAI) that has occurred to date are known to be caused by the H5 or H7 serotype. In particular, the H5 HPAI viruses of the 2.3.4.4 and 2.3.2.1c lineages, which are H5 HPAI subtypes, have been introduced into the country through wild birds, and many cases of infection in wild birds have been reported to date.

In the present invention, the highly pathogenic avian influenza virus may comprise the amino acid sequence of SEQ ID NO: 105.

As used herein, the term "canine influenza" refers to influenza that occurs in animals of the family Canidae. Canine influenza is presumed to be caused by a variant of influenza virus A that is identical to the equine influenza virus H3N8. The influenza A virus is a negative single-stranded RNA virus with an envelope. In addition, an H3N2 virus that is a mutated avian influenza is known. Canine influenza virus is known to cause acute respiratory disease and to have clinical symptoms such as severe cough, fever, and nasal discharge.

In the present invention, the canine influenza may comprise the amino acid sequence of SEQ ID NO: 53 or SEQ ID NO: 56.

As used herein, the term "human papillomavirus (HPV)" is also called human papillomavirus and is a DNA virus that infects through the skin or mucous membranes of humans and various animals. To date, more than 100 types of human papillomavirus have been discovered, and some types of human papillomavirus cause cancers such as cervical cancer and testicular cancer. In particular, human papillomavirus 16 (HPV 16) and human papillomavirus 18 (HPV 18) are found in 70% of cervical cancer patients worldwide and are classified as highrisk groups. Human papillomavirus consists of E1 to E7, which are genes necessary for virus replication, L1 and L2, which are genes expressing capsid proteins that constitute virions, and LCR, which regulates virus replication and transcription. In particular, E6 and E7 are known to induce cancer development by promoting the degradation of p53 and RB proteins, respectively, thereby activating the cell cycle and inhibiting apoptosis.

In the present invention, the human papillomavirus antigen may comprise the amino acid sequence of SEQ ID NO: 139, SEQ ID NO: 142, SEQ ID NO: 145, or SEQ ID NO: 148.

In one embodiment of the present invention, the polynucleotide may comprise a nucleic acid sequence encoding an amino acid sequence of SEQ ID NO: 45 (SFTS B antigen), SEQ ID NO: 85 (SFTS ABDEF antigen), SEQ ID NO: 116 (SFTS A antigen), SEQ ID NO: 119 (SFTS DEF antigen), SEQ ID NO: 48 (SARS antigen), SEQ ID NO: 105 (H5N8 antigen), SEQ ID NO: 53 (H3N2 antigen), SEQ ID NO: 56 (H3N8 antigen), SEQ ID NO: 139 (HPV E6/E7 domain antigen), SEQ ID NO: 142 (HPV E6/E7 sequential arrangement antigen), SEQ ID NO: 145 (HPV E6/E7 cross over arrangement antigen), or SEQ ID NO: 148 (HPV E6/E7 odd and even arrangement antigen) at the 5' terminus of a nucleic acid sequence encoding an amino acid sequence of the 2A protease.

In one embodiment of the present invention, when the polynucleotide is DNA, the nucleic acid sequence may comprise or consist of the nucleotide sequence of SEQ ID NO: 43, SEQ ID NO: 83, SEQ ID NO: 46, SEQ ID NO: 106, SEQ ID NO: 51, or SEQ ID NO: 54. When the polynucleotide is RNA, the nucleic acid sequence may comprise or consist of the nucleotide sequence of SEQ ID NO: 44, SEQ ID NO: 84, SEQ ID NO: 117, SEQ ID NO: 120, SEQ ID NO: 47, SEQ ID NO: 107, SEQ ID NO: 52, SEQ ID NO: 55, SEQ ID NO: 140, SEQ ID NO: 143, SEQ ID NO: 146, or SEQ ID NO: 149.

In addition, the SFTS antigen, COVID antigen, highly pathogenic avian influenza, canine influenza antigen, or human papillomavirus antigen may be composed of an amino acid sequence in which one or several amino acids are deleted, substituted and/or inserted, as long as they have the same biological function or the gene positions encoding the SFTS antigen, COVID antigen, highly pathogenic avian influenza or canine influenza antigen on the chromosome are the same. Specifically, it may comprise or consist of an amino acid sequence having about 90%, about 91%, about 92%, about 93%, about 94%, about 95%, about 96%, about 97%, about 98%, about 99%, or about 100% identity to the amino acid sequence of SEQ ID NO: 45, SEQ ID NO: 85, SEQ ID NO: 116, SEQ ID NO: 119, SEQ ID NO: 48, SEQ ID NO: 105, SEQ ID NO: 53, SEQ ID NO: 56, SEQ ID NO: 139, SEQ ID NO: 142, SEQ ID NO: 145, or SEQ ID NO: 148.

Protease cleavage site

As used herein, the term "protease cleavage site" refers to a site that is specifically cleaved by a proteolytic enzyme.

In the present invention, the protease cleavage site linked to the first target protein may be a site cleaved by a protease derived from a non-structural protein of the self-amplifying virus. At this time, the self-amplifying virus may be a virus of the genus Enterovirus. Preferably, it may be coxsackievirus B5. In one embodiment of the present invention, the protease cleavage site may be a site cleaved by a 2A protease of enterovirus. At this time, the protease cleavage site may be located between the first target protein and the 2A protease of the non-structural protein P2 domain.

As used herein, the term "2A protease of enterovirus" is a proteolytic enzyme that operates first after the RNA of enterovirus is translated into a single polyprotein, and plays a role in cleaving the polyprotein into structural proteins and non-structural proteins. At this time, the 2A protease may cleave between any one amino acid selected from the group consisting of Tyr, Ala, Thr, Val, Phe, and Arg among the C-terminal amino acids of VP1 of the P1 domain and Gly, which is the N-terminal amino acid of the 2A protease of the P2 domain. In one embodiment of the present invention, the 2A protease may cleave between Tyr and Gly. In one embodiment, it may cleave between Ala and Gly. In one embodiment, it may cleave between Thr and Gly. In one embodiment, it may cleave between Val and Gly. In one embodiment, it may cleave between Phe and Gly. In one embodiment, it may cleave between Arg and Gly. Preferably, it may cleave between Tyr among the C-terminal amino acids of VP1 of the P1 domain and Gly among the N-terminal amino acids of the 2A protease of the P2 domain.

Therefore, in the present invention, the 2A protease cleavage site may comprise the amino acid sequence of the C-terminus of VP1 or the C-terminus of VP1, or the C-terminus of VP1 and the N-terminus of the 2A protease (2A pro).

At this time, the amino acid sequence of the N-terminus of VP1 may comprise 1 to 30 consecutive amino acids from the C-terminus of VP1. Specifically, the 2A protease cleavage site may comprise 1, 2, 3, 4, 5, 6, 7, 8, 9, 10, 11, 12, 13, 14, 15, 16, 17, 18, 19, 20, 21, 22, 23, 24, 26, 27, 28, 29, or 30 consecutive amino acids from the C-terminus of VP1. More specifically, it may comprise 1, 2, 3, 4, 5, 6, 7, 8, 9, 10, 11, 12, 13, 14, 15, 16, 17, 18, 19, 20, 21, 22, 23, 24, 26, 27, 28, 29, or 30 consecutive amino acid sequences from the C-terminus of the amino acid sequence of SEQ ID NO: 70 (VP1 protein).

The amino acid sequence of the N-terminus may comprise 1 to 18 amino acid sequences from the N-terminus of the 2A protease. Specifically, the 2A protease cleavage site may comprise 1, 2, 3, 4, 5, 6, 7, 8, 9, 10, 11, 12, 13, 14, 15, 16, 17, or 18 consecutive amino acid sequences from the N-terminus of the 2A protease. More specifically, it may comprise 1, 2, 3, 4, 5, 6, 7, 8, 9, 10, 11, 12, 13, 14, 15, 16, 17, or 18 consecutive amino acid sequences from the N-terminus of the amino acid sequence of SEQ ID NO: 11 (2A proprotein).

In one embodiment of the present invention, the 2A protease cleavage site may comprise any one amino acid sequence selected from SEQ ID NO: 18(T) to SEQ ID NO: 23. Therefore, the polynucleotide of the present invention may comprise a nucleic acid sequence encoding any one amino acid sequence selected from SEQ ID NO: 18 to SEQ ID NO: 23 between the 3' terminus of the nucleic acid sequence encoding the first target protein and the 5' terminus of the nucleic acid sequence encoding the 2A protease. Preferably, it may comprise a nucleic acid sequence encoding the amino acid sequence of SEQ ID NO: 22. In one embodiment of the present invention, when the polynucleotide is DNA, it may comprise or consist of any one nucleotide sequence selected from SEQ ID NO: 24(ACC) to SEQ ID NO: 29. Preferably, it may comprise the nucleotide sequence of SEQ ID NO: 28. When the polynucleotide is RNA, it may comprise or consist of any one nucleotide sequence selected from SEQ ID NO: 24 and SEQ ID NO: 30 to SEQ ID NO: 34. Preferably, it may comprise the nucleotide sequence of SEQ ID NO: 33.

In addition, the 2A protease cleavage site may be composed of an amino acid sequence in which one or several amino acids are deleted, substituted and/or inserted, as long as it has the same biological function or the gene position encoding the 2A protease cleavage site on the chromosome is the same. Specifically, it may comprise or consist of an amino acid sequence having about 90%, about 91%, about 92%, about 93%, about 94%, about 95%, about 96%, about 97%, about 98%, about 99%, or about 100% identity to any one amino acid sequence selected from SEQ ID NO: 18 to SEQ ID NO: 23.

In the present invention, the polynucleotide may further comprise a nucleic acid sequence encoding a target protein. In the present specification, when the polynucleotide comprises a nucleic acid sequence encoding one or more target proteins, the target proteins may be described by dividing them into a first target protein and a second target protein. At this time, the second target protein may be located at the N-terminus of the first target protein. Therefore, in the present invention, the nucleic acid sequence encoding the second target protein may be located at the 5' terminus of the nucleic acid sequence encoding the amino acid sequence of the first target protein in the polynucleotide. The target protein and the first target protein are the same as described above.

In one embodiment of the present invention, the first target protein and the second target protein may be a virus antigen. In one embodiment, the virus antigens may be H3N2 and H3N8, which are subtypes of canine influenza. At this time, the first target protein may be H3N2 or H3N8, and the second target protein may be H3N2 or H3N8. The first and second target proteins may be the same or different from each other. Therefore, in one embodiment of the present invention, when the polynucleotide is DNA, the first target protein may be composed of the nucleotide sequence of SEQ ID NO: 51 or SEQ ID NO: 54, and when the polynucleotide is RNA, it may be composed of the nucleotide sequence of SEQ ID NO: 52 or SEQ ID NO: 55. In one embodiment of the present invention, the first target protein may be H3N8 and may comprise the amino acid sequence of SEQ ID NO: 56, and the second target protein may be H3N2 and may comprise the amino acid sequence of SEQ ID NO: 53. Therefore, the nucleic acid sequence encoding the first target protein of the polynucleotide may comprise the nucleotide sequence of SEQ ID NO: 54 or SEQ ID NO: 55, and the nucleic acid sequence encoding the second target protein may comprise the nucleotide sequence of SEQ ID NO: 51 or SEQ ID NO: 52.

A 2A self-cleaving peptide sequence or a protease cleavage site may exist between the first target protein and the second target protein.

As used herein, the term "2A self-cleaving peptide" refers to a peptide consisting of 18 to 22 amino acids, which may induce ribosomal skipping during protein translation in a cell. The peptide shares the core sequence motif of DxExNPGP and is found in a wide range of viral families. The 2A self-cleaving peptide may be, for example, P2A, E2A, F2A, T2A, etc. In one embodiment, the 2A self-cleaving peptide may be P2A (porcine teschovirus-1 2A) and may comprise the amino acid sequence of SEQ ID NO: 187. In addition, when the 2A self-cleaving peptide is comprised, it may further comprise a linker (GSG, SEQ ID NO: 188) at the N-terminus of the peptide.

The protease cleavage site may be a site cleaved by a protease derived from a nonstructural protein of the self-amplifying virus. The self-amplifying virus may be a virus of the family Picornaviridae, and specifically, may be a virus of the genus Enterovirus. Preferably, it may be coxsackievirus B5. In one embodiment of the present invention, the protease cleavage site may be a site cleaved by 2A protease, 3CD protease, or 3C protease of enterovirus. More specifically, it may be a site cleaved by 2A protease, 3CD protease, or 3C protease of coxsackievirus B5. Here, the protease cleavage site, the 2A protease, and the 2A protease cleavage site are the same as described above.

In the present invention, the "3C protease of enterovirus" is a proteolytic enzyme that operates second after the RNA of enterovirus is translated into a single polyprotein. The "3CD protease (3CD $^{pro}$)" is a precursor of the 3C protease, and is a protein produced in an intermediate step in which the P3 domain is processed into each individual protein by the 3C protease. At this time, the 3CD protease also has protease activity. The 3C protease and the 3CD protease plays a role in cleaving the polyprotein, which is cleaved by 2A protease into structural proteins (P1 domain) and non-structural proteins (P2 domain and P3 domain), into individual proteins (VP0, VP3, VP1, 2A protease, 2B, 2C, 3A, 3B, 3C protease, and 3D polymerase). At this time, "VP0" is an intermediate protein that is additionally processed into VP4 and VP2.

The 3C protease may cleave between any one amino acid selected from the group consisting of Gln, Glu, Ile, and Thr among the C-terminal amino acids of each individual protein (VP3, VP1, 2A protease, 2B, 2C, 3A, 3B, and 3C protease) and any one amino acid selected from the group consisting of Gly, Asn, Ser, Ala, Val, Cys, Trp, and Met among the N-terminal amino acids of each individual protein (VP1, 2A protease, 2B, 2C, 3A, 3B, 3C protease, and 3D polymerase). In one embodiment of the present invention, the 3C protease may cleave between Gln and Gly. In one embodiment, the 3C protease may cleave between Gln and Asn. In one embodiment, the 3C protease may cleave between Gln and Ser. In one embodiment, the 3C protease may cleave between Gln and Ala. In one embodiment, the 3C protease may cleave between Gln and Val. In one embodiment, the 3C protease may cleave between Gln and Cys. In one embodiment, the 3C protease may cleave between Gln and Trp. In one embodiment, the 3C protease may cleave between Gln and Gly. In one embodiment, the 3C protease may cleave between Gln and Met. In one embodiment, the 3C protease may cleave between Glu and Gly. In one embodiment, the 3C protease may cleave between Glu and Asn. In one embodiment, the 3C protease may cleave between Glu and Ser. In one embodiment, the 3C protease may cleave between Glu and Ala. In one embodiment, the 3C protease may cleave between Glu and Val. In one embodiment, the 3C protease may cleave between Glu and Cys. In one embodiment, the 3C protease may cleave between Glu and Trp. In one embodiment, the 3C protease may cleave between Glu and Gly. In one embodiment, the 3C protease may cleave between Glu and Met. In one embodiment, the 3C protease may cleave between Ile and Gly. In one embodiment, the 3C protease may cleave between Ile and Asn. In one embodiment, the 3C protease may cleave between Ile and Ser. In one embodiment, the 3C protease may cleave between Ile and Ala. In one embodiment, the 3C protease may cleave between Ile and Val. In one embodiment, the 3C protease may cleave between Ile and Cys. In one embodiment, the 3C protease may cleave between Ile and Trp. In one embodiment, the 3C protease may cleave between Ile and Gly. In one embodiment, the 3C protease may cleave between Ile and Met. In one embodiment, the 3C protease may cleave between Thr and Gly. In one embodiment, the 3C protease may cleave between Thr and Asn. In one embodiment, the 3C protease may cleave between Thr and Ser. In one embodiment, the 3C protease may cleave between Thr and Ala. In one embodiment, the 3C protease may cleave between Thr and Val. In one embodiment, the 3C protease may cleave between Thr and Cys. In one embodiment, the 3C protease may cleave between Thr and Trp. In one embodiment, the 3C protease may cleave between Thr and Gly. In one embodiment, the 3C protease may cleave between Thr and Met. Preferably, it may cleave between Gln among the C-terminal amino acids of each individual protein and Gly among the N-terminal amino acids.

Specifically, the 3C protease according to the present invention may cleave between Gln, the C-terminal amino acid of VP3 of coxsackievirus B5, and Gly, the N-terminal amino acid of VP1. It may cleave between Gln, the C-terminal amino acid of the 2A protease, and Gly, the N-terminal amino acid of 2B. It may cleave between Gln, the C-terminal amino acid of 2B, and Gly, the N-terminal amino acid of 2C. It may cleave between Gln, the C-terminal amino acid of 2C, and Gly, the N-terminal amino acid of 3A. It may cleave between Gln, the C-terminal amino acid of 3A, and Gly, the N-terminal amino acid of 3B. It may cleave between Gln, the C-terminal amino acid of 3B, and Gly, the N-terminal amino acid of the 3C protease. It may cleave between Gln, the C-terminal amino acid of the 3C protease, and Gly, the N-terminal amino acid of the 3D polymerase.

Therefore, in the present invention, the 3C protease cleavage site may comprise a C-terminal amino acid sequence of any one protein selected from the group consisting of VP3, 2A protease, 2B, 2C, 3A, 3B, and 3C protease.

In addition, in the present invention, the protease cleavage site may comprise any one amino acid sequence selected from the group consisting of the amino acid sequences of the C-terminus of VP3 and the N-terminus of VP1, the C-terminus of the 2A protease and the N-terminus of 2B, the C-terminus of 2B and the N-terminus of 2C, the C-terminus of 2C and the N-terminus of 3A, the C-terminus of 3A and the N-terminus of 3B, the C-terminus of 3B and the N-terminus of the 3C protease, the C-terminus of the 3C protease and the N-terminus of the 3D polymerase.

Specifically, in the present invention, the 3C protease cleavage site may comprise 1 to 22 consecutive amino acids from the C-terminus of the amino acid sequence of any one protein selected from the group consisting of VP3, 2A protease, 2B, 2C, 3A, 3B, and 3C protease.

More specifically, the 3C protease cleavage site may comprise 1, 2, 3, 4, 5, 6, 7, 8, 9, 10, 11, 12, 13, 14, 15, 16, 17, 18, 19, 20, 21, or 22 consecutive amino acids from the C-terminus of any one amino acid sequence selected from the group consisting of SEQ ID NO: 80, SEQ ID NO: 11, SEQ ID NO: 12, SEQ ID NO: 13, SEQ ID NO: 14, SEQ ID NO: 15, and SEQ ID NO: 16.

In addition, in the present invention, the 3C protease cleavage site may comprise 1 to 11 consecutive amino acids from the C-terminus of the amino acid sequence of VP3 and 1 to 11 consecutive amino acids from the N-terminus of the amino acid sequence of VP1. It may comprise 1 to 11 consecutive amino acids from the C-terminus of the amino acid sequence of the 2A protease and 1 to 11 consecutive amino acids from the N-terminus of the amino acid sequence of 2B. It may comprise 1 to 11 consecutive amino acids from the C-terminus of the amino acid sequence of 2B and 1 to 11 consecutive amino acids from the N-terminus of the amino acid sequence of 2C. It may comprise 1 to 11 consecutive amino acids from the C-terminus of the amino acid sequence of 2C and 1 to 11 consecutive amino acids from the N-terminus of the amino acid sequence of 3A. It may comprise 1 to 11 consecutive amino acids from the C-terminus of the amino acid sequence of 3A and 1 to 11 consecutive amino acids from the N-terminus of the amino acid sequence of 3B. It may comprise 1 to 11 consecutive amino acids from the C-terminus of the amino acid sequence of 3B and 1 to 11 consecutive amino acids from the N-terminus of the amino acid sequence of the 3C protease. It may comprise 1 to 11 consecutive amino acids from the C-terminus of the amino acid sequence of the 3C protease and 1 to 11 consecutive amino acids from the N-terminus of the amino acid sequence of the 3D polymerase.

More specifically, the 3C protease cleavage site may comprise 1, 2, 3, 4, 5, 6, 7, 8, 9, 10, or 11 consecutive amino acids from the C-terminus of the amino acid sequence of SEQ ID NO: 80 and 1, 2, 3, 4, 5, 6, 7, 8, 9, 10, or 11 consecutive amino acids from the N-terminus of the amino acid sequence of SEQ ID NO: 70. It may comprise 1, 2, 3, 4, 5, 6, 7, 8, 9, 10, or 11 consecutive amino acids from the C-terminus of the amino acid sequence of SEQ ID NO: 11 and 1, 2, 3, 4, 5, 6, 7, 8, 9, 10, or 11 consecutive amino acids from the N-terminus of the amino acid sequence of SEQ ID NO: 12. It may comprise 1, 2, 3, 4, 5, 6, 7, 8, 9, 10, or 11 consecutive amino acids from the C-terminus of the amino acid sequence of SEQ ID NO: 12 and 1, 2, 3, 4, 5, 6, 7, 8, 9, 10, or 11 consecutive amino acids from the N-terminus of the amino acid sequence of SEQ ID NO: 13. It may comprise 1, 2, 3, 4, 5, 6, 7, 8, 9, 10, or 11 consecutive amino acids from the C-terminus of the amino acid sequence of SEQ ID NO: 13 and 1, 2, 3, 4, 5, 6, 7, 8, 9, 10, or 11 consecutive amino acids from the N-terminus of the amino acid sequence of SEQ ID NO: 14. It may comprise 1, 2, 3, 4, 5, 6, 7, 8, 9, 10, or 11 consecutive amino acids from the C-terminus of the amino acid sequence of SEQ ID NO: 14 and 1, 2, 3, 4, 5, 6, 7, 8, 9, 10, or 11 consecutive amino acids from the N-terminus of the amino acid sequence of SEQ ID NO: 15. It may comprise 1, 2, 3, 4, 5, 6, 7, 8, 9, 10, or 11 consecutive amino acids from the C-terminus of the amino acid sequence of SEQ ID NO: 15 and 1, 2, 3, 4, 5, 6, 7, 8, 9, 10, or 11 consecutive amino acids from the N-terminus of the amino acid sequence of SEQ ID NO: 16. It may comprise 1, 2, 3, 4, 5, 6, 7, 8, 9, 10, or 11 consecutive amino acids from the C-terminus of the amino acid sequence of SEQ ID NO: 16 and 1, 2, 3, 4, 5, 6, 7, 8, 9, 10, or 11 consecutive amino acids from the N-terminus of the amino acid sequence of SEQ ID NO: 17.

The 3CD protease may cleave between VP0 and VP3. Specifically, it may cleave between any one amino acid selected from the group consisting of Gln, Lys, Tyr, and Met among the C-terminal amino acids of VP0 and any one amino acid selected from the group consisting of Gly, Trp, Tyr, and Asn among the N-terminal amino acids of VP3. In one embodiment of the present invention, the 3CD protease may cleave between Gln and Gly. In one embodiment, it may cleave between Gln and Trp. In one embodiment, it may cleave between Gln and Tyr. In one embodiment, it may cleave between Gln and Asn. In one embodiment, it may cleave between Lys and Gly. In one embodiment, it may cleave between Lys and Trp. In one embodiment, it may cleave between Lys and Tyr. In one embodiment, it may cleave between Lys and Asn. In one embodiment, it may cleave between Thr and Gly. In one embodiment, it may cleave between Thr and Trp. In one embodiment, it may cleave between Thr and Tyr. In one embodiment, it may cleave between Thr and Asn. In one embodiment, it may cleave between Met and Gly. In one embodiment, it may cleave between Met and Trp. In one embodiment, it may cleave between Met and Tyr. In one embodiment, it may cleave between Met and Asn. Preferably, it may cleave between Gln among the C-terminal amino acids of VP0 and Gly among the N-terminal amino acids of VP3.

Therefore, in the present invention, the 3CD protease cleavage site may comprise the C-terminal amino acid sequence of the amino acid sequence of VP2.

In addition, the 3CD protease cleavage site may comprise the C-terminal amino acid sequence of VP2 and the N-terminal amino acid sequence of VP3.

Specifically, in the present invention, the 3CD protease cleavage site may comprise 1 to 40 consecutive amino acids from the C-terminus of VP2.

More specifically, the 3CD protease cleavage site may comprise 1, 2, 3, 4, 5, 6, 7, 8, 9, 10, 11, 12, 13, 14, 15, 16, 17, 18, 19, 20, 21, 22, 23, 24, 25 26, 27, 28, 29, 30, 31, 32, 33, 34, 35, 36, 37, 38, 39, or 40 consecutive amino acids from the C-terminus of the amino acid sequence of SEQ ID NO: 79.

In addition, the 3CD protease cleavage site may comprise 1 to 20 consecutive amino acid sequences from the C-terminus of VP2 and 1 to 20 consecutive amino acid sequences from the N-terminus of VP1.

More specifically, it may comprise 1, 2, 3, 4, 5, 6, 7, 8, 9, 10, 11, 12, 13, 14, 15, 16, 17, 18, 19, or 20 consecutive amino acids from the C-terminus of the amino acid sequence of SEQ ID NO: 79 and 1, 2, 3, 4, 5, 6, 7, 8, 9, 10, 11, 12, 13, 14, 15, 16, 17, 18, 19, or 20 consecutive amino acids from the N-terminus of the amino acid sequence of SEQ ID NO: 80.

In one embodiment of the present invention, the protease cleavage site may comprise 6 consecutive amino acids from the C-terminus of the amino acid sequence of 2C and 6 consecutive amino acids from the N-terminus of the amino acid sequence of 3A.

In one embodiment of the present invention, the 3C protease cleavage site may comprise the amino acid sequence of SEQ ID NO: 50. Therefore, in one embodiment of the present invention, the nucleic acid sequence encoding the 3C protease cleavage site may comprise or consist of the nucleotide sequence of SEQ ID NO: 49 or SEQ ID NO: 57, respectively.

In addition, the 3C protease cleavage site and the 3CD protease cleavage site may be composed of an amino acid sequence in which one or several amino acids are deleted, substituted and/or inserted, as long as they have the same biological function or the gene position encoding the 3C protease cleavage site on the chromosome is the same. Specifically, it may comprise or consist of an amino acid sequence having about 90%, about 91%, about 92%, about 93%, about 94%, about 95%, about 96%, about 97%, about 98%, about 99%, or about 100% identity to the amino acid sequence of SEQ ID NO: 50.

Therefore, in one embodiment of the present invention, the polynucleotide of the present invention may comprise a nucleic acid sequence encoding the first and second target proteins, a nucleic acid sequence encoding the amino acid sequences of SEQ ID NO: 53, SEQ ID NO: 50, SEQ ID NO: 56, and SEQ ID NO: 22 in order from the 5' terminus to the 3' terminus, at the 5' terminus of the nucleic acid sequence encoding the 2A protease protein of the nonstructural protein. Therefore, when the polynucleotide is DNA, it may comprise or consist of the nucleotide sequences of SEQ ID NO: 54, SEQ ID NO: 49, SEQ ID NO: 51, and SEQ ID NO: 28 in order from the 5' terminus to the 3' terminus. When the polynucleotide of the present invention is RNA, the nucleic acid sequence may comprise or consist of the nucleotide sequences of SEQ ID NO: 55, SEQ ID NO: 57, SEQ ID NO: 52, and SEQ ID NO: 33 in order from the 5' terminus to the 3' terminus.

In addition, in the present invention, when the second target protein comprises one or more target proteins, the second target protein may be composed of one or more identical or different target proteins, and a protease cleavage site may be located between each target protein. At this time, the protease cleavage site may be a site cleaved by a protease derived from a non-structural protein of a self-amplifying virus. Specifically, it may be a site cleaved by a protease derived from a virus of the family Picornaviridae. More specifically, it may be a site cleaved by a 2A protease, a 3CD protease, or a 3C protease of enterovirus. Preferably, it may be a site cleaved by a 2A protease, a 3CD protease, or a 3C protease of coxsackievirus B5. The second target protein may be an antigen. In addition, the protease cleavage site, the 2A protease, the 3CD protease, the 3C protease, the 2A protease cleavage site, the 3CD protease cleavage site, and the 3C protease cleavage site are the same as described above.

**Stop codon**

In the present invention, the polynucleotide may further comprise at least one stop codon at the 3' terminus of the nucleic acid sequence encoding the non-structural protein of the self-amplifying virus.

As used herein, the term "stop codon" is a signal sequence for stopping translation from mRNA to amino acid, and generally refers to a sequence of a stop codon or a termination codon. There is no corresponding tRNA in the stop codon, but instead, a protein called a 'terminator' binds, and when the stop codon is reached during the translation process, two units of the ribosome are separated, thereby terminating the translation. Generally, the sequence of the stop codon of DNA is TAG, TAA, or TGA, and the sequence of the stop codon of RNA is UAG, UAA, or UGA.

Specifically, the polynucleotide of the present invention may comprise one to three stop codons.

More specifically, the polynucleotide may comprise one stop codon. In one embodiment, when the polynucleotide is DNA, the stop codon may be any one selected from the group consisting of TAG, TAA, and TGA. When the polynucleotide is RNA, the stop codon may be any one selected from the group consisting of UAG, UAA, and UGA.

More specifically, the polynucleotide may comprise two different stop codons that are positioned consecutively.

When the polynucleotide of the present invention is DNA, in one embodiment, when the polynucleotide is DNA, the stop codon may be located in the order of TAG and TAA from the 5' terminus to the 3' terminus. In one embodiment, it may be located in the order of TAG and TGA. In one embodiment, it may be located in the order of TAA and TGA. When the polynucleotide of the present invention is RNA, in one embodiment, it may be located in the order of UAG and UAA from the 5' terminus to the 3' terminus. In one embodiment, it may be located in the order of UAG and UGA. In one embodiment, it may be located in the order of UAA and UGA.

More specifically, the polynucleotide may comprise three different stop codons that are positioned consecutively.

When the polynucleotide of the present invention is DNA, in one embodiment, the stop codon may be located in the order of TAG, TAA, and TGA from the 5' terminus to the 3' terminus. In one embodiment, it may be located in the order of TAG, TGA, and TAA. In one embodiment, it may be located in the order of TAA, TAG, and TGA. In one embodiment, it may be located in the order of TAA, TGA, and TAG. In one embodiment, it may be located in the order of TGA, TAG, and TAA. In one embodiment, it may be located in the order of TGA, TAA, and TAG. In one embodiment of the present invention, the stop codon may comprise or consist of the nucleotide sequence of SEQ ID NO: 9 (TGATAATAG). When the polynucleotide of the present invention is RNA, in one embodiment, it may be located in the order of UAG, UAA, and UGA from the 5' terminus to the 3' terminus. In one embodiment, it may be located in the order of UAG, UGA, and UAA. In one embodiment, it may be located in the order of UAA, UAG, and UGA. In one embodiment, it may be located in the order of UAA, UGA, and UAG. In one embodiment, it may be located in the order of UGA, UAG, and UAA. In one embodiment, it may be located in the order of UGA, UAA, and UAG. In one embodiment of the present invention, the stop codon may comprise or consist of the nucleotide sequence of SEQ ID NO: 68 (UGAUAAUAG).

**5' untranslated region**

In the present invention, the polynucleotide may further comprise a 5' untranslated region (5' UTR) at the 5' terminus.

As used herein, the term "untranslated region" refers to a region within an RNA molecule that is transcribed but not translated into an amino acid sequence. The untranslated region may exist upstream of the start codon (5' UTR) and downstream of the stop codon (3' UTR). The 5' UTR is a region that regulates the translation and transcription and serves to regulate transcription through different mechanisms in viruses, prokaryotes, and eukaryotes. Although it is generally known as an untranslated region, it has been reported to be translated into a protein product and regulate the translation of mRNA.

Elements of the 5' UTR of eukaryotes and prokaryotes differ significantly. The prokaryotic 5' UTR comprises a ribosome binding site (RBS), also known as the Shine-Dalgarno sequence (SD sequence), which is typically located 3-10 base pairs upstream from the start codon. In contrast, the eukaryotic 5' UTR comprises a Kozak consensus sequence (hereinafter referred to as "Kozak sequence") containing the start codon.

The 5' untranslated region of the polynucleotide according to the present invention may comprise an internal ribosomal entry site (IRES).

As used herein, the term "internal ribosomal entry site (IRES)" is a nucleotide sequence that is typically located in the 5' UTR of an RNA virus and may induce translation of RNA in a cap-independent manner. In the present invention, the 5' UTR is a region where a translational initiation complex associated with translation of a target protein binds, and the IRES is a cisacting nucleotide sequence that induces translation of a target protein by forming a complex secondary and tertiary structure.

In the present invention, the IRES may comprise a nucleotide sequence of an IRES derived from a virus or a eukaryotic cell. At this time, the 5' UTR may have a 5' UTR structure comprising an IRES derived from the virus or eukaryotic cell. In one embodiment of the present invention, the 5' UTR and IRES may be derived from the same self-amplifying virus as the non-structural protein. Specifically, they may be derived from a virus of the genus Picornavirus. More specifically, the 5' UTR and IRES may be derived from enterovirus. In one embodiment of the present invention, the 5' UTR may comprise or consist of the nucleic acid sequence of SEQ ID NO: 1 (DNA) or SEQ ID NO: 60 (RNA), and at this time, the IRES may comprise or consist of the nucleic acid sequence of SEQ ID NO: 58 (DNA) or SEQ ID NO: 59 (RNA).

The IRES may comprise a variation. Here, the variation is the same as described above.

Specifically, the variant of the IRES may be one in which a part of the nucleotide sequence of the wild-type IRES is substituted with a different nucleotide sequence.

More specifically, when the polynucleotide is DNA, it may be one in which any one nucleotide selected from the group consisting of the 236th, 386th, and a combination thereof in the nucleotide sequence of SEQ ID NO: 58 is substituted with a nucleotide different from the nucleotide sequence of SEQ ID NO: 58. In one embodiment, it may be one in which the 236th nucleotide in the nucleotide sequence of SEQ ID NO: 58 is substituted with A, T, or G. In one embodiment, it may be one in which the 386th nucleotide in the nucleotide sequence of SEQ ID NO: 58 is substituted with A, T, or C. In one embodiment, it may be one in which the 236th nucleotide in the nucleotide sequence of SEQ ID NO: 58 is substituted with A, T, or G, and the 386th nucleotide is substituted with A, T, or C. Preferably, it may be one in which the 236th nucleotide in the nucleotide sequence of SEQ ID NO: 58, C, is substituted with T (C236T). It may be one in which the 386th nucleotide in the nucleotide sequence of SEQ ID NO: 58, G, is substituted with A (G386A). More preferably, it may be one in which the 236th nucleotide in the nucleotide sequence of SEQ ID NO: 58, C, is substituted with T (C236T), and the 386th nucleotide, G, is substituted with A (G386A).

When the polynucleotide is RNA, it may be one in which any one nucleotide selected from the group consisting of the 236th, 386th, and a combination thereof in the nucleotide sequence of SEQ ID NO: 59 is substituted with a nucleotide different from the nucleotide sequence of SEQ ID NO: 59. In one embodiment, it may be one in which the 236th nucleotide in the nucleotide sequence of SEQ ID NO: 59 is substituted with A, U, or G. In one embodiment, it may be one in which the 386th nucleotide in the nucleotide sequence of SEQ ID NO: 59 is substituted with A, U, or C. In one embodiment, it may be one in which the 236th nucleotide in the nucleotide sequence of SEQ ID NO: 59 is substituted with A, U or G, and the 386th nucleotide is substituted with A, U, or C. Preferably, it may be one in which the 236th nucleotide in the nucleotide sequence of SEQ ID NO: 59, C, is substituted with U (C236U). It may be one in which the 386th nucleotide in the nucleotide sequence of SEQ ID NO: 59, G, is substituted with A (G386A). More preferably, it may be one in which the 236th nucleotide in the nucleotide sequence of SEQ ID NO: 59, C, is substituted with U (C236U), and the 386th nucleotide, G, is substituted with A (G386A).

In one embodiment of the present invention, the variant of the IRES may comprise or consist of the nucleotide sequence of SEQ ID NO: 81 or SEQ ID NO: 82.

**Kozak sequence**

In the present invention, the polynucleotide may further comprise a Kozak sequence.

As used herein, the term "Kozak sequence" refers to a nucleotide sequence located upstream of the start codon where eukaryotic mRNA begins translation into protein, and plays an important role in recognizing the start codon and initiating protein synthesis.

In the present invention, the Kozak sequence may be located at the 5' terminus of the nucleic acid sequence encoding the target protein (the first target protein or the second target protein). In the present invention, the Kozak sequence may comprise any one nucleotide sequence selected from SEQ ID NO: 35 to SEQ ID NO: 37. Preferably, it may comprise or consist of the nucleotide sequence of SEQ ID NO: 35.

**3' untranslated region**

In the present invention, the polynucleotide may further comprise a 3' UTR. At this time, the 3' UTR may be located at the 3' terminus of the stop codon. The 3' UTR is functionally linked to the nucleic acid sequence encoding the target protein and the non-structural protein together with the 5' UTR, thereby improving the translation efficiency of the target protein and the non-structural protein, or a transcript thereof. In addition, it plays an important role in stably maintaining the mRNA, which is a transcript, without being destroyed in the cell.

In the present invention, the 3' UTR may use a 3' UTR derived from a virus or a eukaryotic cell that is identical to the 5' UTR derived from a virus and/or a eukaryotic cell having the IRES nucleotide sequence described above. In one embodiment of the present invention, the 3' UTR may be derived from a self-amplifying virus identical to the nonstructural protein from which the 5' UTR is derived. Specifically, it may be derived from a virus of the genus Picornavirus identical to the 5' UTR. More specifically, it may be derived from enterovirus identical to the 5' UTR. Preferably, it may be derived from coxsackievirus B5.

In one embodiment of the present invention, the 3' UTR may comprise or consist of the nucleotide sequence of SEQ ID NO: 10 (DNA) or SEQ ID NO: 69 (RNA).

**Poly(A) tail**

In the present invention, the polynucleotide may further comprise a poly(A) tail (poly(A) tail) sequence. The poly(A) tail sequence may be located at the 3' terminus of a nucleic acid sequence encoding a non-structural protein of a self-amplifying virus. When the polynucleotide comprises a stop codon, the poly(A) tail sequence may be located at the 3' terminus of the stop codon. When the polynucleotide comprises a 3' UTR, the poly(A) tail sequence may be located at the 3' terminus of the nucleotide sequence of the 3' UTR. The poly(A) tail sequence may further improve the translation efficiency of the target protein while stabilizing the transcribed nucleic acid molecule. The poly(A) tail sequence may comprise about 10 to about 500 adenosine (A) nucleotides. Specifically, it may be a nucleic acid sequence composed of about 10 to about 500, about 50 to about 400, about 100 to about 300, or about 100 to about 200 adenosine nucleotides. Preferably, it may be composed of about 120 adenosine nucleotides. In one embodiment of the present invention, the poly(A) tail sequence may comprise or consist of the nucleotide sequences of SEQ ID NO: 38 to SEQ ID NO: 40. Preferably, it may be composed of the nucleotide sequence of SEQ ID NO: 40.

**Structure of polynucleotide**

The polynucleotide of the present invention may be composed of the following structural formula (I) in order from the 5' terminus to the 3' terminus:

5'-B-C(1)-D-3'            (I)

in the structural formula (I),
5' and 3' are the 5' terminus and the 3' terminus of the polynucleotide, respectively,
B is a nucleic acid sequence encoding a first target protein,
C(1) is a nucleic acid sequence encoding a protease cleavage site, and
D is a nucleic acid sequence encoding a non-structural protein of a self-amplifying virus.

More specifically, the polynucleotide may be composed of the following structural formula (II) in order from the 5' terminus to the 3' terminus:

5'-U-[K]o-[B'-C(2)]n-B-C(1)-D-S-U'-P-3'              (II)

in the structural formula (II),
5' and 3' are the 5' terminus and the 3' terminus of the polynucleotide, respectively,
U is a nucleic acid sequence of a 5' untranslated region,
K is a Kozak sequence,
B' is a nucleic acid sequence encoding a second target protein,
B is a nucleic acid sequence encoding a first target protein,
D is a nucleic acid sequence encoding a non-structural protein of a self-amplifying virus,
S is a stop codon,
U' is a nucleic acid sequence of a 3' untranslated region,
P is a poly(A) tail,
C(1) and C(2) are each independently a nucleic acid sequence encoding a protease cleavage site,

n represents the number of [B'-C(2)] and is an integer from 0 to 10, and
when n is 2 to 10, B' is each a nucleic acid sequence encoding the same or different target protein, and C(2) is also each a nucleic acid sequence encoding the same or different protease cleavage site. In addition, o is an integer of 0 or 1.
At this time, C(1) may be a 2A protease cleavage site, and C(2) may be a 2A protease, a 3CD protease, or a 3C protease cleavage site.
The 5' untranslated region, the target protein, the first target protein, the second target protein, the stop codon, the 3' untranslated region, the poly(A) tail, the protease cleavage site, the 2A, the 3CD, and the 3C protease cleavage site are the same as described above.
The non-structural protein may comprise a P2 domain and a P3 domain of enterovirus. Specifically, it may comprise a P2 domain and a P3 domain in order from the N-terminus to the C-terminus. More specifically, it may comprise a 2A protease, a 2B, a 2C, a 3A, a 3B, a 3C protease, and a 3D polymerase in order from the N-terminus to the C-terminus. At this time, the non-structural protein, the P2 domain, the P3 domain, the 2A protease, the 2B, and the 2C, the 3A, the 3B, the 3C protease, and the 3D polymerase are the same as described above.
When the polynucleotide according to the present invention is RNA, the polynucleotide (hereinafter, interchangeably used with "RNA construct") may be a self-amplifying RNA (saRNA). The RNA construct may be double-stranded or single-stranded, and preferably may be single-stranded.

As used herein, the term "replicon" refers to a self-replicating nucleic acid sequence, and in the present invention, the RNA replicon refers to a RNA molecule that may be replicated by an RNA-dependent RNA polymerase. The RNA replicon may produce one or more than one identical or substantially similar copies of an RNA replicon without a DNA intermediate. Hereinafter, in the present invention, the saRNA viral vector, the RNA replicon, the saRNA, and the saRNA construct may be interchangeably described.

**Embodiments of polynucleotides**

In one embodiment, the polynucleotide may comprise a structure of [5' UTR-Kozak sequence-target protein-2A protease cleavage site-nonstructural protein (2A protease-2B-2C-3A-3B-3V-C-3D)-stop codon-3' UTR-poly(A) tail] in order from the 5' terminus to the C-terminus. In one embodiment, the polynucleotide may comprise the nucleotide sequence of SEQ ID NO: 111 to SEQ ID NO: 115, SEQ ID NO: 131, SEQ ID NO: 134, SEQ ID NO: 151 to SEQ ID NO: 153, SEQ ID NO: 155, SEQ ID NO: 157, SEQ ID NO: 162, or SEQ ID NO: 111.

In one embodiment, the polynucleotide may comprise a structure of [5' UTR-Kozak sequence-second target protein-3C protease cleavage site-first target protein-2A protease cleavage site-nonstructural protein (2Aprotease-2B-2C-3A-3B-3V-C-3D)-stop codon-3' UTR-poly(A) tail] in order from the 5' terminus to the C-terminus. In one embodiment, the polynucleotide may comprise the nucleotide sequence of SEQ ID NO: 137, SEQ ID NO: 138, SEQ ID NO: 192, or SEQ ID NO: 193.

In one embodiment, the polynucleotide may comprise a structure of [5' UTR-Kozak sequence-second target protein-P2A self-cleaving peptide sequence-first target protein-2A protease cleavage site-nonstructural protein (2A protease-2B-2C-3A-3B-3V-C-3D)-stop codon-3' UTR-poly(A) tail] in order from the 5' terminus to the C-terminus. In one embodiment, the polynucleotide may comprise the nucleotide sequence of SEQ ID NO: 190 or SEQ ID NO: 191.

**Recombinant vector**

In another aspect of the present invention, there is provided a recombinant vector loaded with the polynucleotide. Here, the polynucleotide is the same as described above.

As used herein, the term "recombinant" means "to make through genetic manipulation", and means something that does not occur naturally.

As used herein, the term "vector" refers to a genetic construct that comprises essential regulatory elements that are operably linked to express a gene insert, which is an expression vector (or recombinant vector) that may express a target protein in a host cell. The term "operably linked" means that a nucleic acid expression control sequence and a nucleic acid sequence encoding a target protein are functionally linked to perform a general function. The operably linking with the vector may be performed using a genetic recombination technique well known in the technical field to which the present invention belongs, and the site-specific DNA cleavage and ligation may be easily performed using an enzyme or the like generally known in the technical field to which the present invention belongs. Specifically, the recombinant vector may be amplified *in vitro* by polymerase chain reaction (PCR), recombinantly produced by cloning, purified by cleavage and gel-electroporation fractionation, or synthesized by a chemical synthesis method, but is not limited thereto.

In the present invention, the recombinant vector may be used as a gene delivery vehicle for transporting and expressing the polynucleotide according to the present invention. Therefore, it is preferable to load the polynucleotide into a suitable expression vector. A suitable expression vector that may be used in the present invention may comprise a signal sequence for membrane targeting or secretion in addition to expression control elements such as a promoter, a start codon, and a stop codon. The start codon and the stop codon are generally considered to be part of a nucleotide sequence encoding an immunogenic target protein, and must necessarily exhibit function in a subject when the genetic construct is administered, and must be in frame with the coding sequence.

As used herein, the term "promoter" refers to a nucleic acid sequence that controls the synthesis of a transcript, for example, a transcript comprising a coding sequence, by providing a recognition and binding site for RNA polymerase. A general promoter may be constitutive or inducible. In the present invention, the promoter operably linked to the polynucleotide may be one that may operate preferably in an animal cell, more preferably in a mammalian cell, to control transcription of the polynucleotide. The promoter may comprise a promoter derived from a virus, a promoter derived from the genome of a mammalian cell, or a promoter derived from a bacteriophage. For example, it may comprise, but is not limited to, the cytomegalovirus (CMV) promoter, the adenovirus late promoter, the vaccinia virus 7.5K promoter, the SV40 promoter, the tk promoter of HSV, the 94 promoter, the T3 promoter, the SM6 promoter, the RSV promoter, the EF1α promoter, the metallothionein promoter, the β-actin promoter, cancer cell-specific promoters (e.g., the TERT promoter, the PSA promoter, the PSMA promoter, the CEA promoter, the E2F promoter, and the AFP promoter), and tissue-specific promoters (e.g., the albumin promoter).

In one embodiment of the present invention, the polynucleotide may be used as a template for transcribing into a nucleic acid molecule in the form of RNA (saRNA construct, RNA replicon) through *in vitro* transcription (IVT) after being loaded into a suitable expression vector. At this time, a promoter may be located at the 5' terminus of the polynucleotide for transcription from linearized DNA into RNA.

The *"in vitro* transcription" refers to a process in which RNA, particularly mRNA, is synthesized *in vitro* in a cell-free system. At this time, a cloning vector may be applied to the production of a transcript. These cloning vectors are generally designed as transcription vectors and are comprised in the "recombinant vector" in the present invention. In the present invention, when the polynucleotide is RNA, the RNA may be *in vitro* transcribed RNA (IVT-RNA) and may be obtained by *in vitro* transcription using an appropriate DNA template. At this time, the promoter for regulating transcription may be any promoter of RNA polymerase. In addition, the DNA template used for *in vitro* transcription may be obtained by cloning a nucleic acid, particularly a cDNA (the polynucleotide of the present invention), and introducing it into an appropriate vector for *in vitro* transcription (a recombinant vector loaded with the polynucleotide of the present invention). When the nucleic acid (the polynucleotide of the present invention) is RNA, cDNA may be obtained by reverse transcription.

Specifically, the RNA may be synthesized using a DNA-dependent RNA polymerase such as T7 RNA polymerase, and at this time, the expression vector may comprise a T7 promoter at the 5' terminus of the loaded polynucleotide. When another RNA polymerase, such as SP6 or T3 RNA polymerase, is used during IVT using the expression vector loaded with the polynucleotide as a template, the vector may comprise an SP6 or T3 promoter. In one embodiment of the present invention, the RNA polymerase may be T7 RNA polymerase, and the recombinant vector may comprise a T7 promoter. Specifically, the recombinant vector may comprise the nucleic acid sequence of SEQ ID NO: 94 at the 5' terminus upstream of the polynucleotide according to the present invention or may comprise a promoter composed of the sequence.

A vector that may be used as a template for synthesizing the self-amplifying RNA construct may be, for example, a plasmid, a cosmid, a phage, a viral vector, etc. At this time, the polynucleotide sequence may be a DNA sequence, and preferably, a plasmid DNA.

The "plasmid" generally refers to a structure of extrachromosomal genetic material, a circular DNA duplex that may be replicated independently of chromosomal DNA.

In addition, in the present invention, the recombinant vector may spontaneously replicate in a host cell. At this time, the vector may be introduced into a host cell, recombined and inserted into the host cell genome, and replicated together with the genome of the host cell.

At this time, the recombinant vector may further comprise a promoter or regulator, or an enhancer for controlling the expression of the loaded polynucleotide.

In addition, the plasmid may comprise a selection marker such as an antibiotic resistance gene, and a host cell maintaining the plasmid may be cultured under selective conditions.

**Transformed host cell**

In another aspect of the present invention, there is provided a host cell transformed with the recombinant vector. The recombinant vector is the same as described above.

As used herein, the term "transformation" refers to the phenomenon of artificially causing genetic changes by introducing external DNA into a cell, such that the DNA becomes replicable as a chromosomal element or by completion of chromosomal integration by introducing the DNA into the host cell.

The above "host cell" refers to a cell that parasitizes other microorganisms or genes and supplies nutrients, and refers to a cell in which a vector is transformed into a host cell and causes various genetic or molecular effects in the host cell. The host cell is in a state of competence that may accept external DNA, and external DNA such as a vector may be inserted, and if the vector is successfully introduced into the host cell, the genetic trait of the vector is provided to the host cell. The host cell may comprise a prokaryotic cell (e.g., *E. coli*) or a eukaryotic cell (e.g., yeast cell and insect cell), and may comprise a mammalian cell such as a cell derived from a human, mouse, hamster, pig, goat, or primate.

The transformation may be performed by various methods. Specifically, for the transformation method, $CaCl_2$ precipitation, Hanahan method whose efficiency has been increased by using a reducing agent such as dimethyl sulfoxide (DMSO) in $CaCl_2$ precipitation, electroporation, calcium phosphate precipitation, protoplast fusion, agitation using silicon carbide fiber, agrobacteria-mediated transformation, transformation using PEG, dextran sulfate-, lipofectamine-, or dry/inhibition-mediated transformation, or the like may be used. The method for transforming the plasmid of the present invention is not limited to the above examples, and a transformation method commonly used in the art may be used without limitation.

## Method for producing an RNA replicon

In another aspect of the present invention, there is provided a method for producing an RNA replicon, comprising: i) producing a recombinant vector loaded with the polynucleotide according to the present invention; and ii) synthesizing RNA from the recombinant vector. The polynucleotide, the recombinant vector, and the RNA replicon are the same as described above.

The RNA replicon may be obtained by *in vitro* transcription using the DNA of the recombinant vector as a template. Generally, the poly(A) tail sequence is encoded by a poly-(dT) sequence on the DNA template. In the present invention, the poly(A) tail sequence may be added using an enzyme after transcription. In addition, in the present invention, the poly(A) tail sequence may be transcribed from a recombinant vector. Preferably, it may be transcribed from a recombinant vector.

Suitable methods for *in vitro* transcription are well known in the art and are known to those of ordinary skill in the art. For example, they are described in [Molecular Cloning, A Laboratory Manual, 2nd edition. (1989) editor C Nolan, Cold Spring Harbor Laboratory Press]. In addition, various *in vitro* transcription kits are commercially available.

## Vaccine composition

In another aspect of the present invention, there is provided a vaccine composition comprising the polynucleotide or the recombinant vector of the present invention as an active ingredient. Here, the polynucleotide and the recombinant vector are the same as described above.

As used herein, the term "vaccine" refers to a drug used to stimulate the immune system using a living organism for the purpose of preventing a disease. Immune activation refers to a process for efficiently removing an antigen by producing antibodies, stimulating T cells, or stimulating other immune cells (e.g., macrophages, natural killer cells) in the body. A detailed overview of immunology regarding the above is readily understood by those of ordinary skill in the art (Barrett, J.T., Textbook of Immunology, 1983). In the present specification, "vaccine" or "vaccine composition" may be used interchangeably.

In the present invention, the vaccine composition may be for preventing or treating a protozoan, fungal, bacterial, or viral infection. In the present invention, the vaccine composition may be for preventing or treating cancer.

In the vaccine composition, the polynucleotide or recombinant vector as an active ingredient may be comprised in an amount sufficient to induce a typical immune response. In the vaccine composition, the active ingredient may be comprised in an amount of about 0.0001 $\mu$g to about 1000 $\mu$g. Specifically, it may be comprised in an amount of about 0.0001 $\mu$g to about 1000 $\mu$g, about 0.001 $\mu$g to about 100 $\mu$g, or about 0.01 $\mu$g to about 10 $\mu$g, but is not limited thereto.

In the vaccine composition of the present invention, the active ingredient may be comprised in an amount sufficient to induce a typical immune response, an arbitrary amount (effective amount) depending on the purpose, formulation, mixing purpose, and the like. Typically, the effective amount will be determined within a range of 0.001 wt% to 20.0 wt% based on the total weight of the composition. Here, the term "effective amount" refers to the amount of the recombinant microorganism, which is an active ingredient, sufficient for the vaccine composition to induce a typical immune response of the vaccine. This effective amount may be experimentally determined within the normal capabilities of those of ordinary skill in the art.

In addition, the vaccine composition may comprise a pharmaceutically acceptable carrier or diluent. The carrier used in the composition of the present invention comprises a pharmaceutically acceptable carrier, an adjuvant, and a vehicle, and is collectively referred to as a "pharmaceutically acceptable carrier." Here, "pharmaceutically acceptable" means that it does not inhibit the activity of the active ingredient and does not have a toxicity beyond what the application (prescription) target may adapt. Pharmaceutically acceptable carriers that may be used in the compositions of the present invention comprise, but are not limited to, ion exchange, alumina, aluminum stearate, lecithin, serum proteins (e.g., human serum albumin), buffering substances (e.g., various phosphates, glycine, sorbic acid, potassium sorbate, partial glyceride mixtures of saturated vegetable fatty acids), water, salts or electrolytes (e.g., protamine sulfate, disodium hydrogen phosphate, potassium dihydrogen phosphate, sodium chloride, and zinc salts), colloidal silica, magnesium trisilicate, polyvinyl pyrrolidone, cellulose-based substrates, polyethylene glycol, sodium carboxymethylcellulose, polyarylates, waxes, polyethylene-polyoxypropylene-blocked polymers, polyethylene glycol, and wool fat, etc.

Meanwhile, the vaccine composition of the present invention may be administered in a "therapeutically effective amount." The "therapeutically effective amount" may be easily determined by those of ordinary skill in the art based on factors well known in the medical field, such as the type of disease, the patient's age, body weight, health, sex, the patient's sensitivity to the drug, the route of administration, the method of administration, the number of administrations, the treatment period, the drug to be combined or concurrently used, and the like.

As used herein, the term "administration" means introducing a given substance to a subject by an appropriate method, and for the route of administration of the composition, the composition may be administered through any general route as long as it may reach the target tissue. In order for a vaccine to be effective in producing antibodies, the antigenic substance must be introduced into the body so that the antibody-producing mechanism of the vaccinated subject may be realized. Therefore, the polynucleotide or recombinant vector according to the present invention must first be introduced into the body for an immune response.

When administering the polynucleotide or recombinant vector according to the present invention to a subject, it may be administered in the form of a liposome or vesicle by encapsulating it in a lipid nanoparticle (LNP) or coating it with lipid. At this time, the lipidencapsulated product may be in the form of a lipid aggregate or micelle. The LNP may be prepared using a method known in the art.

In the present invention, in order to stimulate a desirable response by an antigen presented by the vaccine composition, it may be administered systemically through parenteral administration. Parenteral administration may be performed in the form of intranasal, endonasal, oral, intravenous, intramuscular, intraarterial, intramedullary, intrathecal, intracardiac, transdermal, subcutaneous, intradermal, intraperitoneal, enteral, topical, sublingual, or rectal administration, but is not limited thereto. Preferably, it may be administered subcutaneously or intramuscularly.

In addition, in the present invention, the vaccine composition may be administered repeatedly once or several times.

Specifically, it may be administered once, twice, three times, four times, or five times repeatedly, but is not limited thereto, and may be administered repeatedly as long as the vaccine may be effective. The vaccine composition may increase the preventive or therapeutic effect on the disease through repeated administration as described above.

The subject to which the vaccine composition according to the present invention is administered may be, for example, a mammal such as a human, a cow, a horse, a pig, a dog, a sheep, a goat, or a cat, and preferably may be a human.

The preferred dosage of the vaccine composition may be prescribed in various ways depending on factors such as the formulation method of the subject of administration, the administration method, the age, body weight, sex, pathological condition, food, administration time, the route of administration, excretion rate, and response sensitivity of the subject of administration, and may be appropriately selected by those of ordinary skill in the art. The dosage suitable for the subject of administration varies depending on the antigenicity of the gene product and is not particularly limited as long as it is an amount sufficient to induce a typical immune response of the conventional vaccine. The dosage may be easily determined as the required amount through a routine experimental process. A typical initial vaccine dose may be from about 0.001 $\mu$g/kg to about 10 mg/kg or from about 0.01 $\mu$g/kg to about 1 mg/kg of antigen, with increases or multiple doses being used as needed to provide the desired level of protection. The vaccine composition may be administered once daily and may be administered daily, every other day, weekly or every other week. Such dosages should not be construed as limiting the scope of the present invention in any way.

The vaccine composition may be prepared in the form of, for example, a powder, a tablet, a capsule, a liquid, an ointment, a cream, a gel, a hydrogel, an aerosol, a spray, a micellar solution, a transdermal patch, a liposome suspension, a polyplex, an emulsion, a lipid nanoparticle (LNP) (having RNA on the surface or encapsulated therein), or any other suitable form that may be administered to a human or mammal in need of treatment or vaccination.

When the vaccine composition is administered intranasally or parenterally, it may be administered in the form of a spray or aerosol, or by inhalation, but is not limited thereto. The composition for intranasal or endonasal administration is prepared according to techniques well known in the pharmaceutical field, and may be prepared as a solution in saline using benzyl alcohol or other suitable preservatives, absorption promoters to enhance bioavailability, fluorocarbons and/or other solubilizing or dispersing agents known in the art.

When administered parenterally, the vaccine composition according to the present invention may be prepared in the form of a sterile injectable aqueous or oily suspension as a sterile injectable preparation. The suspensions may be formulated according to techniques known in the art using suitable dispersing or wetting agents (e.g., Tween 80) and suspending agents. The sterile injectable preparation may also be a sterile injectable solution or suspension in a non-toxic, parenterally acceptable diluent or solvent (e.g., a solution in 1,3-butanediol). Vehicles and solvents that may be used acceptably comprise mannitol, water, Ringer's solution, and isotonic sodium chloride solution. In addition, sterile fixed oils are also commonly used as solvents or suspending media. For this purpose, any fixed oil having a low irritant property, including synthetic mono- or diglycerides, may be used. Fatty acids such as oleic acid and its glyceride derivatives are useful in injectable preparations, as are pharmaceutically acceptable natural oils (e.g., olive oil or castor oil), especially their polyoxyethylated forms.

The above formulation is known in the art, and specific references may be made to literature [Remington's Pharmaceutical Sciences (19th ed., 1995)], etc. The above literature is considered a part of the present specification.

The vaccine composition of the present invention may further comprise an adjuvant to enhance the immunogenicity of the vaccine. Auxiliary components such as an adjuvant may be appropriately selected and used according to the antigen type and the indication of the vaccine composition.

In another aspect of the present invention, there is provided a method for preventing or treating a disease, comprising administering to a subject the polynucleotide, the recombinant vector, or the vaccine composition. At this time, the vaccine composition and the administration are the same as described above. The polynucleotide, the recombinant vector, or the vaccine composition may be administered once or several times repeatedly. Specifically, it may be administered once, twice, three times, four times or five times repeatedly, and may be administered repeatedly without limitation as long as the preventive or therapeutic effect thereof may be sufficiently exhibited. As described above, the preventive or therapeutic effect for the disease may be increased through repeated administration.

As used herein, the term "treatment" may be used to mean both therapeutic treatment and preventive treatment. At this time, the term "prevention" may be used to mean alleviating or reducing a pathological condition or disease in a subject. The treatment comprises all applications or any form of medication for treating a disease in mammals, including humans. In addition, the above term comprises inhibiting or slowing down the progression of a disease; restoring or repairing damaged or defective functions, thereby partially or completely alleviating a disease; or stimulating an inefficient process; or alleviating a serious disease.

The disease may be an infectious disease caused by protozoa, fungi, bacteria or viruses, and may be a cancer disease.

The subject may be a mammal suffering from or likely to suffer from the disease, and preferably may be a human.

The dosage may be prescribed in various ways depending on factors such as the formulation method, the administration method, patient's age, body weight, sex, pathological condition, food, administration time, the route of administration, excretion rate, and response sensitivity.

### Novel protease cleavage site sequence

In another aspect of the present invention, there is provided a non-structural protein 2A protease cleavage site derived from enterovirus, comprising the amino acid sequence of SEQ ID NO: 22. The enterovirus, the non-structural protein, and the 2A protease cleavage site are the same as described above.

In another aspect of the present invention, there is provided a non-structural protein 3C protease cleavage site derived from enterovirus, comprising the amino acid sequence of SEQ ID NO: 50. The enterovirus, the non-structural protein, and the 3C protease cleavage site are the same as described above.

In another aspect of the present invention, there is provided an IRES variant comprising the nucleic acid sequence of SEQ ID NO: 81 or SEQ ID NO: 82. The IRES variant is the same as described above.

### Novel 5' untranslated region variant sequence

In another aspect of the present invention, there is provided a 5' untranslated region variant comprising the nucleic acid sequence of SEQ ID NO: 86 or SEQ ID NO: 87. The 5' untranslated region variant is the same as described above.

### Novel Kozak sequence

In another aspect of the present invention, there is provided a Kozak sequence comprising the nucleic acid sequence of SEQ ID NO: 35. The Kozak sequence is the same as described above.

### Novel 2A protease sequence

In another aspect of the present invention, there is provided a non-structural protein 2A protease derived from enterovirus, comprising the amino acid sequence of SEQ ID NO: 75. The 2A protease is the same as described above.

### Novel severe fever with thrombocytopenia syndrome (SFTS) virus antigen sequence

In another aspect of the present invention, there is provided a severe fever with thrombocytopenia syndrome (SFTS) virus antigen comprising the amino acid sequence of SEQ ID NO: 45, SEQ ID NO: 85, SEQ ID NO: 116, or SEQ ID NO: 119. The severe fever with thrombocytopenia syndrome virus is the same as described above.

### Novel human papillomavirus antigen sequence

In another aspect of the present invention, there is provided a human papillomavirus antigen comprising the amino acid sequence of SEQ ID NO: 138, SEQ ID NO: 141, SEQ ID NO: 144, or SEQ ID NO: 147. The human papillomavirus is the same as described above.

**Mode for Carrying out the Invention**

Hereinafter, the present invention will be described in more detail by way of the following examples. However, the following examples are only for illustrating the present invention, and the scope of the present invention is not limited only to these examples.

**I. Production of optimized self-amplifying RNA**

**Preparation Example 1. Production of vector for producing enterovirus-based self-amplifying RNA**

A clone vector for producing an enterovirus-based self-amplifying RNA construct in which luciferase, which is used as a basic backbone in producing an optimized self-amplifying RNA construct in the present invention, is inserted as a target protein was produced.

Specifically, the clone vector used in the present invention was produced by loading the coxsackievirus B5 gene (SEQ ID NO: 99) into the pUC19-CMV/T7 (Addgene, plasmid #50005) vector in the same manner as described in "Development of a Universal Cloning System for Reverse Genetics of Human enteroviruses" (WS Choi, et al., Microbiol Spectr. 18;e0316722) to produce an infectious clone. In the clone, the P1 domain (structural protein, VP) was substituted with a nucleic acid encoding the target protein (luciferase, SEQ ID NO: 41) using the In-fusion cloning (In-Fusion®HD Cloning Kit, Takara) method to produce a clone vector in the form of a self-amplifying RNA construct (Figure 16).

**Example 1. Selection of 2A protease cleavage site sequence of self-amplifying RNA**

In order to produce a modified self-amplifying RNA (saRNA) construct with an optimized expression level of the target protein according to the present invention, the expression level of the target protein was first confirmed according to the amino acid sequence of the cleavage site of 2A protease, a non-structural protein of enterovirus.

**Example 1.1. Production of self-amplifying RNA constructs according to 2A protease cleavage site sequence**

Specifically, a library vector was produced by inserting the nucleotide sequences of SEQ ID NO: 24 to SEQ ID NO: 29 into the cleavage site of the 2A protease of the clone vector in the form of the enterovirus-based self-amplifying RNA construct in which the luciferase produced in-house by the method of Preparation Example 1 is inserted as the target protein, using a known mutagenesis method, so that 1 (VP1-1 a.a, SEQ ID NO: 18), 5 (VP1-5 a.a, SEQ ID NO: 19), 10 (VP1-10 a.a, SEQ ID NO: 20), 15 (VP1-15 a.a, SEQ ID NO: 21), 20 (VP1-20 a.a, SEQ ID NO: 22), or 25 (VP1-25 a.a, SEQ ID NO: 23) amino acid sequences are included (Figure 2a). BsmbI (NEB) restriction enzyme was added to the above vector DNA and linearized by reaction at 55 °C for 1 to 6 hours, and then the restriction enzyme was inactivated by reaction at 80 °C for 20 minutes. The DNA was confirmed to be linear DNA by electrophoresis on a 0.9% agarose gel, and then purified as follows.

The purified linearized DNA was used as a template and *in vitro* transcription (IVT) was performed by reaction at 37 °C for 3 hours using Invitrogen™MEGAscript™T7 Transcription Kit. After treatment with DNase to remove residual DNA template, the produced RNA was purified using lithium chloride (LiCl) precipitation method (Table 1).

[Table 1]

| | VP1-1a.a saRNA | | VP1-5a.a saRNA | | VP1-10a.a saRNA | | VP1-15a.a saRNA | | VP1-20a.a saRNA | | VP1-25a.a saRNA | |
|---|---|---|---|---|---|---|---|---|---|---|---|---|
| | DNA vector | saRNA | DNA vector | saRNA | DNA vector | saRNA | DNA vector | saRNA | DNA vector | saRNA | DNA vector | saRNA |
| 5'UTR | SEQ ID NO: 1 | SEQ ID NO: 60 | SEQ NO: 160 | SEQ ID NO: 60 | SEQ ID NO: 1 | SEQ ID NO: 60 | SEQ ID NO: 1 | SEQ ID NO: 60 | SEQ ID NO: 1 | SEQ ID NO: 60 | SEQ ID NO: 1 | SEQ ID NO: 60 |
| Target protein | SEQ ID NO: 41 | SEQ ID NO: 42 | SEQ ID NO: 41 | SEQ ID NO: 42 | SEQ ID NO: 41 | SEQ ID NO: 42 | SEQ ID NO: 41 | SEQ ID NO: 42 | SEQ ID NO: 41 | SEQ ID NO: 42 | SEQ ID NO: 41 | SEQ ID NO: 42 |
| 2A protease cleavage site | SEQ ID 24 | SEQ ID NO: 24 | SEQ ID NO: 25 | SEQ ID NO: 30 | SEQ ID NO: 26 | SEQ ID NO: 31 | SEQ ID NO: 27 | SEQ ID NO: 32 | SEQ ID NO: 28 | SEQ ID NO: 33 | SEQ ID NO: 29 | SEQ ID NO: 34 |
| 2A protease | SEQ ID NO: 2 | SEQ ID NO: 61 | SEQ ID NO: 261 | SEQ ID NO: 61 | SEQ ID NO: 2 | SEQ ID NO: 61 | SEQ ID NO: 2 | SEQ ID NO: 61 | SEQ ID NO: 2 | SEQ ID NO: 61 | SEQ ID NO: 2 | SEQ ID NO: 61 |
| 2B | SEQ ID NO: 3 | SEQ ID NO: 62 | SEQ ID NO: 362 | SEQ ID NO: 62 | SEQ ID NO: 3 | SEQ ID NO: 62 | SEQ ID NO: 3 | SEQ ID NO: 62 | SEQ ID NO: 3 | SEQ ID NO: 62 | SEQ ID NO: 3 | SEQ ID NO: 62 |
| 2C | SEQ ID NO: 4 | SEQ ID NO: 63 | SEQ ID NO: 463 | SEQ ID NO: 63 | SEQ ID NO: 4 | SEQ ID NO: 63 | SEQ ID NO: 4 | SEQ ID NO: 63 | SEQ ID NO: 4 | SEQ ID NO: 63 | SEQ ID NO: 4 | SEQ ID NO: 63 |
| 3A | SEQ ID NO: 5 | SEQ ID NO: 64 | SEQ ID NO: 564 | SEQ ID NO: 64 | SEQ ID NO: 5 | SEQ ID NO: 64 | SEQ ID NO: 5 | SEQ ID NO: 64 | SEQ ID NO: 5 | SEQ ID NO: 64 | SEQ ID NO: 5 | SEQ ID NO: 64 |
| 3B | SEQ ID NO: 6 | SEQ ID NO: 65 | SEQ ID NO: 665 | SEQ ID NO: 65 | SEQ ID NO: 6 | SEQ ID NO: 65 | SEQ ID NO: 6 | SEQ ID NO: 65 | SEQ ID NO: 6 | SEQ ID NO: 65 | SEQ ID NO: 6 | SEQ ID NO: 65 |
| 3C protease | SEQ ID NO: 7 | SEQ ID NO: 66 | SEQ ID NO: 766 | SEQ ID NO: 66 | SEQ ID NO: 7, | SEQ ID NO: 66 | SEQ ID NO: 7 | SEQ ID NO: 66 | SEQ ID NO: 7 | SEQ ID NO: 66 | SEQ ID NO: 7 | SEQ ID NO: 66 |
| 3D | SEQ ID NO: 8 | SEQ ID NO: 67 | SEQ ID NO: 867 | SEQ ID NO: 67 | SEQ ID NO: 8 | SEQ ID NO: 67 | SEQ ID NO: 8 | SEQ ID NO: 67 | SEQ ID NO: 8 | SEQ ID NO: 67 | SEQ ID NO: 8 | SEQ ID NO: 67 |
| 3'UTR | SEQ ID NO: 10 | SEQ ID NO: 69 | SEQ ID NO: 10 | SEQ ID NO: 69 | SEQ ID NO: 10 | SEQ ID NO: 69 | SEQ ID NO: 10 | SEQ ID NO: 69 | SEQ ID NO: 10 | SEQ ID NO: 69 | SEQ ID NO: 10 | SEQ ID NO: 69 |
| poly(A) tail | SEQ ID NO: 38 | SEQ ID NO: 38 | SEQ ID NO: 38 | SEQ ID NO: 38 | SEQ ID NO: 38 | SEQ ID NO: 38 | SEQ ID NO: 38 | SEQ ID NO: 38 | SEQ ID NO: 38 | SEQ ID NO: 38 | SEQ ID NO: 38 | SEQ ID NO: 38 |

**Example 1.2. Comparison of expression levels of self-amplifying RNA constructs according to 2A protease cleavage site sequences**

The self-amplifying RNA construct produced by the method of Example 1.1 was transfected into 293T cells to confirm the expression level.

Specifically, 293T cells were inoculated in a 6-well plate at $5 \times 10^5$ cells/well, cultured overnight, and then the medium was replaced with a medium without fetal bovine serum (FBS) 4 hours before transfection to prepare the cells.

Each of the purified RNAs was transfected into the cells at 5 μg per well using Lipofectamine®2000 Transfection Reagent (Invitrogen). 12 hours after transfection, the cells were harvested, centrifuged (12,000 rpm, 3 minutes) to precipitate the cells, and Luciferase Cell Culture Lysis 1× Reagent was added (100 μl) and reacted at room temperature for 30 minutes. After the reaction was completed, the reaction solution was again centrifuged (12,000 rpm, 1 minute), and the luciferase activity of the supernatant (20 μl) was measured using Luciferase Assay System reagent (Promega).

As a result, as shown in Figure 2b, the luminescence of luciferase was highest in the group treated with the self-amplifying RNA construct (VP1-20 a.a) in which 20 amino acids from the C-terminus of VP1 were inserted.

Therefore, the 20 amino acid sequence (SEQ ID NO: 22) from the C-terminus of VP1 was used as the 2A protease cleavage site of the modified self-amplifying RNA construct with optimized expression level of the present invention.

**Example 2. Selection of Kozak sequence and stop codon**

**Example 2.1. Production of self-amplifying RNA constructs according to Kozak sequence and stop codon**

In order to produce a modified self-amplifying RNA construct with an optimized expression level of the target protein in the present invention, the expression level of the target protein according to the Kozak sequence and stop codon was confirmed.

A self-amplifying RNA construct was produced in the same manner as in Example 1.1, and at this time, the recombinant vector was one in which the Kozak sequence of Kozak 1 (SEQ ID NO: 35, CCACC), Kozak 2 (SEQ ID NO: 36, GCCACC), or Kozak 4 (SEQ ID NO: 37, AAG) was inserted into the 3' terminus of the 5' UTR. The stop codon used was a sequence (3× stop) (SEQ ID NO: 9, TGATAATAG) consisting of three different stop codons in succession at the 5' terminus of the 3' UTR (Table 2, Figure 3a).

[Table 2]

| | Kozak1 saRNA | | Kozak2 saRNA | | Kozak4 saRNA | | 3X stop saRNA | |
|---|---|---|---|---|---|---|---|---|
| | DNA vector | saRNA | DNA vector | saRNA | DNA vector | saRNA | DNA vector | saRNA |
| 5'UTR | SEQ ID NO: 1 | SEQ ID NO: 60 | SEQ ID NO: 1 | SEQ ID NO: 60 | SEQ ID NO: 1 | SEQ ID NO: 60 | SEQ ID NO: 1 | SEQ ID NO: 60 |
| Kozak sequence | SEQ ID NO: 35 | SEQ ID NO: 35 | SEQ ID NO: 36 | SEQ ID NO: 36 | SEQ ID NO: 37 | SEQ ID NO: 37 | - | - |
| Target protein | SEQ ID NO: 41 | SEQ ID NO: 42 | SEQ ID NO: 41 | SEQ ID NO: 42 | SEQ ID NO: 41 | SEQ ID NO: 42 | SEQ ID NO: 41 | SEQ ID NO: 42 |
| 2A protease cleavage site | SEQ ID NO: 28 | SEQ ID NO: 33 | SEQ ID NO: 28 | SEQ ID NO: 33 | SEQ ID NO: 28 | SEQ ID NO: 33 | SEQ ID NO: 28 | SEQ ID NO: 33 |
| 2A protease | SEQ ID NO: 2 | SEQ ID NO: 61 | SEQ ID NO: 2 | SEQ ID NO: 61 | SEQ ID NO: 2 | SEQ ID NO: 61 | SEQ ID NO: 2 | SEQ ID NO: 61 |
| 2B | SEQ ID NO: 3 | SEQ ID NO: 62 | SEQ ID NO: 3 | SEQ ID NO: 62 | SEQ ID NO: 3 | SEQ ID NO: 62 | SEQ ID NO: 3 | SEQ ID NO: 62 |
| 2C | SEQ ID NO: 4 | SEQ ID NO: 63 | SEQ ID NO: 4 | SEQ ID NO: 63 | SEQ ID NO: 4 | SEQ ID NO: 63 | SEQ ID NO: 4 | SEQ ID NO: 63 |
| 3A | SEQ ID NO: 5 | SEQ ID NO: 64 | SEQ ID NO: 5 | SEQ ID NO: 64 | SEQ ID NO: 5 | SEQ ID NO: 64 | SEQ ID NO: 5 | SEQ ID NO: 64 |

(continued)

| | Kozak1 saRNA | | Kozak2 saRNA | | Kozak4 saRNA | | 3X stop saRNA | |
|---|---|---|---|---|---|---|---|---|
| | DNA vector | saRNA | DNA vector | saRNA | DNA vector | saRNA | DNA vector | saRNA |
| 3B | SEQ ID NO: 6 | SEQ ID NO: 65 | SEQ ID NO: 6 | SEQ ID NO: 65 | SEQ ID NO: 6 | SEQ ID NO: 65 | SEQ ID NO: 6 | SEQ ID NO: 65 |
| 3C protease | SEQ ID NO: 7 | SEQ ID NO: 66 | SEQ ID NO: 7 | SEQ ID NO: 66 | SEQ ID NO: 7 | SEQ ID NO: 66 | SEQ ID NO: 7 | SEQ ID NO: 66 |
| 3D | SEQ ID NO: 8 | SEQ ID NO: 67 | SEQ ID NO: 8 | SEQ ID NO: 67 | SEQ ID NO: 8 | SEQ ID NO: 67 | SEQ ID NO: 8 | SEQ ID NO: 67 |
| Stop sequence | - | - | - | - | - | - | SEQ ID NO: 9 (TGATA ATAG) | SEQ ID NO: 68 (UGAUA AUAG) |
| 3' UTR | SEQ ID NO: 10 | SEQ ID NO: 69 | SEQ ID NO: 10 | SEQ ID NO: 69 | SEQ ID NO: 10 | SEQ ID NO: 69 | SEQ ID NO: 10 | SEQ ID NO: 69 |
| poly(A) tail | SEQ ID NO: 40 | SEQ ID NO: 40 | SEQ ID NO: 40 | SEQ ID NO: 40 | SEQ ID NO: 40 | SEQ ID NO: 40 | SEQ ID NO: 40 | SEQ ID NO: 40 |

**Example 2.2. Comparison of expression levels of self-amplifying RNA constructs according to Kozak sequence and stop codon**

Expression of each self-amplifying RNA construct produced by the method of Example 2.1 was measured in 293T cells using Luciferase assay reagent (Promega) in the same manner as in Example 1.2. At this time, a clone vector (original P.C) produced in-house from coxsackievirus B5 was used as a control.

As a result, as shown in Figure 3b, the activity of luciferase was highest in the self-amplifying RNA construct (Kozak 4) in which the Kozak sequence of Kozak 4 was inserted. In addition, compared to the control, the activity of luciferase was significantly increased in the self-amplifying RNA construct comprising the stop codon of SEQ ID NO: 68 (SEQ ID NO: 9 in the case of DNA).

Therefore, the Kozak sequence of the modified self-amplifying RNA construct with optimized expression level of the present invention used was the sequence of SEQ ID NO: 42, and the stop codon used was the 3× stop of SEQ ID NO: 68 (SEQ ID NO: 9 in the case of DNA).

**Example 3. Selection of poly(A) tail sequence**

**Example 3.1. Production of self-amplifying RNA constructs according to poly(A) tail sequence**

In order to produce a modified self-amplifying RNA construct with an optimized expression level of the target protein of the present invention, the expression level of the target protein according to the poly(A) tail (poly(A) tail) sequence was confirmed.

A self-amplifying RNA construct was produced in the same manner as in Example 1.1, and at this time, the recombinant vector was produced by inserting 50 (50× poly(A), SEQ ID NO: 38), 70 (70× poly(A), SEQ ID NO: 39), or 120 (120× poly(A), SEQ ID NO: 40) poly(A) at the 3' terminus of the 3' UTR (Table 3, Figure 4a).

[Table 3]

| | 50× poly(A) saRNA | | 70× poly(A) saRNA | | 120× poly(A) saRNA | |
|---|---|---|---|---|---|---|
| | DNA vector | saRNA | DNA vector | saRNA | DNA vector | saRNA |
| 5' UTR | SEQ ID NO: 1 | SEQ ID NO: 60 | SEQ ID NO: 1 | SEQ ID NO: 60 | SEQ ID NO: 1 | SEQ ID NO: 60 |

(continued)

| | 50× poly(A) saRNA | | 70× poly(A) saRNA | | 120× poly(A) saRNA | |
|---|---|---|---|---|---|---|
| | DNA vector | saRNA | DNA vector | saRNA | DNA vector | saRNA |
| Kozak sequence | SEQ ID NO: 35 | SEQ ID NO: 35 | SEQ ID NO: 35 | SEQ ID NO: 35 | SEQ ID NO: 35 | SEQ ID NO: 35 |
| Target protein | SEQ ID NO: 41 | SEQ ID NO: 42 | SEQ ID NO: 41 | SEQ ID NO: 42 | SEQ ID NO: 41 | SEQ ID NO: 42 |
| 2A protease cleavage site | SEQ ID NO: 28 | SEQ ID NO: 33 | SEQ ID NO: 28 | SEQ ID NO: 33 | SEQ ID NO: 28 | SEQ ID NO: 33 |
| 2A protease | SEQ ID NO: 2 | SEQ ID NO: 61 | SEQ ID NO: 2 | SEQ ID NO: 61 | SEQ ID NO: 2 | SEQ ID NO: 61 |
| 2B | SEQ ID NO: 3 | SEQ ID NO: 62 | SEQ ID NO: 3 | SEQ ID NO: 62 | SEQ ID NO: 3 | SEQ ID NO: 62 |
| 2C | SEQ ID NO: 4 | SEQ ID NO: 63 | SEQ ID NO: 4 | SEQ ID NO: 63 | SEQ ID NO: 4 | SEQ ID NO: 63 |
| 3A | SEQ ID NO: 5 | SEQ ID NO: 64 | SEQ ID NO: 5 | SEQ ID NO: 64 | SEQ ID NO: 5 | SEQ ID NO: 64 |
| 3B | SEQ ID NO: 6 | SEQ ID NO: 65 | SEQ ID NO: 6 | SEQ ID NO: 65 | SEQ ID NO: 6 | SEQ ID NO: 65 |
| 3C protease | SEQ ID NO: 7 | SEQ ID NO: 66 | SEQ ID NO: 7 | SEQ ID NO: 66 | SEQ ID NO: 7 | SEQ ID NO: 66 |
| 3D | SEQ ID NO: 8 | SEQ ID NO: 67 | SEQ ID NO: 8 | SEQ ID NO: 67 | SEQ ID NO: 8 | SEQ ID NO: 67 |
| Stop sequence | SEQ ID NO: 9 | SEQ ID NO: 68 | SEQ ID NO: 9 | SEQ ID NO: 68 | SEQ ID NO: 9 | SEQ ID NO: 68 |
| 3' UTR | SEQ ID NO: 10 | SEQ ID NO: 69 | SEQ ID NO: 10 | SEQ ID NO: 69 | SEQ ID NO: 10 | SEQ ID NO: 69 |
| poly(A) tail | SEQ ID NO: 38 | SEQ ID NO: 38 | SEQ ID NO: 39 | SEQ ID NO: 39 | SEQ ID NO: 40 | SEQ ID NO: 40 |

**Example 3.2. Comparison of expression levels of self-amplifying RNA constructs according to poly(A) tail sequence**

Expression of each self-amplifying RNA construct produced by the method of Example 3.1 was measured in 293T cells using Luciferase assay reagent (Progmega) in the same manner as in Example 1.2.

In addition, the self-amplifying RNA construct was encapsulated in NanoAssemblr®Ignite (Precision NanoSystems) using a lipid nanoparticle (LNP) biodelivery system (ALC-0315 (MEC, HY-138170)/cholesterol (Sigma, C8667)/DMG-PEG 2000 (Avanti Polar Lipids, 880151P)/DOPE (Avanti Polar Lipids, 850725P) and administered to mice to confirm the expression level at the animal level. At this time, the 5-6 weeks-old female BALB/c mice were used (n = 3), and 1 μg of each self-amplifying RNA construct was administered intramuscularly to each mouse. Four hours after administration, luciferin was administered intravenously to the mice at a concentration of 150 mg/kg per mouse, and the luciferase activity in the mice was observed by IVIS Spectrum (Perkin Elmer).

As a result, as shown in Figure 4b, the activity of luciferase was highest in the self-amplifying RNA construct into which 120× poly(A) tail was inserted at the cellular and animal levels.

Therefore, the poly(A) tail of the modified self-amplifying RNA construct with optimized expression level of the present invention used was the sequence of SEQ ID NO: 40.

**II. Antigen expression using optimized self-amplifying RNA**

**Example 4. Production of optimized self-amplifying RNA and confirmation of expression level**

**Example 4.1. Production of optimized self-amplifying RNA**

An optimized self-amplifying RNA construct was produced from a recombinant vector comprising the nucleotide sequence of the 2A protease cleavage site (VP1-20a.a, SEQ ID NO: 28), the Kozak sequence (Kozak1, SEQ ID NO: 35), and the poly(A) tail (120× poly(A), SEQ ID NO: 40) selected in Examples 1 to 3 in the same manner as in Example 1.1 (Table 4).

In order to compare the improved degree of expression level of the modified self-amplifying RNA construct, mRNA and conventional enterovirus-based self-amplifying RNA construct (saRNA) were produced together and used as an experimental group (Figure 5). At this time, mRNA was prepared by cloning the target protein into the structure of 5' UTR (SEQ ID NO: 95) - target protein (SEQ ID NO: 41) - 3' UTR (SEQ ID NO: 97) - poly(A) tail (50× poly(A), SEQ ID NO: 38) using 5' and 3' UTR derived from human globulin, and linearizing it, and then using it as template DNA to react at 37 °C for 6 hours using the MESSAGE mMACHINE™ T7 Transcription Kit (Invitrogen), and then purifying it in the same manner as the self-amplifying RNA construct (Table 4, Figure 5).

[Table 4]

|  | Modified saRNA | | mRNA | |
| --- | --- | --- | --- | --- |
|  | DNA vector | saRNA | DNA | mRNA |
| 5' UTR | SEQ ID NO: 1 | SEQ ID NO: 60 | SEQ ID NO: 95 | SEQ ID NO: 96 |
| Kozak sequence | SEQ ID NO: 35 | SEQ ID NO: 35 | - | - |
| Target protein | SEQ ID NO: 41 (luciferase) | SEQ ID NO: 42 (luciferase) | SEQ ID NO: 41 (luciferase) | SEQ ID NO: 42 (luciferase) |
| 2A protease recognize site | SEQ ID NO: 28 | SEQ ID NO: 33 | - | - |
| 2A protease | SEQ ID NO: 2 | SEQ ID NO: 61 | - | - |
| 2B | SEQ ID NO: 3 | SEQ ID NO: 62 | - | - |
| 2C | SEQ ID NO: 4 | SEQ ID NO: 63 | - | - |
| 3A | SEQ ID NO: 5 | SEQ ID NO: 64 | - | - |
| 3B | SEQ ID NO: 6 | SEQ ID NO: 65 | - | - |
| 3C protease | SEQ ID NO: 7 | SEQ ID NO: 66 | - | - |
| 3D | SEQ ID NO: 8 | SEQ ID NO: 67 | - | - |
| Stop sequence | SEQ ID NO: 9 | SEQ ID NO: 68 | - | - |
| 3' UTR | SEQ ID NO: 10 | SEQ ID NO: 69 | SEQ ID NO: 97 | SEQ ID NO: 98 |
| poly(A) tail | SEQ ID NO: 40 | SEQ ID NO: 40 | SEQ ID NO: 38 | SEQ ID NO: 38 |

**Example 4.2. Confirmation of expression level of optimized self-amplifying RNA**

The expression level of the modified self-amplifying RNA construct produced by the method of Example 4.1 was confirmed at the cellular and animal levels. For confirmation at the cellular level, 293T cells were used, inoculated into a 12-well plate ($2.5 \times 10^5$ cells/well) the day before transfection, and then cultured overnight.

Transfection and luciferase activity measurement were performed in the same manner as in Example 1.2. During transfection, RNA was treated at a concentration of 0.01 μg, 0.1 μg, 0.5 μg, or 1 μg.

As a result, as shown in Figure 6, the luciferase activity in the group treated with the modified self-amplifying RNA construct of the present invention was the highest at all concentrations of RNA.

In order to confirm the expression at the animal level, the mRNA, the self-amplifying RNA construct, or the modified self-amplifying RNA construct was encapsulated in NanoAssemblr®Ignite (Precision NanoSystems) using the LNP biodelivery system (ALC-0315/cholesterol/PEG/DOPE) in the same manner as in Example 3.2, and then administered intramuscularly to the mice. At this time, RNA was administered at a concentration of 0.01 μg, 0.1 μg, or 1 μg per mouse. Four hours after administration, luciferin was administered intravenously to the mice at a concentration of 150 mg/kg per mouse, and the luciferase activity in the mice was observed using IVIS Spectrum (Perkin Elmer).

As a result, as shown in Figure 7, the highest luciferase activity was observed in the group administered with the modified self-amplifying RNA construct of the present invention. In particular, the activity was clearly observed even in the group administered with 0.01 μg, and no luciferase activity was observed in the group administered with mRNA and enterovirus-based conventional self-amplifying RNA construct at the same concentration.

**Example 5. Confirmation of antigen expression level of optimized self-amplifying RNA construct comprising antigen**

**Example 5.1. Production of optimized self-amplifying RNA construct comprising antigen**

In the optimized modified self-amplifying RNA construct of Example 4.1, the target protein was replaced with a severe fever with thrombocytopenia syndrome (SFTS) virus antigen, a SARS-CoV2 spike protein antigen, a highly pathogenic avian influenza (HPAI) virus antigen, or a canine influenza virus antigen (Canine influenza) instead of luciferase, to produce a recombinant vector, and an optimized self-amplifying RNA construct was produced from the recombinant vector in the same manner as in Example 1.1 (Tables 5 to 7). At this time, the used severe fever with thrombocytopenia syndrome virus antigens were the nucleic acid sequence (SEQ ID NO: 43) encoding the SFTS B antigen (SEQ ID NO: 45) and the nucleic acid sequence (SEQ ID NO: 83) encoding the SFTS ABDEF antigen (SEQ ID NO: 85). The nucleic acid sequence (SEQ ID NO: 46) encoding the SARS-CoV2 spike protein antigen (SEQ ID NO: 48) was used, respectively. The used highly pathogenic avian influenza virus antigen was the nucleic acid sequence (SEQ ID NO: 106) encoding the H5N8 antigen (SEQ ID NO: 105). For linearization of vector DNA, it was reacted with BsmBI (NEB) restriction enzyme at 55 °C for 6 hours and inactivated at 80 °C for 10 minutes.

In order to compare the improved degree of expression level of the modified self-amplifying RNA construct, mRNA was also produced together and used as an experimental group. At this time, mRNA was prepared by cloning the target protein into the structure of 5' UTR-target protein-3' UTR-poly(A) tail (50) using 5' and 3' UTR derived from human globulin, and linearizing it, and then using it as template DNA to react at 37 °C for 6 hours using the MESSAGE mMACHINE™ T7 Transcription Kit (Invitrogen), and then purifying it in the same manner as the self-amplifying RNA construct (Tables 5 to 7).

[Table 5]

|  | Modified SFTS B antigen saRNA | | Modified SFTS ABDEF antigen saRNA | | SFTS B antigen mRNA | |
|---|---|---|---|---|---|---|
|  | DNA vector (SEQ ID NO: 127) | saRNA (SEQ ID NO: 113) | DNA vector (SEQ ID NO: 129) | saRNA (SEQ ID NO: 115) | DNA (SEQ ID NO: 161) | mRNA (SEQ ID NO: 123) |
| 5' UTR | SEQ ID NO: 1 | SEQ ID NO: 60 | SEQ ID NO: 1 | SEQ ID NO: 60 | SEQ ID NO: 95 | SEQ ID NO: 96 |
| Kozak sequence | SEQ ID NO: 35 | SEQ ID NO: 35 | SEQ ID NO: 35 | SEQ ID NO: 35 | - | - |
| Target protein | SEQ ID NO: 43 | SEQ ID NO: 44 | SEQ ID NO: 83 | SEQ ID NO: 84 | SEQ ID NO: 43 | SEQ ID NO: 44 |
| 2A protease recognize site | SEQ ID NO: 28 | SEQ ID NO: 33 | SEQ ID NO: 28 | SEQ ID NO: 33 | - | - |
| 2A protease | SEQ ID NO: 2 \| | SEQ ID NO: 61 | SEQ ID NO: 2 | SEQ ID NO: 61 | SEQ ID NO: 2 | SEQ ID NO: 61 |
| 2B | SEQ ID NO: 3 | SEQ ID NO: 62 | SEQ ID NO: 3 | SEQ ID NO: 62 | SEQ ID NO: 3 | SEQ ID NO: 62 |
| 2C | SEQ ID NO: 4 | SEQ ID NO: 63 | SEQ ID NO: 4 | SEQ ID NO: 63 | SEQ ID NO: 4 | SEQ ID NO: 63 |
| 3A | SEQ ID NO: 5 | SEQ ID NO: 64 | SEQ ID NO: 5 | SEQ ID NO: 64 | SEQ ID NO: 5 | SEQ ID NO: 64 |
| 3B | SEQ ID NO: 6 | SEQ ID NO: 65 | SEQ ID NO: 6 | SEQ ID NO: 65 | SEQ ID NO: 6 | SEQ ID NO: 65 |
| 3C protease | SEQ ID NO: 7 | SEQ ID NO: 66 | SEQ ID NO: 7 | SEQ ID NO: 66 | SEQ ID NO: 7 | SEQ ID NO: 66 |

(continued)

| | Modified SFTS B antigen saRNA | | Modified SFTS ABDEF antigen saRNA | | SFTS B antigen mRNA | |
|---|---|---|---|---|---|---|
| | DNA vector (SEQ ID NO: 127) | saRNA (SEQ ID NO: 113) | DNA vector (SEQ ID NO: 129) | saRNA (SEQ ID NO: 115) | DNA (SEQ ID NO: 161) | mRNA (SEQ ID NO: 123) |
| 3D | SEQ ID NO: 8 | SEQ ID NO: 67 | SEQ ID NO: 8 | SEQ ID NO: 67 | SEQ ID NO: 8 | SEQ ID NO: 67 |
| Stop sequence | SEQ ID NO: 9 | SEQ ID NO: 68 | SEQ ID NO: 9 | SEQ ID NO: 68 | - | - |
| 3' UTR | SEQ ID NO: 10 | SEQ ID NO: 69 | SEQ ID NO: 10 | SEQ ID NO: 69 | SEQ ID NO: 97 | SEQ ID NO: 98 |
| poly(A) tail | SEQ ID NO: 40 | SEQ ID NO: 40 | SEQ ID NO: 40 | SEQ ID NO: 40 | SEQ ID NO: 38 | SEQ ID NO: 38 |

[Table 6]

| | Modified SARS antigen saRNA | | SARS antigen mRNA | |
|---|---|---|---|---|
| | DNA vector | saRNA | DNA | mRNA |
| | (SEQ ID NO: 163) | (SEQ ID NO: 162) | (SEQ ID NO: 165) | (SEQ ID NO: 164) |
| 5' UTR | SEQ ID NO: 1 | SEQ ID NO: 60 | SEQ ID NO: 95 | SEQ ID NO: 96 |
| Kozak sequence | SEQ ID NO: 35 | SEQ ID NO: 35 | - | - |
| Target protein | SEQ ID NO: 46 | SEQ ID NO: 47 | SEQ ID NO: 46 | SEQ ID NO: 47 |
| 2A protease recognize site | SEQ ID NO: 28 | SEQ ID NO: 33 | - | - |
| 2A protease | SEQ ID NO: 2 | SEQ ID NO: 61 | SEQ ID NO: 2 | SEQ ID NO: 61 |
| 2B | SEQ ID NO: 3 | SEQ ID NO: 62 | SEQ ID NO: 3 | SEQ ID NO: 62 |
| 2C | SEQ ID NO: 4 | SEQ ID NO: 63 | SEQ ID NO: 4 | SEQ ID NO: 63 |
| 3A | SEQ ID NO: 5 | SEQ ID NO: 64 | SEQ ID NO: 5 | SEQ ID NO: 64 |
| 3B | SEQ ID NO: 6 | SEQ ID NO: 65 | SEQ ID NO: 6 | SEQ ID NO: 65 |
| 3C protease | SEQ ID NO: 7 | SEQ ID NO: 66 | SEQ ID NO: 7 | SEQ ID NO: 66 |
| 3D | SEQ ID NO: 8 | SEQ ID NO: 67 | SEQ ID NO: 8 | SEQ ID NO: 67 |
| Stop sequence | SEQ ID NO: 9 | SEQ ID NO: 68 | - | - |
| 3' UTR | SEQ ID NO: 10 | SEQ ID NO: 69 | SEQ ID NO: 97 | SEQ ID NO: 98 |
| poly(A) tail | SEQ ID NO: 40 | SEQ ID NO: 40 | SEQ ID NO: 38 | SEQ ID NO: 38 |

[Table 7]

| | Modified H5N8 antigen saRNA | | H5N8 antigen mRNA | |
|---|---|---|---|---|
| | DNA vector (SEQ ID NO: 166) | saRNA (SEQ ID NO: 111) | DNA (SEQ ID NO: 167) | mRNA (SEQ ID NO: 110) |
| 5' UTR | SEQ ID NO: 1 | SEQ ID NO: 60 | SEQ ID NO: 95 | SEQ ID NO: 96 |
| Kozak sequence | SEQ ID NO: 35 | SEQ ID NO: 35 | - | - |
| Target protein | SEQ ID NO: 106 | SEQ ID NO: 107 | SEQ ID NO: 106 | SEQ ID NO: 107 |
| 2A protease recognize site | SEQ ID NO: 28 | SEQ ID NO: 33 | - | - |
| 2A protease | SEQ ID NO: 2 | SEQ ID NO: 61 | SEQ ID NO: 2 | SEQ ID NO: 61 |
| 2B | SEQ ID NO: 3 | SEQ ID NO: 62 | SEQ ID NO: 3 | SEQ ID NO: 62 |

(continued)

| | Modified H5N8 antigen saRNA | | H5N8 antigen mRNA | |
|---|---|---|---|---|
| | DNA vector (SEQ ID NO: 166) | saRNA (SEQ ID NO: 111) | DNA (SEQ ID NO: 167) | mRNA (SEQ ID NO: 110) |
| 2C | SEQ ID NO: 4 | SEQ ID NO: 63 | SEQ ID NO: 4 | SEQ ID NO: 63 |
| 3A | SEQ ID NO: 5 | SEQ ID NO: 64 | SEQ ID NO: 5 | SEQ ID NO: 64 |
| 3B | SEQ ID NO: 6 | SEQ ID NO: 65 | SEQ ID NO: 6 | SEQ ID NO: 65 |
| 3C protease | SEQ ID NO: 7 | SEQ ID NO: 66 | SEQ ID NO: 7 | SEQ ID NO: 66 |
| 3D | SEQ ID NO: 8 | SEQ ID NO: 67 | SEQ ID NO: 8 | SEQ ID NO: 67 |
| Stop sequence | SEQ ID NO: 9 | SEQ ID NO: 68 | - | - |
| 3' UTR | SEQ ID NO: 10 | SEQ ID NO: 69 | SEQ ID NO: 97 | SEQ ID NO: 98 |
| poly(A) tail | SEQ ID NO: 40 | SEQ ID NO: 40 | SEQ ID NO: 38 | SEQ ID NO: 38 |

**Example 5.2. Confirmation of expression level of optimized self-amplifying RNA**

The expression level of the modified self-amplifying RNA construct produced by the method of Example 5.1 was confirmed at the cellular level by Western blot. At this time, the efficiency of the cell delivery system (LNP biodelivery system, lipofectamine) was also confirmed using RNA comprising SFTS virus antigen (Figure 8).

First, in order to prepare the LNP-RNA experimental group, each of the above RNAs (RNA comprising SFTS antigen) was encapsulated in NanoAssemblr®Ignite (Precision NanoSystems) using the LNP biodelivery system (ALC-0315/Cholesterol/PEG/DOPE). The encapsulated RNA (LNP-RNA) was diluted 10-fold in $1 \times$ TE (Tris-EDTA) buffer containing 2% (v/v) Triton X-100 (Sigma Aldrich), and then the fluorescence of LNP-RNA was measured at 485 nm (excitation) and 528 nm (emission) wavelengths using a microplate reader (BMG LABTECH, UK). At this time, the RNA concentration corresponding to the fluorescence value was also measured.

Transfection was performed by treating the cells with the LNP-RNA or each RNA prepared as described above at various concentrations. At this time, for each RNA, transfection was performed using Lipofectamine®2000 Transfection Reagent (Invitrogen) in the same manner as in Example 1.2. In addition, the cells were inoculated in a 6-well plate at a concentration of $1 \times 10^6$ cells/well the day before transfection, and then the medium was replaced with medium without fetal bovine serum 4 hours before transfection, and the cells were cultured and prepared, and transfection was performed.

The medium was replaced 4 hours after transfection, and cultured for an additional 24 hours. After 24 hours, cells were harvested by centrifugation (12,000 rpm, 1 minute), and the cells were lysed by adding cell lysis buffer (1 ml) and vortexing. After reacting at room temperature for 10 minutes, the protein concentration was measured, and protein samples were prepared by mixing 10 $\mu$g of protein with $2\times$ sample buffer (SDS sample buffer with the same volume as the protein) and reacting at 95 °C for 7 minutes. Each protein sample was subjected to electrophoresis in SDS-PAGE. Thereafter, it was transferred to the membrane, and blocking was performed with 5% lipoprotein solution, and then the primary antibody was added and reacted at 4 °C for 16 hours. Anti-Gc antibody (Novusbio, NBP2-41153, 1:2000) and anti-Spike antibody (Sino Biological, 40591-MM42, 1:2000) were used for each antigen as primary antibodies.

After the primary antibody reaction, the membrane was washed 5 times for 10 minutes each with TBS-T buffer, and then the secondary antibody was added and reacted at room temperature for 1 to 2 hours. As a secondary antibody, anti-rabbit-HRP antibody (abcam, ab205718, 1:2000) and anti-mouse-HPR antibody (Invitrogen, 62-6520, 1:5000) were used. After the secondary antibody reaction, each membrane was washed 5 times for 10 minutes each with TBS-T and then detected using HRP reagent (Millipore).

As a result, as shown in Figure 8, RNA using LNP showed a higher expression level than RNA transfected using lipofectamine (top). In addition, compared to mRNA, it was confirmed that antigen expression was significantly higher in the optimized modified self-amplifying RNA construct of the present invention comprising SFTS antigen (Figure 8) or SARS-CoV2 antigen (Figure 9).

**III. Production of multiple-antigen self-amplifying RNA**

**Example 6. Production of multiple-antigen self-amplifying RNA and confirmation of expression level**

**Example 6.1. Production of multiple-antigen self-amplifying RNA**

In order to produce a vaccine capable of expressing two multi-antigens, a self-cleaving peptide, P2A (porcine teschovirus-1 2A) or a 3C protease cleavage site sequence of enterovirus was inserted into an enterovirus-based self-amplifying RNA construct, and a multiple-antigen self-amplifying RNA construct was produced in the same manner as in Example 1.1.

At this time, luciferase was used as the first target protein, and canine influenza H3N2 virus antigen was used as the second target protein. The multiple-antigen self-amplifying RNA construct was composed in the order of [5' UTR-Kozak sequence-second target protein (H3N2 virus antigen)-P2A self-cleaving peptide or 3C protease cleavage site-first target protein (luciferase)-2A protease cleavage site-2A protease-2B-2C-3C protease-3D-stop sequence-3' UTR-poly(A) tail] (P2A-comprising self-amplifying RNA construct: SEQ ID NO: 190, 3C protease cleavage site-comprising self-amplifying RNA construct: SEQ ID NO: 192). At this time, in order to optimize the expression of multiple antigens, the positions of the target proteins were crossed to prepare self-amplifying RNA constructs, and the expression levels of the proteins were compared (5' UTR-Kozak sequence-first target protein (luciferase)-P2A or 3C protease cleavage site-second target protein (H3N2 virus antigen)-2A protease cleavage site-2A protease-2B-2C-3C protease-3D-stop sequence-3' UTR-poly(A) tail]) (P2A-comprising self-amplifying RNA construct: SEQ ID NO: 194, 3C protease cleavage site-comprising self-amplifying RNA construct: SEQ ID NO: 196). In addition, a single-antigen (luciferase) self-amplifying RNA construct was also prepared and used.

First, the expression level of the multiple-antigen self-amplifying RNA construct was confirmed at the cellular level by luciferase and Western blot. Measurement of luciferase activity was performed using Lipofectamine®2000 Transfection Reagent (Invitrogen) in the same manner as in Example 1.2, and Western blot was performed in the same manner as in Example 5.2. At this time, the multiple-antigen self-amplifying RNA construct or the single-antigen self-amplifying construct (using 1 μg or 5 μg, respectively) was transfected into each cell.

In addition, the primary antibody used in the Western blot was an anti-H3N2 antibody produced in-house and diluted to 1:2000, and the secondary antibody was an anti-mouse-HRP antibody (Invitrogen, 62-6520, 1:20000). The anti-H3N2 antibody was used by producing an acellular vaccine by the following method. First, 50 μl of purified H3N2 hemagglutinin protein (Mybiosource, 32-5658) at a concentration of 0.1 μg/μl and adjuvant (AddaVax, Invivogen) were mixed at a ratio of 1:1 and administered intramuscularly to the mice. After the first administration, three additional administrations were performed at two-week intervals, and antibodies were produced by directly isolating from the blood.

As a result, as shown in Figure 37, it was confirmed that the luminescence of luciferase was lower in the multiple-antigen self-amplifying RNA construct compared to the single-antigen self-amplifying RNA construct, but the luminescence of luciferase was high in each self-amplifying RNA construct comprising the P2A sequence and the 3C protease cleavage sequence.

In addition, as shown in Figure 38, it was confirmed that the construct comprising the P2A sequence and the construct comprising the 3C protease cleavage site showed similar levels of H3N2 expression levels.

Through the above results, it was confirmed that the multiple-antigen self-amplifying RNA construct can simultaneously express two antigens.

**Example 6.2. Production of multiple-antigen self-amplifying RNA comprising canine influenza antigen**

Using H3N2 and H3N8, which are subtypes of canine influenza, two antigens were inserted into an enterovirus-based self-amplifying RNA construct, and a 3C protease cleavage site amino acid sequence (SEQ ID NO: 50) was inserted between the antigens to produce a multiple-antigen self-amplifying RNA construct in the same manner as in Examples 1.1 and 4.1 above. At this time, H3N2 and H3N8, which are subtypes of canine influenza, were used as antigens (Table 8, Figure 10, and top of Figure 11).

In addition, a self-amplifying RNA construct comprising only a H3N2 antigen and mRNA were also produced and used. At this time, mRNA was prepared by cloning the target protein into the structure of 5' UTR-target protein-3' UTR-poly(A) tail (50) using 5' and 3' UTR derived from human globulin, and linearizing it, and then using it as template DNA to react using the MESSAGE mMACHINE™ T7 Transcription Kit (Invitrogen) in the same manner as in Example 4.1.

[Table 8]

| | | H3N2 antigen saRNA | | H3N2/H3N8 antigen saRNA | | H3N2 antigen mRNA | |
|---|---|---|---|---|---|---|---|
| | | DNA vector (SEQ ID NO: 132) | saRNA (SEQ ID NO: 131) | DNA vector (SEQ ID NO: 138) | saRNA (SEQ ID NO: 137) | DNA (SEQ ID NO: 130) | mRNA (SEQ ID NO: 130) |
| 5' UTR | | SEQ ID NO: 1 | SEQ ID NO: 60 | SEQ ID NO: 1 | SEQ ID NO: 60 | SEQ ID NO: 95 | SEQ ID NO: 96 |

(continued)

| | H3N2 antigen saRNA | | H3N2/H3N8 antigen saRNA | | H3N2 antigen mRNA | |
|---|---|---|---|---|---|---|
| | DNA vector (SEQ ID NO: 132) | saRNA (SEQ ID NO: 131) | DNA vector (SEQ ID NO: 138) | saRNA (SEQ ID NO: 137) | DNA (SEQ ID NO: 130) | mRNA (SEQ ID NO: 130) |
| Kozak sequence | SEQ ID NO: 35 | SEQ ID NO: 35 | SEQ ID NO: 35 | SEQ ID NO: 35 | - | - |
| Target protein 2 | - | - | SEQ ID NO: 51 | SEQ ID NO: 52 | - | - |
| 3C protease cleavage site | - | - | SEQ ID NO: 49 | SEQ ID NO: 57 | - | - |
| Target protein 1 | SEQ ID NO: 51 | SEQ ID NO: 52 | SEQ ID NO: 54 | SEQ ID NO: 55 | SEQ ID NO: 51 | SEQ ID NO: 52 |
| 2A protease cleavage site | SEQ ID NO: 28 | SEQ ID NO: 33 | SEQ ID NO: 28 | SEQ ID NO: 33 | - | - |
| 2A protease | SEQ ID NO: 2 | SEQ ID NO: 61 | SEQ ID NO: 2 | SEQ ID NO: 61 | SEQ ID NO: 2 | SEQ ID NO: 61 |
| 2B | SEQ ID NO: 3 | SEQ ID NO: 62 | SEQ ID NO: 3 | SEQ ID NO: 62 | SEQ ID NO: 3 | SEQ ID NO: 62 |
| 2C | SEQ ID NO: 4 | SEQ ID NO: 63 | SEQ ID NO: 4 | SEQ ID NO: 63 | SEQ ID NO: 4 | SEQ ID NO: 63 |
| 3A | SEQ ID NO: 5 | SEQ ID NO: 64 | SEQ ID NO: 5 | SEQ ID NO: 64 | SEQ ID NO: 5 | SEQ ID NO: 64 |
| 3B | SEQ ID NO: 6 | SEQ ID NO: 65 | SEQ ID NO: 6 | SEQ ID NO: 65 | SEQ ID NO: 6 | SEQ ID NO: 65 |
| 3C protease | SEQ ID NO: 7 | SEQ ID NO: 66 | SEQ ID NO: 7 | SEQ ID NO: 66 | SEQ ID NO: 7 | SEQ ID NO: 66 |
| 3D | SEQ ID NO: 8 | SEQ ID NO: 67 | SEQ ID NO: 8 | SEQ ID NO: 67 | SEQ ID NO: 8 | SEQ ID NO: 67 |
| Stop sequence | SEQ ID NO: 9 | SEQ ID NO: 68 | SEQ ID NO: 9 | SEQ ID NO: 68 | - | - |
| 3' UTR | SEQ ID NO: 10 | SEQ ID NO: 69 | SEQ ID NO: 10 | SEQ ID NO: 69 | SEQ ID NO: 97 | SEQ ID NO: 98 |
| poly(A) tail | SEQ ID NO: 40 | SEQ ID NO: 40 | SEQ ID NO: 40 | SEQ ID NO: 40 | SEQ ID NO: 38 | SEQ ID NO: 38 |

**Example 6.3. Confirmation of antigen expression of multiple-antigen self-amplifying RNA**

The expression level of the multiple-antigen self-amplifying RNA construct produced by the method of Example 6.2 was confirmed at the cellular level by Western blot. For each RNA, transfection was performed using Lipofectamine®2000 Transfection Reagent (Invitrogen) in the same manner as in Example 1.2. At this time, each RNA was treated at a concentration of 0.1 μg, 1 μg, or 5 μg per well. The medium was replaced 4 hours after transfection, and the cells were cultured for an additional 24 hours. At this time, P.C WT virus was used as a control. At this time, P.C WT virus is canine influenza virus (A/canine/Korea/AS-01/2012). Western blot was performed in the same manner as in Example 5.2 and Example 6.1 above.

As a result, as shown in Figure 11, it was confirmed that the antigen expression was significantly increased in the group treated with the self-amplifying RNA construct compared to the group treated with mRNA (bottom left). It was observed that the expression level was slightly low in the group treated with the multiple-antigen self-amplifying RNA construct compared to the group treated with the self-amplifying RNA construct comprising a single antigen. In addition, two types of antigens were observed in a fused form and an isolated form in the group treated with the multiple-antigen self-amplifying RNA construct (bottom right).

Through the above results, it was confirmed that the multiple-antigen self-amplifying RNA construct can express two antigens simultaneously.

## IV. Production of optimized self-amplifying RNA with improved proliferation rate

### Example 7. Virus cell and animal adaptation experiments for increased virus proliferation rate

### Example 7.1. Production of cell-adapted coxsackievirus B5

A cell-adapted virus was produced by infecting Vero cells with wild-type coxsackievirus B5 (Vp8-B5 in Figure 12). At this time, coxsackievirus B5 was isolated after passage from the cerebrospinal fluid of an infant showing aseptic meningitis. Vero cells were cultured in DMEM (Gibco, cat. No. 12430-054) containing 10% fetal bovine serum, and prepared by inoculating 6-well plates at a concentration of $1 \times 10^6$ cells/well the day before infection.

On the day of infection, the virus was washed with physiological saline and then diluted 1:100 in DMEM (Gibco, cat. No. 12430-054) without fetal bovine serum, and the cells treated therewith. After treatment, the cells were cultured for 2 hours, then the culture solution was replaced with DMEM containing 2% fetal bovine serum (FBS), and the cells were cultured for an additional 2 to 4 days to check for cell lesions.

When morphological modification was observed in 80% to 90% of the infected cells, the cells were harvested and centrifuged (1,200 rpm, 3 minutes), and then the cell pellet was frozen and thawed three times repeatedly to extract the virus from the cells. The extracted virus was stored at -80 °C until the next infection. The above process was performed eight times repeatedly.

As a result, it was confirmed that the subcultured coxsackievirus B5 showed an increased virus proliferation rate within infected cells compared to wild-type coxsackievirus B5, and morphological modification of the cells appeared more rapidly.

### Example 7.2. Production of mouse-adapted coxsackievirus B5

The cell-adapted coxsackievirus B5 obtained in the same manner as in Example 7.1 above was administered intraperitoneally to 3-4 weeks-old female BALB/c mice (SAMTACO) at a concentration of 5.5 TCID/200 μl per mouse. The body weight change was observed for 7 days after administration to determine whether the virus caused pathogenicity in the mice. As a result, it was confirmed that the body weight was decreased after 3-4 days after infection. Four days after infection when the body weight loss of the mouse was observed, the mouse was sacrificed by dislocating the cervical vertebrae, and then the intestines were separated. The separated intestine was transferred to a tube and then homogenized by adding 500 μl of culture solution. The homogenized intestine tissue was centrifuged at 12,000 rpm for 3 minutes, and then the supernatant was harvested. The supernatant was used to measure the virus concentration, and other mice were infected therewith in the same manner as above. The above infection process was performed a total of 24 times (VP8Mp24-B5 in Figure 12).

### Example 7.3. Evaluation of proliferation rate of cell- and mouse-adapted coxsackievirus B5

In order to confirm the change in the proliferation rate of coxsackievirus B5 obtained in the same manner as in Example 7.1 and Example 7.2 above, an intermediate tissue culture infection dose (TCID 50) experiment was performed using Vero cells.

Specifically, the viruses used were cell-adapted coxsackievirus B5 (VP8-B5), cell- and mouse-adapted coxsackievirus (VP8M24-B5), and wild-type coxsackievirus B5 (WT-B5). Vero cells were prepared by inoculating a 96-well plate at a concentration of $1 \times 10^6$ cells/well the day before infection. Each virus was prepared by serially diluting a total of 7 times by 1/10 from the stock solution.

The Vero cells prepared as described above were washed with physiological saline and then treated with each prepared concentration of virus (50 μl/well). After two hours, the medium was replaced with DMEM medium containing 2% FBS, and the cells were further cultured. Three days after infection, when morphological modification was observed in 80% to 90% of the cells, each cell was treated with 10% formalin and reacted for 20 minutes to fix the cells. Then, the cells were stained with 1.25% crystal violet dye for 30 minutes and then washed with distilled water, and the cells remaining in each well were observed. In addition, the results were quantified using the Reed-Muench method.

As a result, it was confirmed that the titer of the virus was significantly improved in the group treated with VP8-B5 or VP8M24-B5 compared to WT-B5 (Figure 13).

**Example 7.4. Analysis of nucleic acid sequence of mouse-adapted coxsackievirus B5**

The nucleotide sequence of mouse-adapted coxsackievirus 5 obtained in the same manner as in Example 7.2 above was analyzed.

Specifically, RNA was extracted from the virus to produce cDNA, and then the entire virus gene was amplified using universal primers (SEQ ID NO: 108, SEQ ID NO: 109). Sanger sequencing was performed using the PCR products obtained through the above process.

As a result, mutations in the nucleotide sequence were confirmed in the 5' UTR (2 sites) and 2A protease (2 sites) of the nucleic acid sequence of mouse-adapted coxsackievirus B5 compared to wild-type coxsackievirus B5 (Table 9 and Figure 14). The mutated sequences are underlined in the table below.

[Table 9]

| Domain | | Sequence | SEQ ID NO |
|---|---|---|---|
| 5' UTR | DNA | TTAAAACAGCCTGTGGGTTGTACCCACCCACAGGGCCCACT GGGCGCTAGCACTCTGGTATCACGGTACCTTTGTGTGCCTGT TTTATATCCCCTTTCCCCGATTGTAACTTAGAAGATGTACACA CTGATCAATAGCGGGCATAGCACACCAGCTATGTCTTGATCA AGCACTCCTGTATCCCCGGACCAAGTATCAATAGACTGCTCA CGCGGTTGAAGGAGAAAACGTTCGT **T** ACCCGGCTAACTAC TTCGAGAAACCCAGTAGTATCATGAAAGTTGCGAGGCGTTTC GTTCAGCACACCCCAGTGTAGATCGGGTCGATGAGTCACC GCATCCCCACGGGCGACCGTGGCGGTGGCTGCGCTGGCGG CCTGCCTACGG **A** GCAACCCGTAGGACGCTTCAATACAGAC ATGGTGCGAAGAGTCGATTGAGCTAGTTAGTAGTCCTCCGGC CCCTGAATGCGGCTAATCCTAACTGCGGAGCACATGCCCTCA ACCCAGGGGGGTATTGTGTCGTAACGGGCAACTCTGCAGCGG AACCGACTACTTTGGGTGTCCGTGTTTCCTTTTATCCTTATAC TGGCTGCTTATGGTGACAATTGAAAGATTGTTGCCATATAGCT ATTGGATTGGCCATCCAGTGTCTAATAGAGCTGTTATTTACCT CTTTGTTGGATTTATACCACTCAACCTAAAAGAAGTTAAAAC ACTACAGCTCATTTTACAACTGAATACAGCAAA | 86 |
| | RNA | UUAAAACAGCCUGUGGGUUGUACCCACCCACAGGGCCCAC UGGGCGCUAGCACUCUGGUAUCACGGUACCUUUGUGUGCC UGUUUUAUAUCCCCUUUCCCCGAUUGUAACUUAGAAGAU | 87 |

(continued)

| Domain | Sequence | SEQ ID NO |
|---|---|---|
| | GUACACACUGAUCAAUAGCGGGCAUAGCACACCAGCUAUG UCUUGAUCAAGCACUCCUGUAUCCCCGGACCAAGUAUCAA UAGACUGCUCACGCGGUUGAAGGAGAAAACGUUCGU **U** AC CCGGCUAACUACUUCGAGAAACCCAGUAGUAUCAUGAAA GUUGCGAGGCGUUUCGUUCAGCACACCCCCAGUGUAGAUC GGGUCGAUGAGUCACCGCAUCCCCACGGGCGACCGUGGC GGUGGCUGCGCUGGCGGCCUGCCUACGG **A** GCAACCCGUA GGACGCUUCAAUACAGACAUGGUGCGAAGAGUCGAUUGA GCUAGUUAGUAGUCCUCCGGCCCCUGAAUGCGGCUAAUCC UAACUGCGGAGCACAUGCCCUCAACCCAGGGGGUAUUGUG UCGUAACGGGCAACUCUGCAGCGGAACCGACUACUUUGGG UGUCCGUGUUUCCUUUUAUCCUUAUACUGGCUGCUUAUG GUGACAAUUGAAAGAUUGUUGCCAUAUAGCUAUUGGAUU GGCCAUCCAGUGUCUAAUAGAGCUGUUAUUUACCUCUUU GUUGGAUUUAUACCACUCAACCUAAAAGAAGUUAAAACA CUACAGCUCAUUUUACAACUGAAUACAGCAAA | |

(continued)

| Domain | | Sequence | SEQ ID NO |
|---|---|---|---|
| 2A<sup>pro</sup> | DNA | GGTTCATGTGGGCAACAATCAGGAGCTGTGTACGTGGGCAA TTACAGGATTGTAAACAGACACCTGGCAACGAGCCACGATT GGCAAAATTGCGTGTGGGAGGACTACAACAGGGATCTCCTG GTCAGCACAACAACTGCTCATGGTTGTGATACTATAGCACGG TGCCAATGTACTGCTGGCGTGTACTTTTGTGCATCTAGAAAC AAGCACTACCCAGTGAGCTTCGAGGGTCCAGGTCTAGTGAA AGTTCAGGAG **A** GCGAGTACTACCCCGAGAG **G** TACCAGTC GCACGTACTCCTTGCAGTTGGATTTTCAGAACCGGGTGATTG TGGAGGTATTTTGAGGTGTGAACATGGAGTCATTGGGCTCGT TACCATGGGTGGTGAGGGTGTAGTAGGCTTCGCTGACGTGC GGGATCTTTTGTGGTTGGAAGACGACGCCATGGAGCAG | 73 |
| | RNA | GGUUCAUGUGGGCAACAAUCAGGAGCUGUGUACGUGGGC AAUUACAGGAUUGUAAACAGACACCUGGCAACGAGCCAC GAUUGGCAAAAUUGCGUGUGGGAGGACUACAACAGGGAU CUCCUGGUCAGCACAACAACUGCUCAUGGUUGUGAUACUA UAGCACGGUGCCAAUGUACUGCUGGCGUGUACUUUUGUG CAUCUAGAAACAAGCACUACCCAGUGAGCUUCGAGGGUCC AGGUCUAGUGAAAGUUCAGG **A** GAGCGAGUACUACCCCGA GAG **G** UACCAGUCGCACGUACUCCUUGCAGUUGGAUUUUC AGAACCGGGUGAUUGUGGAGGUAUUUUGAGGUGUGAACA UGGAGUCAUUGGGCUCGUUACCAUGGGUGGUGAGGGUGU AGUAGGCUUCGCUGACGUGCGGGAUCUUUUGUGGUUGGA AGACGACGCCAUGGAGCAG | 74 |
| | AA | GSCGQQSGAVYVGNYRIVNRHLATSHDWQNCVWEDYNRDLL VSTTTAHGCDTIARCQCTAGVYFCASRNKHYPVSFEGPGLVKV QE **S** EYYPERYQSHVLLAVGFSEPGDCGGILRCEHGVIGLVTM GGEGVVGFADVRDLLWLEDDAMEQ | 75 |

Therefore, the enterovirus-based optimized self-amplifying RNA construct of the present invention may comprise the components of Table 10 and Table 11 below (Figure 15).

[Table 10]

| | Single-antigen saRNA | | | |
|---|---|---|---|---|
| | Wild-type | | Variant | |
| | DNA vector | saRNA | DNA vector | saRNA |
| 5' UTR | SEQ ID NO: 1 | SEQ ID NO: 61 | SEQ ID NO: 86 | SEQ ID NO: 87 |

(continued)

| | Single-antigen saRNA | | | |
|---|---|---|---|---|
| | Wild-type | | Variant | |
| | DNA vector | saRNA | DNA vector | saRNA |
| Kozak sequence | SEQ ID NO: 35 | SEQ ID NO: 35 | SEQ ID NO: 35 | SEQ ID NO: 35 |
| Target protein | GOI | GOI | GOI | GOI |
| 2A protease recognize site | SEQ ID NO: 28 | SEQ ID NO: 33 | SEQ ID NO: 28 | SEQ ID NO: 33 |
| 2A protease | SEQ ID NO: 2 | SEQ ID NO: 61 | SEQ ID NO: 73 | SEQ ID NO: 74 |
| 2B | SEQ ID NO: 3 | SEQ ID NO: 62 | SEQ ID NO: 3 | SEQ ID NO: 62 |
| 2C | SEQ ID NO: 4 | SEQ ID NO: 63 | SEQ ID NO: 4 | SEQ ID NO: 63 |
| 3A | SEQ ID NO: 5 | SEQ ID NO: 64 | SEQ ID NO: 5 | SEQ ID NO: 64 |
| 3B | SEQ ID NO: 6 | SEQ ID NO: 65 | SEQ ID NO: 6 | SEQ ID NO: 65 |
| 3C protease | SEQ ID NO: 7 | SEQ ID NO: 66 | SEQ ID NO: 7 | SEQ ID NO: 66 |
| 3D | SEQ ID NO: 8 | SEQ ID NO: 67 | SEQ ID NO: 8 | SEQ ID NO: 67 |
| Stop sequence | SEQ ID NO: 9 | SEQ ID NO: 68 | SEQ ID NO: 9 | SEQ ID NO: 68 |
| 3' UTR | SEQ ID NO: 10 | SEQ ID NO: 69 | SEQ ID NO: 10 | SEQ ID NO: 69 |
| poly(A) tail | SEQ ID NO: 40 | SEQ ID NO: 40 | SEQ ID NO: 40 | SEQ ID NO: 40 |

[Table 11]

| | Multiple-antigen saRNA | | | |
|---|---|---|---|---|
| | Wild-type | | Variant | |
| | DNA vector | saRNA | DNA vector | saRNA |
| 5' UTR | SEQ ID NO: 1 | SEQ ID NO: 61 | SEQ ID NO: 86 | SEQ ID NO: 87 |
| Kozak sequence | SEQ ID NO: 35 | SEQ ID NO: 35 | SEQ ID NO: 35 | SEQ ID NO: 35 |
| Target protein 2 | GOI | GOI | GOI | GOI |
| 3C protease cleavage site | SEQ ID NO: 49 | SEQ ID NO: 57 | SEQ ID NO: 49 | SEQ ID NO: 57 |
| Target protein 1 | GOI | GOI | GOI | GOI |
| 2A protease cleavage site | SEQ ID NO: 28 | SEQ ID NO: 33 | SEQ ID NO: 28 | SEQ ID NO: 33 |
| 2A protease | SEQ ID NO: 2 | SEQ ID NO: 61 | SEQ ID NO: 73 | SEQ ID NO: 74 |
| 2B | SEQ ID NO: 3 | SEQ ID NO: 62 | SEQ ID NO: 3 | SEQ ID NO: 62 |
| 2C | SEQ ID NO: 4 | SEQ ID NO: 63 | SEQ ID NO: 4 | SEQ ID NO: 63 |
| 3A | SEQ ID NO: 5 | SEQ ID NO: 64 | SEQ ID NO: 5 | SEQ ID NO: 64 |
| 3B | SEQ ID NO: 6 | SEQ ID NO: 65 | SEQ ID NO: 6 | SEQ ID NO: 65 |
| 3C protease | SEQ ID NO: 7 | SEQ ID NO: 66 | SEQ ID NO: 7 | SEQ ID NO: 66 |
| 3D | SEQ ID NO: 8 | SEQ ID NO: 67 | SEQ ID NO: 8 | SEQ ID NO: 67 |
| Stop sequence | SEQ ID NO: 9 | SEQ ID NO: 68 | SEQ ID NO: 9 | SEQ ID NO: 68 |
| 3' UTR | SEQ ID NO: 10 | SEQ ID NO: 69 | SEQ ID NO: 10 | SEQ ID NO: 69 |
| poly(A) tail | SEQ ID NO: 40 | SEQ ID NO: 40 | SEQ ID NO: 40 | SEQ ID NO: 40 |

**V. Evaluation of *in vivo* efficacy of self-amplifying RNA**

**Example 8. Evaluation of vaccine efficacy of self-amplifying RNA comprising highly pathogenic avian influenza virus antigen**

**Example 8.1. Production of self-amplifying RNA and mRNA and animal experiment**

In order to evaluate the efficacy of self-amplifying RNA as a construct at the animal level, the self-amplifying RNA (SEQ ID NO: 111) or mRNA (mRNA vaccine, SEQ ID NO: 167) (Table 7) comprising highly pathogenic avian influenza produced in the same manner as in Example 5.1 above was encapsulated (saRNA-LNP, mRNA-LNP) in NanoAssemblr®Ignite (Precision NanoSystems) using a lipid nanoparticle biodelivery system (ALC-0315 (MEC, HY-138170)/cholesterol (Sigma, C8667)/DMG-PEG 2000 (Avanti Polar Lipids, 880151P)/DOPE (Avanti Polar Lipids, 850725P) in the same manner as in Example 5.2 above, and administered intramuscularly to the mice at a concentration of 10 $\mu$g, 1 $\mu$g, 0.1 $\mu$g, or 0.01 $\mu$g, respectively (first immunization). For a control, 5 $\mu$g of HA hemagglutinin purified protein (Sinobiological, A/broiler duck/Korea/Buan2/2014) Hemagglutinin) (50 $\mu$l) and adjuvant (AddaVax, Invivogen) were mixed at a ratio of 1:1 and administered intramuscularly to the mice. At this time, PBS was administered to the control. Four weeks after the first administration (immunization), self-amplifying RNA or mRNA was administered to the mice in the same manner as above (second immunization). Blood was collected from the mice at 4 weeks after the first administration of the vaccine and 2 weeks after the second administration (6 weeks after the first administration), and serum was obtained (Figure 17).

**Example 8.2. Evaluation of neutralizing antibody formation**

In the laboratory mice of Example 8.1 above, blood was collected from the mice 4 weeks after the first administration of the vaccine and 2 weeks after the second administration (6 weeks after the first administration), and serum was obtained and determine whether neutralizing antibodies were formed. At this time, the serum obtained as described above was inactivated by reacting at 56 °C for 1 hour and then used.

The formation of neutralizing antibodies was evaluated by confirming the hemagglutination assay and T cell response using MDCK cells.

Specifically, for the hemagglutination assay, MDCK cells (Madin-Darby canine kidney cells) were inoculated into a 96-well plate at a concentration of $1 \times 10^6$ cells/well and then cultured overnight at 37 °C to prepare the cells. For the virus, highly pathogenic avian influenza virus (A/mallard/Korea/W452/2014(H5N8)) was diluted in TCID 100, and serum was diluted at a ratio of 10:1 with serum-free medium containing trypsin (Thermo Fisher, 4370285) ($1\times$) treated with TPCK (tosylsulfonyl pheny-lalanyl chloromethyl ketone, Thermo Fisher scientific, 20233). Then, the virus dilution and trypsin dilution were mixed at a ratio of 1:1 and reacted at 37 °C for 1 hour to prepare a virus dilution.

The MDCK cells prepared as described above were washed twice with PBS, treated with 50 $\mu$l of the prepared virus dilution per well, and reacted at 37 °C to infect the cells. One hour after infection, the cell culture solution was replaced with serum-free medium containing trypsin (Thermo Fisher, 4370285) ($1\times$) containing TPCK (Thermo Fisher scientific, 20233) and further cultured. 48 hours after infection, the cell culture solution was serially diluted 1/2 in PBS for a total of 11 times, and then each of the dilutions was mixed with turkey red blood cell dilution at a concentration of 0.5% at a ratio of 1:1 and reacted at room temperature for 30 minutes. At this time, if neutralizing antibodies were not formed, the virus and red blood cells would aggregate.

As a result, as shown in Figure 18 (top), for the serum obtained 4 weeks after the first vaccine administration, it was confirmed that the formation of neutralizing antibodies was increased depending on the administered vaccine concentration in the serum of the group administered with the self-amplifying RNA vaccine (saRNA-2.3.4.4).

On the other hand, the formation of neutralizing antibodies was not confirmed in the group administered with the mRNA vaccine (mRNA-2.3.4.4). In addition, for the serum obtained 2 weeks after the second vaccine administration (6 weeks after the first vaccine administration), it was confirmed that the formation of neutralizing antibodies was increased depending on the administered vaccine concentration in the serum of the group administered with the self-amplifying RNA vaccine (saRNA-2.3.4.4). In the group administered with the mRNA vaccine (mRNA-2.3.4.4), the formation of neutralizing antibodies was confirmed only in the group administered with the high-dose (10 $\mu$g, 1 $\mu$g), but the formation of neutralizing antibodies was not confirmed in the group administered with the low-dose (0.1 $\mu$g, 0.01 $\mu$g).

T cell response was confirmed using ELIspot analysis of cells secreting antigen-specific IFN-$\gamma$. At this time, as each antibody required for the experiment, BD™ ELISPOT Mouse IFN$\gamma$ ELISPOT Set (BD, Cat. 551083) was used.

Specifically, the capture antibody was diluted in DPBS at a ratio of 1:200, and then 100 $\mu$l was added to each well of each 96-well plate, and then coated by reacting at 4 °C for 18 hours. Thereafter, each well was washed with DPBS (Welgene) and blocked with RPMI 1640 medium containing 10% heat-treated BCS (bovine calf serum, Welgene) and 1% penicillin/streptomycin (Gibco). Then, the spleen cells isolated from the experimental mouse of Example 8 above were inoculated into each well at a concentration of $1.0 \times 10^6$ cells/well, and then the cells were stimulated by treating them with 20 $\mu$g/ml of H5N8 peptide (Peptron).

At this time, for the negative control, spleen cells from mice administered with naive, saRNA-B virus (10 $\mu$g), and

protein + adjuvant (5 µg) were used.

Thereafter, each well was washed three times with PBS-T (200 µl), and then the primary antibody was diluted in DPBS containing 10% BCS at a ratio of 1:250 and added to each well at 100 µl to react. Thereafter, the cells were treated with the secondary antibody, and the number of spots generated through the HRP substrate reaction was counted.

As a result, as shown in the bottom of Figure 18, it was confirmed that the group administered with the self-amplifying RNA (saRNA-2.3.4.4) efficiently produced neutralizing antibodies even at low concentrations compared to the group administered with mRNA (mRNA-2.3.4.4).

In addition, it was confirmed that T cells were activated depending on the administered vaccine concentration in both the group administered with the self-amplifying RNA (saRNA-2.3.4.4) and the group administered with mRNA (mRNA-2.3.4.4). In particular, it was confirmed that a dose-dependent T cell response was shown. In particular, the group administered with the self-amplifying RNA (saRNA-2.3.4.4) showed about twice as much T cell activity when administered at 10 µg compared to the group administered with mRNA at the same concentration (mRNA-2.3.4.4), and about four times as much T cell activity when administered at 1 µg. From the above results, it was confirmed that the CD8 T cell response in the group administered with the self-amplifying RNA (saRNA-2.3.4.4) was higher then that of the group administered with mRNA (mRNA-2.3.4.4).

### Example 8.3. Measurement of change in body weight and survival rate of mice

In Example 8.1 above, at 2 weeks after the second vaccine administration (6 weeks after the first vaccine administration), the laboratory mice were infected via the nasal cavity with 452 virus (10MLD, A/mallard/Korea/W452/2014(H5N8)), and the body weight and survival rate of the mice were monitored for 14 days.

As a result, as shown in Figure 19, the group administered with the self-amplifying RNA (saRNA-2.3.4.4) showed a 100% survival rate. On the other hand, the group administered with mRNA (mRNA-2.3.4.4) showed a 100% survival rate only at the high-concentration (10 µg, 1 µg), and the group administered with the low-concentration (0.1 µg, 0.01 µg) showed a survival rate of 0% after 9 days, similar to the group (the group administered with PBS, naive).

### Example 8.4. Measurement of virus titer remaining in mouse tissues

Some of the laboratory mice of Example 8.3 above were sacrificed on the 3rd or 5th day after virus infection, and the tissues of each organ were extracted to measure the virus titer remaining in each organ.

As a result, as shown in Figures 20a and 20b, in the case of the group administered with the self-amplifying RNA (saRNA-2.3.4.4), the virus titer was not confirmed in any administration group except for the lung tissue of the group administered with 0.01 µg. On the other hand, in the case of the group administered with mRNA (mRNA-2.3.4.4), in the group administered with the high-concentration (10 µg, 1 µg), no virus titers were confirmed in any tissues, but in the group administered with the low-concentration (0.1 µg, 0.01 µg), virus titers were confirmed in all organs. Through the above results, it was confirmed that self-amplifying RNA had superior activity compared to mRNA.

### Example 9. Evaluation of neutralizing antibodies against severe fever with thrombocytopenia syndrome virus

### Example 9.1. Production of self-amplifying RNA and animal experiment

In order to evaluate the efficacy of self-amplifying RNA as a construct at the animal level, self-amplifying RNA (self-amplifying RNA vaccine) comprising a severe fever with thrombocytopenia syndrome virus (SFTS) antigen was produced in the same manner as in Example 5.1 above (Table 5 and Table 12). At this time, A type (SEQ ID NO: 116), B type (SEQ ID NO: 45), DEF type (SEQ ID NO: 119), or ABDEF type (SEQ ID NO: 85) was used as a severe fever with thrombocytopenia syndrome virus antigen.

[Table 12]

| | Modified SFTS A antigen saRNA | | Modified SFTS DEF antigen saRNA | |
|---|---|---|---|---|
| | DNA vector (SEQ ID NO: 126) | saRNA (SEQ ID NO: 112) | DNA vector (SEQ ID NO: 128) | saRNA (SEQ ID NO: 114) |
| 5' UTR | SEQ ID NO: 1 | SEQ ID NO: 60 | SEQ ID NO: 1 | SEQ ID NO: 60 |
| Kozak sequence | SEQ ID NO: 35 | SEQ ID NO: 35 | SEQ ID NO: 35 | SEQ ID NO: 35 |
| Target protein | SEQ ID NO: 118 | SEQ ID NO: 117 | SEQ ID NO: 121 | SEQ ID NO: 12C |
| 2A protease recognize site | SEQ ID NO: 28 | SEQ ID NO: 33 | SEQ ID NO: 28 | SEQ ID NO: 33 |

(continued)

| | Modified SFTS A antigen saRNA | | Modified SFTS DEF antigen saRNA | |
|---|---|---|---|---|
| | DNA vector (SEQ ID NO: 126) | saRNA (SEQ ID NO: 112) | DNA vector (SEQ ID NO: 128) | saRNA (SEQ ID NO: 114) |
| 2A protease | SEQ ID NO: 2 | SEQ ID NO: 61 | SEQ ID NO: 2 | SEQ ID NO: 61 |
| 2B | SEQ ID NO: 3 | SEQ ID NO: 62 | SEQ ID NO: 3 | SEQ ID NO: 62 |
| 2C | SEQ ID NO: 4 | SEQ ID NO: 63 | SEQ ID NO: 4 | SEQ ID NO: 63 |
| 3A | SEQ ID NO: 5 | SEQ ID NO: 64 | SEQ ID NO: 5 | SEQ ID NO: 64 |
| 3B | SEQ ID NO: 6 | SEQ ID NO: 65 | SEQ ID NO: 6 | SEQ ID NO: 65 |
| 3C protease | SEQ ID NO: 7 | SEQ ID NO: 66 | SEQ ID NO: 7 | SEQ ID NO: 66 |
| 3D | SEQ ID NO: 8 | SEQ ID NO: 67 | SEQ ID NO: 8 | SEQ ID NO: 67 |
| Stop sequence | SEQ ID NO: 9 | SEQ ID NO: 68 | SEQ ID NO: 9 | SEQ ID NO: 68 |
| 3' UTR | SEQ ID NO: 10 | SEQ ID NO: 69 | SEQ ID NO: 10 | SEQ ID NO: 69 |
| poly(A) tail | SEQ ID NO: 40 | SEQ ID NO: 40 | SEQ ID NO: 40 | SEQ ID NO: 40 |

The self-amplifying RNA (self-amplifying RNA vaccine) or mRNA (mRNA vaccine) produced as described above was encapsulated (saRNA-LNP, mRNA-LNP) in NanoAssemblr®Ignite (Precision NanoSystems) using a lipid nanoparticle biodelivery system (ALC-0315(MEC, HY-138170)/cholesterol (Sigma, C8667)/DMG-PEG 2000 (Avanti Polar Lipids, 880151P)/DOPE (Avanti Polar Lipids, 850725P) in the same manner as in Example 8.1, and administered to BALB/c mice (6 weeks old, n = 2) or C57BL/6 mice (6 weeks old, n = 5) at a concentration of 5 $\mu$g/100 $\mu$l/mouse per mouse (first immunization). Three weeks after the first administration (immunization), the self-amplifying RNA vaccine with the same concentration was administered in the same manner (second immunization). 2 weeks after the second administration (5 weeks after the first administration), blood was collected from the mice through orbital blood collection, and serum was obtained (Figure 21a).

**Example 9.2. Evaluation of neutralizing antibody production**

**Example 9.2.1. Evaluation of neutralizing antibody production in mice**

The production of neutralizing antibodies was confirmed using immunofluorescence staining in Vero E6 cells.
Specifically, Vero E6 cells were cultured in DMEM (Gibco, cat. No. 12430-054) containing 10% fetal bovine serum, and then inoculated into a 96-well plate at a concentration of $1.5 \times 10^4$ cells/well and cultured for 24 hours and prepared. Antibodies obtained from the mice were inactivated by reacting at 56 °C for 30 minutes. The cells were serially diluted a total of 10 times with 1/2 of the antibody to SFTS D type virus (NCCP no. 43265) (100 TCID$_{50}$) in PBS, mixed at a ratio of 1:1 with the virus, reacted at 37 °C for 1 hour, and then treated. One hour after treatment, the medium was replaced with DMEM containing 2% fetal bovine serum, and further cultured at 37 °C for 120 hours.
120 hours after serum and virus treatment, the cells were fixed with 80% acetone at 4 °C for 10 minutes, dried for 5 minutes, and then washed with PBS. The fixed cells were treated with 3% BSA solution (100 $\mu$l), blocked for 1.5 hours, and then washed 6 times with PBST. Thereafter, the cells were treated with 50 $\mu$l of the primary antibody (anti-SFTS antibody) per well, and then reacted at 37 °C for 3 hours. At this time, the primary antibody is an antibody produced in-house, and is an antibody obtained two weeks after the second vaccination by intramuscularly inoculating into C57BL/6 or BALB/c mice twice at three-week intervals with a concentration of 5 $\mu$g/mouse or 1 $\mu$g/mouse using an LNP vaccine constructed by our company against the antigen of saRNA-A, saRNA-B, saRRNA-DEF, or saRNA-ABDEF.
After the reaction was completed, each well was washed 5 times with PBST (100 $\mu$l) to remove unbound antibodies, and then the secondary antibody (Alexa Fluor 488 goat anti-mouse IgG antibody, Invitrogen, A11029) was diluted at a ratio of 1:500 in PBST, and the cells were treated therewith (50 $\mu$l/well), and then reaction was performed at 37 °C for 1.5 hours. Then, each well was washed three times with PBST, and the cells were treated with 200 $\mu$l of PBST per well, and the cells were observed using a fluorescence microscope.
As a result, as shown in Figure 21b, in the case of antibodies obtained from BALB/c mice, FITC fluorescence values were observed in Vero E6 cells infected with SFTS virus. On the other hand, the fluorescence values were not observed in Vero E6 cells not infected with SFTS virus. In addition, as shown in Figure 21c, in the case of antibodies obtained from C57BL/6 mice, it was confirmed that the neutralizing antibody value for the SFTS D type virus showed a maximum of 32 in the case of self-amplifying RNA vaccine. That is, it was confirmed that antibodies against the SFTS virus were formed through the self-amplifying RNA vaccine produced by our company.

**Example 9.2.2. Evaluation of neutralizing antibody production in ferrets**

The self-amplifying RNA (4 types) (saRNA-LNP, self-amplifying RNA vaccine) produced and encapsulated in the same manner as in Example 9.1 was administered intramuscularly at a concentration of 15 μg/500 μl/mouse per mouse (first immunization). After the first administration, the second and third administrations were performed at 3-week intervals in the same manner as the first administration (secondary and tertiary immunizations). Blood was collected at 3 weeks after the first, second, or third administration, and serum was obtained by centrifugation (8,500 rpm, 10 minutes) (Figure 22a).

The production of neutralizing antibodies included in the serum of the ferrets was confirmed through cell experiments. At this time, the serum was inactivated by reacting at 56 °C for 30 minutes and then used.

Specifically, Vero E6 cells were prepared by inoculating at a concentration of $1 \times 10^4$ cells/well in a 96-well plate and culturing for 24 hours. The virus was prepared by mixing the serum and 100 TCID50 of SFTS B type (NCCP no. 43273), SFTS C type (NCCP no. 43332), SFTS D type (NCCP no. 43265), or SFTS E type virus (NCCP no. 43333) at a ratio of 1:1 and reacting at 37 °C for 1 hour.

The cells prepared as described above were treated with 50 μl of the virus and serum mixture per well, reacted for 1 hour, and then the medium was replaced with DMEM medium containing 2% fetal bovine serum, and then the cells were cultured for 120 hours. After 120 hours of infection, the cells were fixed with 80% acetone at 4 °C for 10 minutes. The cells were dried for 5 minutes, washed with PBS (150 μl), and then blocked by treatment with 3% BSA solution (100 μl) for 1.5 hours. Then, the cells were washed three times with PBST (100 μl) and then treated with 50 μl of the primary antibody produced in-house per well, and the reaction was performed at 37 °C for 3 hours. Thereafter, the cells were washed five times with PBST (100 μl) and then treated with the secondary antibody, and the reaction was performed at 37 °C for 1.5 hours. At this time, as a secondary antibody, anti-mouse IgG (gamma) antibody, Human Serum Adsorbed and Peroxidase-Labeled (Seracare, 5220-0460) was diluted at a ratio of 1:1000 in PBST, and the cells were treated with 50 μl per well. After the secondary antibody reaction, the cells were stained using the DAB staining method, and then the cells were observed to confirm the degree of antibody production.

As a result, it was confirmed that the average neutralizing antibody value was from a minimum of 40 to a maximum of 320 at 3 weeks after the first vaccination (Figure 22b), and the average neutralizing antibody value was from a minimum of 160 to a maximum of 905 at 3 weeks after the second vaccination (Figure 22c). In addition, it was confirmed that the average neutralizing antibody value was from a minimum of 320 to a maximum of 5120 at 3 weeks after the third vaccination (Figure 22d).

**Example 10. Evaluation of efficacy of canine influenza viral vaccine**

**Example 10.1. Production of self-amplifying RNA and confirmation of expression at the cellular level**

A single-antigen self-amplifying RNA or a multiple-antigen self-amplifying RNA comprising a canine influenza virus antigen (H3N2; SEQ ID NO: 53, H3N8; SEQ ID NO: 56) was produced in the same manner as in Example 6.2 above (Tables 8 and 13).

[Table 13]

|  | H3N2 single-antigen saRNA | |
| --- | --- | --- |
|  | DNA vector (SEQ ID NO: 132) | saRNA (SEQ ID NO: 131) |
| 5' UTR | SEQ ID NO: 1 | SEQ ID NO: 60 |
| Kozak sequence | SEQ ID NO: 35 | SEQ ID NO: 35 |
| Target protein | SEQ ID NO: 51 | SEQ ID NO: 52 |
| 2A protease recognize site | SEQ ID NO: 28 | SEQ ID NO: 33 |
| 2A protease | SEQ ID NO: 2 | SEQ ID NO: 61 |
| 2B | SEQ ID NO: 3 | SEQ ID NO: 62 |
| 2C | SEQ ID NO: 4 | SEQ ID NO: 63 |
| 3A | SEQ ID NO: 5 | SEQ ID NO: 64 |

(continued)

| | H3N2 single-antigen saRNA | |
| --- | --- | --- |
| | DNA vector (SEQ ID NO: 132) | saRNA (SEQ ID NO: 131) |
| 3B | SEQ ID NO: 6 | SEQ ID NO: 65 |
| 3C protease | SEQ ID NO: 7 | SEQ ID NO: 66 |
| 3D | SEQ ID NO: 8 | SEQ ID NO: 67 |
| Stop sequence | SEQ ID NO: 9 | SEQ ID NO: 68 |
| 3' UTR | SEQ ID NO: 10 | SEQ ID NO: 69 |
| poly(A) tail | SEQ ID NO: 40 | SEQ ID NO: 40 |

The self-amplifying RNA (self-amplifying RNA vaccine) was encapsulated (saRNA-LNP) in NanoAssemblr®Ignite (Precision NanoSystems) using the lipid nanoparticle biodelivery system (ALC-0315 (MEC, HY-138170)/cholesterol (Sigma, C8667)/DMG-PEG 2000 (Avanti Polar Lipids, 880151P)/DOPE (Avanti Polar Lipids, 850725P), and then 293T cells were treated therewith at various concentrations to confirm the self-amplifying RNA expression level in the cells in the same manner as in Example 5.2. Anti-H3N2 antibody and anti-H3N8 antibody were used as primary antibodies by diluting them at a ratio of 1:2000 or 1:1000, respectively. At this time, the anti-H3N2 antibody was an antibody produced in-house in the same manner as in Example 6.2 above. The cells were inoculated in a 6-well plate at a concentration of $1 \times 10^6$ cells/well the day before transfection, and then the medium was replaced with medium without fetal bovine serum 4 hours before transfection, and the cells were cultured and prepared.

The medium was replaced 4 hours after transfection, and cultured for an additional 24 hours. After 24 hours, the cell pellet was obtained by centrifugation (12,000 rpm, 1 minute), and then the cells were lysed by treating with cell lysis buffer (1 ml) and vortexing. The lysate was reacted at room temperature for 10 minutes, and then the protein concentration was measured. The protein samples were prepared by mixing 10 μg of protein with 2× sample buffer (SDS sample buffer) at a ratio of 1:1 and then reacting at 95 °C for 7 minutes. Each protein sample was subjected to electrophoresis in SDS-PAGE. Thereafter, it was transferred to the membrane, and blocking was performed with 5% lipoprotein solution, and then the primary antibody was added and reacted at 4 °C for 16 hours. After the primary antibody reaction, the membrane was washed 5 times for 10 minutes each with TBS-T buffer, and then the secondary antibody was added and reacted at room temperature for 1 to 2 hours. After the secondary antibody reaction, each membrane was washed 5 times for 10 minutes each with TBS-T, and then detected using HRP reagent (Millipore).

The primary antibody (anti-H3N2 antibody) was produced in-house and used at a dilution of 1:2000, and the secondary antibody was anti-mouse-HRP antibody (Invitrogen, 62-6520, 1:5000). At this time, the anti-H3N2 antibody was prepared as an LNP vaccine for the saRNA-H3N8 antigen constructed by our company, and administered intramuscularly to the mice at a concentration of 10 μg/mouse twice at 4-week intervals, and then the serum was separated and used at 2 weeks after the second vaccination. The serum was obtained by collecting blood in a tube that did not contain an anticoagulant, leaving it for 24 hours, and then centrifuging (8,500 rpm, 15 minutes, repeated 2 times).

The secondary antibody used was an anti-mouse-HPR antibody (1:5000, Invitrogen, 62-6520). After the secondary antibody reaction, each membrane was washed 5 times for 10 minutes each with TBS-T, and then detected using HRP reagent (Millipore).

As a result, as shown in Figure 23, it was confirmed that the expression of each antigen was increased in a concentration-dependent manner depending on the self-amplifying RNA treatment.

Example 10.2. Animal experiment

The self-amplifying RNA (self-amplifying RNA vaccine) encapsulated in the same manner as in Example 10.1 above was administered to BALB/c mice at various concentrations. At this time, as the self-amplifying RNA, H3N2 single-antigen self-amplifying RNA (S.A.H3N2), H3N8 single-antigen self-amplifying RNA (S.A.H3N8) and H3N2 and H3N8 multiple-antigen self-amplifying RNA (S.A.H3N2+H3N8) were used, and administered intramuscularly at a concentration of 5 μg, 1 μg, or 0.1 μg per mouse (first immunization).

At this time, the positive control was used by mixing inactivated canine influenza virus (A/canine/Korea/AS-01/2012) and canine influenza H3N8 virus (A/canine/Massachusetts/26810/2016) with Aluminum Hydroxide Gel Adjuvant (InvivoGen) at a 1:1 ratio. The inactivated canine virus was produced by inoculating H3N2 or H3N8 canine influenza virus into a hatching egg, culturing at 37 °C for 48 hours, obtaining allantoic fluid, and then mixing it with formalin (containing formalin at a final concentration of 0.025%), and inactivating it at 4 °C for 72 hours. Whether the virus was inactivated was determined by confirming that the virus did not proliferate after inoculating another hatching egg with the virus. As a positive control, inactivated H3N2 or H3N8 canine influenza viruses were mixed and then inoculated at a dose of $10^6$ HAU or higher. As a negative control, PBS without antigen was inoculated and used.

PBS was administered to the group (N.C) that did not contain antigen as a control.

In addition, the second administration was performed 4 weeks after the first administration in the same manner as the first administration (second immunization). Blood was collected from the mice at 2 weeks and 4 weeks after the first administration, and 2 weeks after the second administration (6 weeks after the first administration), and serum was obtained (Figure 24).

**Example 10.3. Evaluation of neutralizing antibody production**

The production of neutralizing antibodies was evaluated using serum obtained from the laboratory mice of Example 10.2 above (4 weeks and 6 weeks after the first administration). At this time, the above serum was prepared by mixing at a ratio of 1:3 (serum:RDE) using the RDE Receptor Destroying Enzyme kit (SEIKEN), reacting at 37 °C for 18 hours, and then inactivating RDE at 56 °C for 30 minutes.

The formation of neutralizing antibodies was evaluated in two ways using a hemagglutination inhibition reaction and a cell experiment. The hemagglutination inhibition reaction test and the cell experiment were performed in the same manner as in Example 8.2 above. At this time, the blood cells were treated with the canine influenza H3N2 virus (A/canine/Korea/AS-01/2012) at the level of HAU 4-8 (HA unit), or diluted to TCID100, mixed with serum at a ratio of 1:1, reacted at 37 °C for 1 hour, and then used in the cell experiment.

For the canine influenza H3N8 virus (A/canine/Massachusetts/26810/2016), the virus was produced in-house and used, and the blood cells were treated therewith at the same level as above at the HAU 4-8 (HA unit) level, or diluted to TCID100, mixed with serum at a ratio of 1:1, reacted at 37 °C for 1 hour, and then used in cell experiments.

At this time, the canine influenza H3N8 virus was produced by the following method.

The used cells were MDCK (Marbin-Darby canine kidney) cells and 293T cells, and co-cultured by inoculating them in a 6-well plate at a ratio of 1:2 (MDCK cells: $0.25 \times 10^6$ cells/well, 293T cells: $0.5 \times 10^6$ cells/well) the day before transfection.

24 hours after inoculation, the medium was replaced with a medium without fetal bovine serum, and then the cells were used in the experiment. Canine influenza viruses were prepared by loading polynucleotides (SEQ ID NO: 169, SEQ ID NO: 171, SEQ ID NO: 173, SEQ ID NO: 183, SEQ ID NO: 185, SEQ ID NO: 177, SEQ ID NO: 179, SEQ ID NO: 181) encoding the amino acid sequence of PB2 (SEQ ID NO: 170), PB1 (SEQ ID NO: 172), PA (SEQ ID NO: 174), H3N2-HA (SEQ ID NO: 184), H3N8-HA (SEQ ID NO: 186), NP (SEQ ID NO: 178), NA (SEQ ID NO: 180) or M (SEQ ID NO: 182) into the PHW2000 vector (Hoffmann et al ., PNAS, 97:6108-6113 (2000)), and then mixing equal amounts (1 μg) of each vector (plasmid DNA).

The plasmid DNA prepared as described above and transfection reagent (TransIT-LT1, Mirus Bio) (18 μl) were mixed in an EP tube and reacted at room temperature for 3 minutes. After the reaction, a medium (194 μl) without fetal bovine serum was added and reacted for an additional 45 minutes at room temperature. The prepared MDCK cells and 293T cells were treated with the reaction solution and transfected by culturing at 37 °C for 24 hours. 24 hours after transfection, the cell culture solution was replaced with a medium (1 ml) without fetal bovine serum and cultured for an additional 24 hours. Then, a medium without fetal bovine serum containing 1 μg/ml of TPCK was added and cultured for 48 hours. After 48 hours, the cell culture solution was obtained and used as a transfection sample.

In order to confirm the production of virus after transfection, MDCK cells were prepared by inoculating at a concentration of $2.5 \times 10^5$ cells/well in a 6-well plate and culturing for 24 hours. After 24 hours, the cell culture solution was replaced with MEM medium (Corning, 10-010-CV) without fetal bovine serum, and then the cells were treated with the transfection sample (1 ml) prepared by the above method and reacted at 37 °C for 1 hour. A medium without fetal bovine serum containing 1 μg/ml of TPCK was added, and the cells were cultured for 48 hours, and then the production of virus was confirmed through hemagglutination assay, and the production of canine influenza H3N8 virus was confirmed before use.

At this time, the hemagglutination reaction was prepared by serially diluting PBS and virus 1/2 each 11 times in a 96-well round bottom plate, and the reaction solution was treated with 0.5% turkey red blood cell dilution solution at a ratio of 1:1, and then reacted at room temperature for 30 minutes. At this time, if the virus was generated, it was confirmed that the virus and blood cells were bound.

As a result, as shown in Figures 25a and 25b, it was confirmed that neutralizing antibodies were formed in all groups administered with self-amplifying RNA after the second administration. At this time, the self-amplifying RNA comprising H3N2 showed a titer of 1024 MNT in the group administered with 1 μg and the group administered with 5 μg, and the self-amplifying RNA comprising H3N8 showed a titer of 40960MNT in the group administered with 1 μg and the group administered with 5 μg. In addition, high neutralizing antibody production was confirmed even in the group administered with 0.1 μg.

**Example 10.4. Measurement of change in body weight and survival rate of mice**

The self-amplifying RNA was administered to the mice in the same manner as in Example 10.1 above (first and second administration). At this time, 4 weeks after the first administration (28 days after vaccination, 28 dpv) and 2 weeks after the second administration (6 weeks after the first administration, 42 days after vaccination (42 dpv)), all administration groups except the control were infected via the nasal cavity with H3N2 or H3N8 canine influenza virus (10MLD), respectively, and the body weight and survival rate of the mice were monitored for 14 days. In addition, some mice were sacrificed on the 3rd and 5th days after infection, lung tissue was extracted, and the virus titer in the lung tissue was measured (Figure 26).

As a result, as shown in Figures 27a to 27d, it was confirmed that the survival rate was high and the body weight loss was low in the group administered with multiple-antigen self-amplifying RNA (S.A.H3N2+H3N8) compared to the group administered with single-antigen self-amplifying RNA (S.A.H3N2 or S.A.H3H8).

In addition, as shown in Figures 28a and 28b, in the case of mice infected with the virus 4 weeks (28 dpv) after the first vaccine administration, it was confirmed that the virus titer in the lung tissue was reduced in the group administered with multiple-antigen self-amplifying RNA (S.A.H3N2+H3N8) compared to the group administered with single-antigen self-amplifying RNA (S.A.H3N2 or S.A.H3H8). In particular, when examining the lung tissues extracted on the 5th day (5 dpi) after virus infection, it was confirmed that the proliferation of the H3N2 virus was significantly reduced in the lung tissues of mice infected with the H3N2 virus, and no proliferation of the virus was observed in the lung tissues of mice infected with the H3N8 virus (Figure 28a). In addition, in the case of mice infected with the virus 2 weeks (42 dpv) after the second vaccine administration, it was confirmed that the survival rate of all mice was higher and the body weight loss was less in the group administered with self-amplifying RNA compared to the control. In addition, it was confirmed that no viruses proliferated in the lung tissues extracted 3 and 5 days after infection (Figure 28b).

Through the above results, it was confirmed that the vaccine efficacy was superior in the case of administering H3N2 antigen and H3N8 antigen in combination compared to the case of administering H3N2 antigen or H3N8 antigen alone, and it was confirmed that in the case of administration in combination, sufficient virus inhibition effect could be achieved with only the first administration.

**Example 11. Evaluation of efficacy in preventing or treating cancer caused by human papillomavirus**

**Example 11.1. Production of self-amplifying RNA comprising human papillomavirus antigen and confirmation of expression at the cellular level**

The following fusion protein was used as a human papillomavirus antigen (E6/E7 domain, sequential arrangement of E6/E7, cross over arrangement of E6/E7 (Cross over), odd and even arrangement of E6/E7) (Figure 29a).

The E6/E7 domain (SEQ ID NO: 139) is a fusion protein comprising the amino acid sequences of the E6 domain (SEQ ID NO: 159), linker (SEQ ID NO: 189, GGGGS), and E7 domain (SEQ ID NO: 160) of human papillomavirus in order from the N-terminus to the C-terminus.

The sequential arrangement of E6/E7 (Continuous) (SEQ ID NO: 142) is a fusion protein that divides the E6 domain (SEQ ID NO: 159) and E7 domain into 8 fragments and 7 fragments, respectively, and sequentially comprises the E6 domain and E7 domain from the N-terminus to the C-terminus.

Specifically, the E6 domain was divided into a fragment (E6-1) consisting of the 18th to 43rd amino acid sequence of the amino acid sequence of SEQ ID NO: 159, a fragment (E6-2) consisting of the 28th to 61st amino acid sequence of the amino acid sequence of SEQ ID NO: 159, a fragment (E6-3) consisting of the 42nd to 70th amino acid sequence of the amino acid sequence of SEQ ID NO: 159, a fragment (E6-4) consisting of the 48th to 79th amino acid sequence of the amino acid sequence of SEQ ID NO: 159, a fragment (E6-5) consisting of the 66th to 92nd amino acid sequence of the amino acid sequence of SEQ ID NO: 159, a fragment (E6-6) consisting of the 82nd to 122nd amino acid sequence of the amino acid sequence of SEQ ID NO: 159, a fragment (E6-7) consisting of the 100th to 143rd amino acid sequence of the amino acid sequence of SEQ ID NO: 159, and a fragment (E6-8) consisting of the 126th to 151th amino acid sequence of the amino acid sequence of SEQ ID NO: 159. The E7 domain was divided into a fragment (E7-1) consisting of the 5th to 21st amino acid sequence of the amino acid sequence of SEQ ID NO: 160, a fragment (E7-2) consisting of the 9th to 33rd amino acid sequence of the amino acid sequence of SEQ ID NO: 160, a fragment (E7-3) consisting of the 24th to 45th amino acid sequence of the amino acid sequence of SEQ ID NO: 160, a fragment (E7-4) consisting of the 36th to 64th amino acid sequence of the amino acid sequence of SEQ ID NO: 160, a fragment (E7-5) consisting of the 47th to 69th amino acid sequence of the amino acid sequence of SEQ ID NO: 160, a fragment (E7-6) consisting of the 65th to 88th amino acid sequence of the amino acid sequence of SEQ ID NO: 160, and a fragment (E7-7) consisting of the 72nd to 95th amino acid sequence of the amino acid sequence of SEQ ID NO: 160. The sequential arrangement of E6/E7 (Continuous) is a fusion protein that sequentially comprises the above respective fragments in order from the N-terminus to the C-terminus as E6-1, E6-2, E6-3, E6-4, E6-5, E6-6, E6-7, E6-8, E7-1, E7-2, E7-3, E7-4, E7-5, E7-6, and E7-7.

The cross over arrangement of E6/E7 is a fusion protein that sequentially comprises the E6 domain fragments (8 types) and the E7 domain fragments (7 types) in order from the N-terminus to the C-terminus as E6-1, E7-1, E6-2, E7-2, E6-3, E7-3, E6-4, E7-4, E6-5, E7-5, E6-6, E7-6, E6-7, E7-7, and E6-8.

The odd and even arrangement of E6/E7 is a fusion protein that sequentially comprises the E6 domain fragments (8 types) and the E7 domain fragments (7 types) in order from the N-terminus to the C-terminus as E6-1, E6-3, E6-5, E6-7, E6-2, E6-4, E6-6, E6-8, E7-1, E7-3, E7-5, E7-7, E7-2, E7-4, and E7-6.

The self-amplifying RNA constructs comprising the E6/E7 domain (SEQ ID NO: 139), the sequential arrangement of E6/E7 (Continuous) (SEQ ID NO: 142), the cross over arrangement of E6/E7 (Cross over) (SEQ ID NO: 145), or the odd and even arrangement of E6/E7 (Odd-Even) (SEQ ID NO: 148) of the human papillomavirus (type 16) described above were produced in the same manner as in Example 5.1, and each self-amplifying RNA was designated as G1, G2, G3, and G4 (also referred to as target protein). The nucleotide sequences of each self-amplifying RNA are shown in Tables 14 and 15.

[Table 14]

|  | HPV E6/E7 domain antigen saRNA | | HPV E6/E7 sequential arrangement antigen saRNA | |
| --- | --- | --- | --- | --- |
|  | DNA vector (SEQ ID NO: 152) | saRNA (G1) (SEQ ID NO: 151) | DNA vector (SEQ ID NO: 154) | saRNA (G2) (SEQ ID NO: 153) |
| 5' UTR | SEQ ID NO: 1 | SEQ ID NO: 60 | SEQ ID NO: 1 | SEQ ID NO: 60 |
| Kozak sequence | SEQ ID NO: 35 | SEQ ID NO: 35 | SEQ ID NO: 35 | SEQ ID NO: 35 |
| Target protein | SEQ ID NO: 141 | SEQ ID NO: 140 | SEQ ID NO: 144 | SEQ ID NO: 143 |
| 2A protease recognize site | SEQ ID NO: 28 | SEQ ID NO: 33 | SEQ ID NO: 28 | SEQ ID NO: 33 |
| 2A protease | SEQ ID NO: 2 | SEQ ID NO: 61 | SEQ ID NO: 2 | SEQ ID NO: 61 |
| 2B | SEQ ID NO: 3 | SEQ ID NO: 62 | SEQ ID NO: 3 | SEQ ID NO: 62 |
| 2C | SEQ ID NO: 4 | SEQ ID NO: 63 | SEQ ID NO: 4 | SEQ ID NO: 63 |
| 3A | SEQ ID NO: 5 | SEQ ID NO: 64 | SEQ ID NO: 5 | SEQ ID NO: 64 |
| 3B | SEQ ID NO: 6 | SEQ ID NO: 65 | SEQ ID NO: 6 | SEQ ID NO: 65 |
| 3C protease | SEQ ID NO: 7 | SEQ ID NO: 66 | SEQ ID NO: 7 | SEQ ID NO: 66 |
| 3D | SEQ ID NO: 8 | SEQ ID NO: 67 | SEQ ID NO: 8 | SEQ ID NO: 67 |
| Stop sequence | SEQ ID NO: 9 | SEQ ID NO: 68 | SEQ ID NO: 9 | SEQ ID NO: 68 |
| 3' UTR | SEQ ID NO: 10 | SEQ ID NO: 69 | SEQ ID NO: 10 | SEQ ID NO: 69 |
| poly(A) tail | SEQ ID NO: 40 | SEQ ID NO: 40 | SEQ ID NO: 40 | SEQ ID NO: 40 |

[Table 15]

|  | HPV E6/E7 cross over arrangement antigen saRNA | | HPV E6/E7 odd and even arrangement saRNA | |
| --- | --- | --- | --- | --- |
|  | DNA vector (SEQ ID NO: 156) | saRNA (G3) (SEQ ID NO: 155) | DNA vector (SEQ ID NO: 158) | saRNA (G4) (SEQ ID NO: 157) |
| 5' UTR | SEQ ID NO: 1 | SEQ ID NO: 60 | SEQ ID NO: 1 | SEQ ID NO: 60 |
| Kozak sequence | SEQ ID NO: 35 | SEQ ID NO: 35 | SEQ ID NO: 35 | SEQ ID NO: 35 |
| Target protein | SEQ ID NO: 147 | SEQ ID NO: 146 | SEQ ID NO: 149 | SEQ ID NO: 148 |
| 2A protease recognize site | SEQ ID NO: 28 | SEQ ID NO: 33 | SEQ ID NO: 28 | SEQ ID NO: 33 |
| 2A protease | SEQ ID NO: 2 | SEQ ID NO: 61 | SEQ ID NO: 2 | SEQ ID NO: 61 |
| 2B | SEQ ID NO: 3 | SEQ ID NO: 62 | SEQ ID NO: 3 | SEQ ID NO: 62 |
| 2C | SEQ ID NO: 4 | SEQ ID NO: 63 | SEQ ID NO: 4 | SEQ ID NO: 63 |
| 3A | SEQ ID NO: 5 | SEQ ID NO: 64 | SEQ ID NO: 5 | SEQ ID NO: 64 |

(continued)

| | HPV E6/E7 cross over arrangement antigen saRNA | | HPV E6/E7 odd and even arrangement saRNA | |
|---|---|---|---|---|
| | DNA vector (SEQ ID NO: 156) | saRNA (G3) (SEQ ID NO: 155) | DNA vector (SEQ ID NO: 158) | saRNA (G4) (SEQ ID NO: 157) |
| 3B | SEQ ID NO: 6 | SEQ ID NO: 65 | SEQ ID NO: 6 | SEQ ID NO: 65 |
| 3C protease | SEQ ID NO: 7 | SEQ ID NO: 66 | SEQ ID NO: 7 | SEQ ID NO: 66 |
| 3D | SEQ ID NO: 8 | SEQ ID NO: 67 | SEQ ID NO: 8 | SEQ ID NO: 67 |
| Stop sequence | SEQ ID NO: 9 | SEQ ID NO: 68 | SEQ ID NO: 9 | SEQ ID NO: 68 |
| 3' UTR | SEQ ID NO: 10 | SEQ ID NO: 69 | SEQ ID NO: 10 | SEQ ID NO: 69 |
| poly(A) tail | SEQ ID NO: 40 | SEQ ID NO: 40 | SEQ ID NO: 40 | SEQ ID NO: 40 |

The expression of the self-amplifying RNA constructs produced as described above was confirmed at the cellular level in the same manner as in Example 5.2, and a human papillomavirus antigen was produced.

**Example 11.2. Evaluation of cancer prevention activity**

The self-amplifying RNA produced in the same manner as in Example 11.1 above was encapsulated (RNA-LNP) in the same manner as in Example 10.1 above, and administered intramuscularly to each mouse (C57/BL6, 6 weeks old, female, n = 8) at a concentration of 5 µg/50 µl/mouse (first immunization). Two weeks after the first administration, the second administration was performed in the same manner as above (second immunization). Three weeks after the second administration, the TC-1 tumor cell line was transplanted into the flank of the mouse through subcutaneous injection at a concentration of $2 \times 10^5$ cells/100 µl/mouse, and the body weight and tumor size of the mouse were measured 15 times for 19 days. At this time, PBS was administered to the control, and the group administered with G1 was used as a positive control.

In addition, the tumor size was calculated by measuring the width (W), length (L), and height (H) using the following mathematical formula I (https:/biopticon.com/resources/tumor-volume-measurements-by-calipers/).

$$\text{<Mathematical formula I>}$$

$$\text{Tumor size } (V_{(t)}) = 6/\pi \times L \times W \times H10$$

Blood was collected from the mice at 2 weeks of the first administration and 2 weeks after the second administration (4 weeks after the first administration), and plasma was obtained, and some mice (n = 3) were sacrificed at 2 weeks after the second administration, and the spleen was extracted (Figure 30).

**Example 11.2.1. Confirmation of T cell response**

Four weeks after the first vaccination, the second vaccination was performed. Two weeks later, the spleen was extracted to obtain cells, and then experiments were conducted. At this time, mice that were not injected with the TC-1 tumor cell line (n = 3) were used. In addition, the group administered with G1 was used as a positive control.

Specifically, the capture antibody included in the BD™ ELISPOT Mouse IFNγ ELISPOT Set (BD, 551083) was diluted in DPBS at a ratio of 1:200, and then 100 µl was added to each well of each 96-well plate, and then coated by reacting at 4 °C for 18 hours. Thereafter, each well was washed with DPBS (Welgene) and blocked with RPMI 1640 medium containing 10% heat-treated BCS (bovine calf serum, Welgene) and 1% penicillin/streptomycin (Gibco). Then, the spleen cells isolated from the laboratory mice of Example 11.2 above (mice not injected with the TC-1 tumor cell line, n = 3) were inoculated into each well at a concentration of $1.0 \times 10^6$ cells/well, and then the cells were stimulated by treating them with 20 µg/ml of E6 and E7 peptides (Miltenyibiotec, 130-095-997 and 130-095-999). At this time, the spleen cells of naive mice were used as a negative control. Thereafter, each well was washed three times with PBS-T (200 µl), and then the primary antibody (BD™ ELISPOT Mouse IFNγ ELISPOT Set, BD, 551083) was diluted in DPBS containing 10% BCS at a ratio of 1:250 and added to each well (100 µl) for reaction. Thereafter, the cells were treated with the secondary antibody (BD™ ELISPOT Mouse IFNγ ELISPOT Set, BD, 551083), and the number of spots generated through the HRP substrate reaction was counted.

As a result, as shown in Figure 31, the lowest T cell response was observed in the group administered with G1, and high T cell responses were observed in the groups administered with G2 to G4.

**Example 11.2.2. Cancer prevention effect of** self-amplifying **RNA**

After transplanting tumors in the laboratory mice of Example 11.2 above, the body weight and tumor size of the mice were monitored for 19 days after vaccination.

As a result, it was confirmed that tumor formation and growth were inhibited in all of the group administered with G1 (positive control), the group administered with G2, the group administered with G3, and the group administered with G4 compared to the negative control (PBS). In particular, tumor formation was observed only in the group administered with G1, which is the positive control (2 mice), and tumor formation was not observed in the other administration groups. Through the above results, it was confirmed that self-amplifying RNA can be sufficiently used as a cancer prevention vaccine.

**Example 11.3. Evaluation of tumor growth inhibitory activity**

In order to confirm the tumor growth inhibition effect, TC-1 cells were transplanted into the flank of mice (C57/BL6, 6 weeks old, female) at a concentration of $1 \times 10^5$ cells/100 $\mu$l per mouse via subcutaneous injection. On the 3rd and 7th days after tumor cell transplantation, self-amplifying RNA was administered (only the first administration was performed) in the same manner as in Example 11.2 above, and the body weight and tumor size of the mice were measured for 22 days (a total of 22 times). At this time, the concentration of self-amplified RNA administered to each mouse was 5 $\mu$g/50 $\mu$l/mouse or 1 $\mu$g/50 $\mu$l/mouse (Figure 34). At this time, PBS was administered to the negative control.

As a result, as shown in Figure 35 and Figures 36a to 36d, it was confirmed that tumor growth was inhibited in all groups administered with 5 $\mu$g of self-amplifying RNA (vaccine) compared to the negative control (PBS). In addition, in the case of the group administered with G4, it was confirmed that tumor growth was inhibited in all laboratory mice even in the group administered with 1 $\mu$g.

Through the above results, it was confirmed that tumor growth was inhibited or tumor was eliminated with only the first administration of self-amplifying RNA.

**Claims**

1. A polynucleotide comprising a nucleic acid sequence encoding a fusion protein, comprising:

   a non-structural protein of a self-amplifying virus;
   a first target protein; and
   a protease cleavage site.

2. The polynucleotide according to claim 1, wherein the non-structural protein is derived from a non-structural protein of a virus of the family Picornaviridae.

3. The polynucleotide according to claim 2, wherein the non-structural protein comprises a P2 domain and a P3 domain of enterovirus.

4. The polynucleotide according to claim 3, wherein the P2 domain comprises 2A protease, 2B, and 2C.

5. The polynucleotide according to claim 4, wherein

   the 2A protease comprises a nucleotide sequence encoding the amino acid sequence of SEQ ID NO: 11,
   the 2B comprises a nucleotide sequence encoding the amino acid sequence of SEQ ID NO: 12, and
   the 2C comprises a nucleotide sequence encoding the amino acid sequence of SEQ ID NO: 13.

6. The polynucleotide according to claim 4, wherein the 2A protease comprises a mutation.

7. The polynucleotide according to claim 6, wherein the mutation is a substitution of the 87th amino acid in the amino acid sequence of SEQ ID NO: 11.

8. The polynucleotide according to claim 7, wherein the mutation comprises the amino acid sequence of SEQ ID NO: 75.

9. The polynucleotide according to claim 3, wherein the P3 domain comprises 3A, 3B, 3C protease, and 3D polymerase.

10. The polynucleotide according to claim 9, wherein

the 3A comprises a nucleotide sequence encoding the amino acid sequence of SEQ ID NO: 14,
the 3B comprises a nucleotide sequence encoding the amino acid sequence of SEQ ID NO: 15,
the 3C protease comprises a nucleotide sequence encoding the amino acid sequence of SEQ ID NO: 16, and
the 3D polymerase comprises a nucleotide sequence encoding the amino acid sequence of SEQ ID NO: 17.

11. The polynucleotide according to claim 1, wherein the protease cleavage site is cleaved by a protease derived from a non-structural protein of a self-amplifying virus.

12. The polynucleotide according to claim 11, wherein the protease cleavage site is cleaved by 2A protease of enterovirus.

13. The polynucleotide according to claim 12, wherein the protease cleavage site comprises a nucleotide sequence encoding the amino acid sequence of SEQ ID NO: 22.

14. The polynucleotide according to claim 1, wherein the polynucleotide further comprises a nucleic acid sequence encoding a second target protein.

15. The polynucleotide according to claim 14, wherein the protease cleavage site is located between the second target protein and the first target protein.

16. The polynucleotide according to claim 15, wherein the protease cleavage site is derived from a non-structural protein of a self-amplifying virus.

17. The polynucleotide according to claim 16, wherein the protease cleavage site is cleaved by a protease derived from a virus of the family Picornaviridae.

18. The polynucleotide according to claim 17, wherein the protease cleavage site is cleaved by 2A protease, 3CD protease, or 3C protease of enterovirus.

19. The polynucleotide according to claim 18, wherein the protease cleavage site comprises a nucleotide sequence encoding the amino acid sequence of SEQ ID NO: 22 or SEQ ID NO: 50.

20. The polynucleotide according to claim 1 or 14, wherein the first target protein and/or the second target protein is an antigen.

21. The polynucleotide according to claim 20, wherein the antigen is a severe fever with thrombocytopenia syndrome virus antigen.

22. The polynucleotide according to claim 21, wherein the antigen comprises any one amino acid sequence selected from the group consisting of SEQ ID NO: 45, SEQ ID NO: 85, SEQ ID NO: 116, and SEQ ID NO: 119.

23. The polynucleotide according to claim 20, wherein the antigen is a SARS-CoV2 virus antigen.

24. The polynucleotide according to claim 23, wherein the antigen comprises the amino acid sequence of SEQ ID NO: 48.

25. The polynucleotide according to claim 20, wherein the antigen is a highly pathogenic avian influenza virus antigen.

26. The polynucleotide according to claim 25, wherein the antigen comprises the amino acid sequence of SEQ ID NO: 105.

27. The polynucleotide according to claim 20, wherein the antigen is a canine influenza virus antigen.

28. The polynucleotide according to claim 27, wherein the antigen comprises the amino acid sequence of SEQ ID NO: 53 or SEQ ID NO: 56.

29. The polynucleotide according to claim 20, wherein the antigen is a human papillomavirus antigen.

30. The polynucleotide according to claim 29, wherein the antigen comprises any one amino acid sequence selected from the group consisting of SEQ ID NO: 139, SEQ ID NO: 142, SEQ ID NO: 145, and SEQ ID NO: 148.

31. The polynucleotide according to claim 20, wherein the antigen is a tumor antigen.

32. The polynucleotide according to claim 31, wherein the tumor antigen is selected from the group consisting of 5T4, 707-AP, 9D7, AFP, AlbZIP HPG1, $\alpha$-5-$\beta$-1-integrin, $\alpha$-5-$\beta$-6-integrin, $\alpha$-actinin-4/m, $\alpha$-methylacyl-coA racemase, ART-4, ARTC1/m, B7H4, BAGE-1, BCL-2, ber/abl, $\beta$-catenin/m, BING-4, BRCA1/m, BRCA2/m, CA 15-3/CA 27-29, CA 19-9, CA72-4, CA125, calreticulin, CAMEL, CASP-8/m, cathepsin B, cathepsin L, CD19, CD20, CD22, CD25, CD30, CD33, CD4, CD52, CD55, CD56, CD80, CDC27/m, CDK4/m, CDKN2A/m, CEA, CLCA2, CML28, CML66, COA-1/m, coactosin-like protein (COTL1), collagen type XXIII, COX-2, CT_9/BRD6, Cten, cyclin B1, cyclin D1, CypB, CYPB1, DAM-10, DAM-6, DEK-CAN, EFTUD2/m, EGFR, ELF2/m, EMMPRIN, EpCam, EphA2, EphA3, ErbB3, ETV6-AML1, EZH2, FGF-5, FN, Frau-1, G250, GAGE-1, GAGE-2, GAGE-3, GAGE-4, GAGE-5, GAGE-6, GAGE7b, GAGE-8, GDEP, GnT-V, gp100, GPC3, GPNMB/m, HAGE, HAST-2, hepsin, Her2/neu, HERV-K-MEL, HLA-A*0201-R17I, HLA-A11/m, HLA-A2/m, HNE, NKX3.1, HOM-TES-14/SCP-1, HOM-TES-85, HPV-E6, HPV-E7, HSP70-2M, HST-2, hTERT, iCE, IGF-1R, IL-13Ra2, IL-2R, IL-5, immature laminin Receptor, kallikrein-2(KLK2), kallikrein-4(LKL4), Ki67, KIAA0205, KIAA0205/m, KK-LC-1, K-Ras/m, LAGE-A1, LDLR-FUT, MAGE-A1, MAGE-A2, MAGE-A3, MAGE-A4, MAGE-A6, MAGE-A9, MAGE-A10, MAGE-A12, MAGE-B1, MAGE-B2, MAGE-B3, MAGE-B4, MAGE-B5, MAGE-B6, MAGE-B10, MAGE-B16, MAGE-B17, MAGE-C1, MAGE-C2, MAGE-C3, MAGE-D1, MAGE-D2, MAGE-D4, MAGE-E1, MAGE-E2, MAGE-F1, MAGE-H1, MAGEL2, mammaglobin-A, MART-1/melan-A, MART-2, MART_2/m, matrix protein 22, MC1R, M-CSF, ME1/m, mesothelin, MG50/PXDN, MMP11, MN/CA IX-antigen, MRP-3, MUC-1, MUC-2, MUM-1/m, MUM-2/m, MUM-3/m, myosin class I/m, NA88-A, N-acetylglucosaminyltransferase-V, Neo-PAP, Neo-PAP/m, NFYC/m, NGEP, NMP22, NPM/ALK, N-Ras/m, NSE, NY-ESO-1, NY-ESO-B, OA1, OFA-iLRP, OGT, OGT/m, OS-9, OS-9/m, osteocalcin, osteopontin, p15, p190 minor ber-abl, p53, p53/m, PAGE-4, PAI-1, PAI-2, PART-1, PATE, PDEF, Pim-1-kinase, Pin-1, Pml/PARalpha, POTE, PRAME, PRDX5/m, prostein, proteinase-3 (PR3), PSA, PSCA, PSGR, PSM, PSMA, PTPRK/m, RAGE-1, BAF600/m, RHAMM/CD168, RU1, RU2, S-100, SAGE, SART-1, SART-2, SART-3, SCC, SIRT2/m, Sp17, SSX-1, SSX_2/HOM-MEL-40, SSX-4, STAMP-1, STEAP, survivin, survivin-2B, SYT-SSX-1, SYT-SSX-2, TA-90, TAG-72, TARP, TEL-AML1, TGF$\beta$, TGF$\beta$RII, TGM-4, TPI/m, TRAG-3, TRG, TRP-1, TRP-2/6b, TRP/INT2, TRP-p8, tyrosinase, UPA, VEGF, VEGFR-2/FLK-1, and WT1.

33. The polynucleotide according to claim 1, wherein the polynucleotide further comprises at least one stop codon at the 3' terminus of a nucleic acid sequence encoding a non-structural protein of a self-amplifying virus.

34. The polynucleotide according to claim 33, wherein the stop codon comprises the nucleotide sequence of SEQ ID NO: 9 or SEQ ID NO: 68.

35. The polynucleotide according to claim 1, wherein the polynucleotide further comprises a 5' untranslated region (5' UTR) at the 5' terminus.

36. The polynucleotide according to claim 35, wherein the 5' untranslated region comprises an IRES (internal ribosomal entry site).

37. The polynucleotide according to claim 36, wherein the IRES comprises the nucleotide sequence of SEQ ID NO: 58 or SEQ ID NO: 59.

38. The polynucleotide according to claim 37, wherein the IRES comprises a mutation.

39. The polynucleotide according to claim 38, wherein the mutation is

a substitution of any one nucleotide selected from the group consisting of the 236th, 386th, and a combination thereof in the nucleotide sequence of SEQ ID NO: 58; or
a substitution of any one nucleotide selected from the group consisting of the 236th, 386th, and a combination thereof in the nucleotide sequence of SEQ ID NO: 59.

40. The polynucleotide according to claim 39, wherein the mutation comprises the nucleotide sequence of SEQ ID NO: 81 or SEQ ID NO: 82.

41. The polynucleotide according to claim 1, wherein the polynucleotide further comprises a Kozak sequence.

42. The polynucleotide according to claim 41, wherein the Kozak sequence comprises the nucleotide sequence of SEQ ID NO: 35.

43. The polynucleotide according to claim 1, wherein the polynucleotide further comprises a untranslated region (3' UTR) at the 3' terminus of the polynucleotide.

44. The polynucleotide according to claim 35 or 43, wherein the untranslated region is derived from a self-amplifying virus.

45. The polynucleotide according to claim 44, wherein the untranslated region is derived from a virus of the family Picornaviridae.

46. The polynucleotide according to claim 1, wherein the polynucleotide further comprises a poly(A) tail (poly(A) tail) sequence.

47. The polynucleotide according to claim 1, wherein the polynucleotide is composed of the following structural formula (I) in order from the 5' terminus to the 3' terminus:

$$5'\text{-}B\text{-}C(1)\text{-}D\text{-}3' \qquad (I)$$

in the structural formula (I),
5' and 3' are the 5' terminus and the 3' terminus of the polynucleotide, respectively,
B is a nucleic acid sequence encoding a first target protein,
C(1) is a nucleic acid sequence encoding a protease cleavage site, and
D is a nucleic acid sequence encoding a non-structural protein of a self-amplifying virus.

48. The polynucleotide according to claim 47, wherein the polynucleotide is composed of the following structural formula (II) in order from the 5' terminus to the 3' terminus:

$$5'\text{-}U\text{-}[K]o\text{-}[B'\text{-}C(2)]n\text{-}B\text{-}C(1)\text{-}D\text{-}S\text{-}U'\text{-}P\text{-}3' \qquad (II)$$

in the structural formula (II),
5' and 3' are the 5' terminus and the 3' terminus of the polynucleotide, respectively,
U is a nucleic acid sequence of a 5' untranslated region,
K is a Kozak sequence,
B' is a nucleic acid sequence encoding a second target protein,
B is a nucleic acid sequence encoding a first target protein,
D is a nucleic acid sequence encoding a non-structural protein of a self-amplifying virus,
S is a stop codon,
U' is a nucleic acid sequence of a 3' untranslated region,
P is a poly(A) tail,
C(1) and C(2) are each independently a nucleic acid sequence encoding a protease cleavage site,
n is an integer from 0 to 10, and when n is 2 to 10, B' is each a nucleic acid sequence encoding the same or different target protein, and C(2) is also each a nucleic acid sequence encoding the same or different protease cleavage site, and
o is an integer of 0 or 1.

49. The polynucleotide according to claim 47 or 48, wherein the non-structural protein comprises a P2 domain and a P3 domain of enterovirus in order from the N-terminus to the C-terminus.

50. The polynucleotide according to claim 49, wherein the P2 domain comprises 2A protease, 2B, and 2C in order from the N-terminus to the C-terminus.

51. The polynucleotide according to claim 49, wherein the P3 domain comprises 3A, 3B, 3C, and 3D in order from the N-terminus to the C-terminus.

52. The polynucleotide according to claim 1, wherein the polynucleotide is DNA or RNA.

53. A recombinant vector comprising the polynucleotide according to claim 1.

54. A method for producing an RNA replicon, comprising:

       i) producing the recombinant vector according to claim 53; and
       ii) synthesizing RNA from the recombinant vector.

55. A vaccine composition comprising the polynucleotide according to claim 1 or the recombinant vector according to claim 53 as an active ingredient.

56. A non-structural protein 2A protease cleavage site derived from enterovirus, comprising the amino acid sequence of SEQ ID NO: 22.

57. A non-structural protein 3C protease cleavage site derived from enterovirus, comprising the amino acid sequence of SEQ ID NO: 50.

58. An IRES comprising the nucleic acid sequence of SEQ ID NO: 81 or SEQ ID NO: 82.

59. A 5' untranslated region comprising the nucleic acid sequence of SEQ ID NO: 86 or SEQ ID NO: 87.

60. A Kozak sequence comprising the nucleic acid sequence of SEQ ID NO: 35.

61. A non-structural protein 2A protease derived from enterovirus, comprising the amino acid sequence of SEQ ID NO: 75.

62. A severe fever with thrombocytopenia syndrome (SFTS) virus antigen comprising the amino acid sequence of SEQ ID NO: 45, SEQ ID NO: 85, SEQ ID NO: 116, or SEQ ID NO: 119.

63. A human papillomavirus (HPV) antigen comprising the amino acid sequence of SEQ ID NO: 139, SEQ ID NO: 142, SEQ ID NO: 145, or SEQ ID NO: 148.

64. A method for preventing or treating a disease, comprising:
administering to a subject the polynucleotide according to claim 1, the recombinant vector according to claim 53, or the vaccine composition according to claim 55.

Figure 1a

Figure 1b

Figure 2a

| | | |
|---|---|---|
| (SEQ ID NO: 18) | T | GSCGQ | VP1-1a.a |
| (SEQ ID NO: 19) | AMQTT | GSCGQ | VP1-5a.a |
| (SEQ ID NO: 20) | RTEITAMQTT | GSCGQ | VP1-10a.a |
| (SEQ ID NO: 21) | GVTESRTEITAMQTT | GSCGQ | VP1-15a.a |
| (SEQ ID NO: 22) | NFEPTGVTESRTEITAMQTT | GSCGQ | VP1-20a.a |
| (SEQ ID NO: 23) | KAGNVNFEPTGVTESRTGITAMQTT | GSCGQ | VP1-25a.a |

⟶ 2A protease cleavage site

Figure 2b

Figure 3a

Figure 3b

Figure 4a

Figure 4b

50 P.A    70 P.A    120 P.A

Figure 5

Figure 6

Figure 7

Figure 8

Figure 9

Figure 10

Figure 11

Figure 12

Figure 13

Figure 14

Figure 15

Single-antigen self-amplifying RNA construct

Multiple-antigen self-amplifying RNA construct

LEALFQ/GPPVYR (SEQ ID NO: 50)

Figure 16

Figure 17

Figure 18

Figure 19

Figure 20a

Day 3 after infection

Group 1 : saRNA-2.3.4.4 10ug ①
Group 2 : saRNA-2.3.4.4 1ug ②
Group 3 : saRNA-2.3.4.4 0.1ug ③
Group 4 : saRNA-2.3.4.4 0.01ug ④
Group 5 : mRNA-2.3.4.4 10ug ⑤
Group 6 : mRNA-2.3.4.4 1ug ⑥
Group 7 : mRNA-2.3.4.4 0.1ug ⑦
Group 8 : mRNA-2.3.4.4 0.01ug ⑧
Group 9 : saRNA-B virus 10ug ⑨
Group 10 : Protein + adjuvant 5ug ⑩
Group 11 : Naive ⑪

Figure 20b

Day 5 after infection

Group 1 : saRNA-2.3.4.4 10ug ①
Group 2 : saRNA-2.3.4.4 1ug ②
Group 3 : saRNA-2.3.4.4 0.1ug ③
Group 4 : saRNA-2.3.4.4 0.01ug ④
Group 5 : mRNA-2.3.4.4 10ug ⑤
Group 6 : mRNA-2.3.4.4 1ug ⑥
Group 7 : mRNA-2.3.4.4 0.1ug ⑦
Group 8 : mRNA-2.3.4.4 0.01ug ⑧
Group 9 : saRNA-B virus 10ug ⑨
Group 10 : Protein + adjuvant 5ug ⑩
Group 11 : Naive ⑪

Figure 21a

Figure 21b

**In vivo antibody expression(FITC/BALB/c)**

Figure 21c

**Neutralizing antibody in serum(C57BL/6)**

Figure 22a

Figure 22b

## Prime vaccine S.N test

Figure 22c

Boost vaccine S.N test

Figure 22d

Second boost vaccine S.N test

Figure 23

Figure 24

In-vitro
Transcription RNA

LNP

Mouse (BALB/c)

1st Vaccination

2nd Vaccination

Blood
collection
at Week 2

Blood
collection
at Week 4

Blood
collection
at Week 6

Figure 25a

Canine influenza H3N2 virus

Figure 25b

Canine influenza H3N8 virus

Figure 26

Figure 27a

Figure 27b

## H3N8 monovalent

S.A H3N8 0.1ug
S.A H3N8 1ug
S.A H3N8 5ug
P.C (H3N2+H3N8)
N.C (PBS)

Weight change(%)

days post-infection(dpi)

S.A H3N8 0.1ug
S.A H3N8 1ug
S.A H3N8 5ug
P.C (H3N2+H3N8)
N.C

Survival rate(%)

days post-infection(dpi)

## H3N2/H3N8 bivalent

S.A H3N2/H3N8 0.1ug
S.A H3N2/H3N8 1ug
S.A H3N2/H3N8 5ug
P.C (H3N2+H3N8)
N.C (PBS)

Weight change(%)

days post-infection(dpi)

S.A H3N2+H3N8 0.1ug
S.A H3N2+H3N8 1ug
S.A H3N2+H3N8 5ug
P.C (H3N2+H3N8)
N.C

Survival rate(%)

days post-infection(dpi)

Figure 27c

Figure 27d

Canine influenza H3N2 challenge

Canine influenza H3N8 challenge

Figure 28a

EP 4 692 344 A1

Figure 28b

Figure 29a

Figure 29b

Figure 29c

saRNA-HPV expression

G1 Protein link
5  1  0.1  0.05  0.01  N.C
35kDa
DNA size : 813bp
Protein size : 32kDa

G2 Continuous
5  1  0.1  0.05  0.01  N.C
45kDa
DNA size : 1278bp
Protein size : 49kDa

G3 Cross-Over
5  1  0.1  0.05  0.01  N.C
45kDa
DNA size : 1278bp
Protein size : 49kDa

G4 Odd-Even
5  1  0.1  0.05  0.01  N.C
45kDa
DNA size : 1278bp
Protein size : 49kDa

1st antibody : E7-mouse
2nd antibody : mouse HRP

Figure 30

1st Immunization    2nd Immunization    Spleen Harvest

Week 0     Week 2     Week 4    Week 5

C57/BL6
(6 weeks old, female)
5ug

Blood collection     Blood collection    Tumor transplant $2 \times 10^5$ cells/mouse

Figure 31

**T cell response**

E6 — IFN-$\gamma$ secretory cells /$10^6$ splenocytes; PBS, G1, G2, G3, G4; NS

E7 — IFN-$\gamma$ secretory cells /$10^6$ splenocytes; PBS, G1, G2, G3, G4; NS, NS, *

Figure 32

**HPV vaccination**

$V_t (cm^3)$ vs days post-infection (dpi)

- PBS
- G1 : Protein GGGGS
- G2 : Continuous
- G3 : Cross-over
- G4 : Odd-even

Figure 33

1x10⁵ cells/mouse
Tumor transplant
LNP treatment

Day -3    Day 0

Figure 34

Figure 35

Figure 36a

Figure 36b

Figure 36c

Figure 36d

Figure 37

Figure 38

## INTERNATIONAL SEARCH REPORT

| | International application No. |
|---|---|
| | **PCT/KR2024/002461** |

| **A.** | **CLASSIFICATION OF SUBJECT MATTER** |
|---|---|

**C12N 15/11**(2006.01)i; **C12N 15/62**(2006.01)i; **C12N 9/48**(2006.01)i; **C07K 14/005**(2006.01)i; **A61K 39/12**(2006.01)i; **A61P 31/12**(2006.01)i

According to International Patent Classification (IPC) or to both national classification and IPC

| **B.** | **FIELDS SEARCHED** |
|---|---|

Minimum documentation searched (classification system followed by classification symbols)

C12N 15/11(2006.01); A61K 39/00(2006.01); A61K 39/12(2006.01); C12N 15/85(2006.01); C12N 15/86(2006.01); C12N 7/00(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 자가 증폭 RNA(self amplifying RNA), 비구조 단백질(non-structural protein), 프로테아제 절단 부위(protease cleavage site), 목적 단백질(target protein), 항원(antigen)

| **C.** | **DOCUMENTS CONSIDERED TO BE RELEVANT** | |
|---|---|---|

| Category* | Citation of document, with indication, where appropriate, of the relevant passages | Relevant to claim No. |
|---|---|---|
| X | KR 10-2022-0038669 A (IMPERIAL COLLEGE INNOVATIONS LIMITED) 29 March 2022 (2022-03-29)<br>See paragraphs [0003], [0010]-[0011], [0013], [0015]-[0016], [0025], [0034], [0183], [0184], [0191]-[0244], [0273], [0280]-[0283] and [0297]. | 1-2,14-15,20-21 ,23,25,27,29,31 -36,43-47,52-55 |
| Y | | 3-4,6,9,28,30, 41-42,49-51 |
| A | | 5,7-8,10-13,16-19,22, 24,26,37-40,48,56-63 |
| Y | LIU, Dan et al. Rescue and characterization of a recombinant HY12 bovine enterovirus carrying a foreign HA epitope in the 3A nonstructural protein. Archives of Virology. 15 March 2019 (online publication date), vol. 164, pp. 1309-1321.<br>See pages 1309-1310; and figure 1. | 3-4,6,9,49-51 |

| ✓ Further documents are listed in the continuation of Box C. | ✓ See patent family annex. |
|---|---|

| * | Special categories of cited documents: |
|---|---|
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **25 June 2024** | **25 June 2024** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**INTERNATIONAL SEARCH REPORT**

| International application No. |
| --- |
| **PCT/KR2024/002461** |

### C.    DOCUMENTS CONSIDERED TO BE RELEVANT

| Category* | Citation of document, with indication, where appropriate, of the relevant passages | Relevant to claim No. |
| --- | --- | --- |
| Y | GenBank: AFN69181.1 (18 February 2015).<br>    See entire document. | 28 |
| X | US 6004557 A (EDWARDS, Stirling John et al.) 21 December 1999 (1999-12-21)<br>    See claims 1 and 4; and SEQ ID NO: 10. | 63 |
| Y | | 30 |
| X | KR 10-2459458 B1 (NATIONAL INSTITUTE OF ADVANCED INDUSTRIAL SCIENCE AND TECHNOLOGY et al.) 25 October 2022 (2022-10-25)<br>    See paragraph [0426]; and SEQ ID NO: 57. | 60 |
| Y | | 41-42 |

Form PCT/ISA/210 (second sheet) (July 2022)

## INTERNATIONAL SEARCH REPORT

| | |
|---|---|
| International application No. | |
| **PCT/KR2024/002461** | |

**Box No. I**     **Nucleotide and/or amino acid sequence(s) (Continuation of item 1.c of the first sheet)**

1.  With regard to any nucleotide and/or amino acid sequence disclosed in the international application, the international search was carried out on the basis of a sequence listing:

    a.  ☑ forming part of the international application as filed.

    b.  ☐ furnished subsequent to the international filing date for the purposes of international search (Rule 13*ter*.1(a)),

        ☐ accompanied by a statement to the effect that the sequence listing does not go beyond the disclosure in the international application as filed.

2.  ☐ With regard to any nucleotide and/or amino acid sequence disclosed in the international application, this report has been established to the extent that a meaningful search could be carried out without a WIPO Standard ST.26 compliant sequence listing.

3.  Additional comments:

Form PCT/ISA/210 (continuation of first sheet) (July 2022)

**INTERNATIONAL SEARCH REPORT**

International application No.

**PCT/KR2024/002461**

| Box No. II | Observations where certain claims were found unsearchable (Continuation of item 2 of first sheet) |

This international search report has not been established in respect of certain claims under Article 17(2)(a) for the following reasons:

1. ☑ Claims Nos.: **64**
because they relate to subject matter not required to be searched by this Authority, namely:

Claim 64 pertains to a method for treatment of the human body by therapy (PCT Article 17(2)(a)(i) and PCT Rule 39.1(iv)).

2. ☐ Claims Nos.:
because they relate to parts of the international application that do not comply with the prescribed requirements to such an extent that no meaningful international search can be carried out, specifically:

3. ☑ Claims Nos.: **64**
because they are dependent claims and are not drafted in accordance with the second and third sentences of Rule 6.4(a).

Form PCT/ISA/210 (continuation of first sheet) (July 2022)

## INTERNATIONAL SEARCH REPORT
### Information on patent family members

International application No.

**PCT/KR2024/002461**

| Patent document cited in search report | | | Publication date (day/month/year) | Patent family member(s) | | | Publication date (day/month/year) |
|---|---|---|---|---|---|---|---|
| KR | 10-2022-0038669 | A | 29 March 2022 | CN | 114402078 | A | 26 April 2022 |
| | | | | EP | 3987042 | A1 | 27 April 2022 |
| | | | | JP | 2022-537562 | A | 26 August 2022 |
| | | | | US | 2022-0265807 | A1 | 25 August 2022 |
| | | | | WO | 2020-254804 | A1 | 24 December 2020 |
| US | 6004557 | A | 21 December 1999 | EP | 0796273 | A1 | 24 September 1997 |
| | | | | EP | 0796273 | B1 | 17 May 2006 |
| | | | | JP | 10-510989 | A | 27 October 1998 |
| | | | | US | 6303128 | B1 | 16 October 2001 |
| | | | | US | 6306397 | B1 | 23 October 2001 |
| | | | | WO | 96-19496 | A1 | 27 June 1996 |
| KR | 10-2459458 | B1 | 25 October 2022 | CN | 107109400 | A | 29 August 2017 |
| | | | | CN | 107109400 | B | 08 June 2021 |
| | | | | EP | 3246406 | A1 | 22 November 2017 |
| | | | | EP | 3246406 | B1 | 09 March 2022 |
| | | | | JP | 2021-006032 | A | 21 January 2021 |
| | | | | JP | 2022-141733 | A | 29 September 2022 |
| | | | | JP | 6770224 | B2 | 14 October 2020 |
| | | | | JP | 7174958 | B2 | 18 November 2022 |
| | | | | KR | 10-2017-0101926 | A | 06 September 2017 |
| | | | | US | 10544431 | B2 | 28 January 2020 |
| | | | | US | 11834667 | B2 | 05 December 2023 |
| | | | | US | 11926840 | B2 | 12 March 2024 |
| | | | | US | 2017-0369903 | A1 | 28 December 2017 |
| | | | | US | 2020-0157571 | A1 | 21 May 2020 |
| | | | | US | 2020-0190537 | A1 | 18 June 2020 |
| | | | | WO | 2016-114405 | A1 | 21 July 2016 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **VOGEL et al.** *Clin Microbiol Rev.*, 1995, vol. 8 (3), 406-410
- **BOGERS et al.** *J Infect Dis.*, 2015, vol. 211 (6), 947-955
- Molecular Cloning, A Laboratory Manual. Cold Spring Harbor Laboratory Press, 1989
- **BARRETT, J.T.** *Textbook of Immunology*, 1983
- Remington's Pharmaceutical Sciences. 1995
- **WS CHOI et al.** *Microbiol Spectr.*, vol. 18, e0316722
- **HOFFMANN et al.** *PNAS*, 2000, vol. 97, 6108-6113